(19) Europäisches Patentamt
European Patent Office
Office européen des brevets

(11) EP 2 110 432 B1

(12) # EUROPEAN PATENT SPECIFICATION

(45) Date of publication and mention of the grant of the patent:
**21.01.2015 Bulletin 2015/04**

(51) Int Cl.:
***C12N 9/12*** *(2006.01)* ***C12P 19/34*** *(2006.01)*

(21) Application number: **09100097.6**

(22) Date of filing: **17.12.2002**

(54) **High fidelity DNA polymerase compositions and uses therefor**

High-Fidelity-DNA-Polymerase-Zusammensetzungen und Verwendungen dafür

Compositions d'ADN polymérase haute fidélité et leurs utilisations

(84) Designated Contracting States:
**DE FR GB**

(30) Priority: **21.12.2001 US 35091**
**20.02.2002 US 79241**
**30.07.2002 US 208508**
**23.08.2002 US 227110**

(43) Date of publication of application:
**21.10.2009 Bulletin 2009/43**

(62) Document number(s) of the earlier application(s) in accordance with Art. 76 EPC:
**02795911.3 / 1 463 808**

(73) Proprietor: **Agilent Technologies, Inc.**
**Santa Clara, CA 95051 (US)**

(72) Inventors:
- **Hogrefe, Hooly**
  **San Diego, CA 92122 (US)**
- **Borns, Michael**
  **Escondido, CA 92026 (US)**
- **Sorge, Joseph A.**
  **Wilson, WY 93014 (US)**

(74) Representative: **Grünecker, Kinkeldey, Stockmair & Schwanhäusser**
**Leopoldstrasse 4**
**80802 München (DE)**

(56) References cited:
**EP-A- 1 132 474** **WO-A-01/25483**
**WO-A2-01/23583**

- **BOEHLKE ET AL.: "PCR performance of the B-type DNA polymerase from the thermophilic euryarchaeon Thermococcus aggregans improved by mutations in the Y-GG/A motif" NUCLEIC ACIDS RESEARCH, vol. 28, no. 20, 15 October 2000 (2000-10-15), pages 3910-3917, XP002171733 ISSN: 0305-1048**
- **MATTILA P. ET AL.: "Fidelity of DNA synthesis by the Thermococcus litoralis DNA polymerase - an extremely heat stable enzyme with proofreading activity" NUCLEIC ACIDS RESEARCH, vol. 19, no. 18, January 1991 (1991-01), pages 4967-4973, XP000606190 ISSN: 0305-1048**
- **GARDNER A.F. AND JACK W.E.: "Determinants of nucleotide sugar recognition in an archaeon DNA polymerase" NUCLEIC ACIDS RESEARCH, vol. 27, no. 12, 1999, pages 2545-2553, XP002328832**
- **KOMORI K. AND ISHINO Y.: "Functional interdependence of DNA polymerizing and 3'->5' exonucleolytic activities in Pyrococcus furiosus DNA polymerase I" PROTEIN ENGINEERING, vol. 13, no. 1, 2000, pages 41-47, XP002328833**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 110 432 B1

## Description

### FIELD OF THE INVENTION

The present invention is related to the field of high fidelity polynucleotide synthesis.

### BACKGROUND OF THE INVENTION

DNA polymerases catalyze the synthesis of DNA and can be found in all cells as well as being encoded in numerous viruses. Although all DNA polymerases possess 5'-3' DNA polymerization activity, DNA polymerases differ from one another in numerous other properties. For example, some enzymatic activities that are possessed by some DNA polymerases, but absent in other DNA polymerases include: double stranded DNA 5'-3' exonuclease activity, single-stranded DNA 3'-5' exonuclease activity, double-stranded 3'-5' DNA exonuclease activity, RNase H activity, reverse transcriptase activity, and the like. Additionally, different DNA polymerases may have different optimal pH and temperature ranges for activity. Furthermore, DNA polymerases may differ in the rate in which they catalyze DNA synthesis.

Purified DNA polymerases have numerous uses in vitro. A detailed description of DNA polymerases, including methods for their isolation, can be found among other places, in DNA Replication 2nd edition, by Kornberg and Baker, W. H. Freeman & Company, New York, N.Y. 1991. In vitro uses of DNA polymerases include, for example, the labeling and synthesis of hybridization probes, DNA sequencing, and DNA amplification. A DNA amplification method employing DNA polymerases that has been particularly useful is the polymerase chain reaction (PCR) technique which employs the use of a thermostable DNA polymerase.

The first thermostable DNA polymerase that is widely used for DNA amplification is Taq DNA polymerase isolated from the thermostable, aerobic bacterium *Thermus aquaticus*. Taq DNA polymerase's enzymatic activity at high temperatures allows for primer extension and sequencing of polynucleotide templates with complex secondary structures (i.e., by PCR amplification). However, Taq DNA polymerase has significant error rate when incorporating nucleotides due to the lack of 3'-5' exonuclease activity (i.e., proofreading activity), and therefore may not be suitable if the amplified sequence is to be used in further gene structural/functional studies or cloning.

In the last 10 years, numerous studies have quantified the error rate of thermostable DNA polymerases, and several enzymes have been found to copy DNA more accurately than Taq DNA polymerase (referred to as high fidelity DNA polymerases). U.S. Patent describing DNA polymerases include Nos. 4,492,130; 4,946,786; 5,210,036; 5,420,029; 5,489,523; 5,506,137; 5,545,552; 5,618,711; 5,624,833; 6,238,905; 6,100,078; 6,077,664; 5,968,799; 5,948,663; 5,885,713; 5,834,285; 5,756,334; 5,747,298; 5,744,312; 5,624,833; 5,602,011; 5,556,772.

High fidelity polymerases alone should definitely increase fidelity rates but usually do not amplify long fragments as efficient as a DNA polymerase lacking a 3'-5' exonuclease activity (e.g., Taq DNA polymerase). Enzyme mixtures that combine a standard polymerase with a small amount of proofreading polymerase may provide a balance between fidelity and yield. A study published in 1994 illustrated that the use of a high level of a DNA polymerase lacking 3'-5' exonuclease activity (an exo- DNA polymerase, Klentaq-1) with a very low level of a thermostable DNA polymerase exhibiting 3'-5' exonuclease activity (an $exo^+$ DNA polymerase such as Pfu, Vent, or Deep Vent) generated products with increased base-pair fidelity with a maximum yield of 35 kb DNA from 1 ng of lambda DNA template (Barnes, Proceedings of the National Academy of Sciences, 91:2216-20, 1994). Similarly, U.S. Patent Nos. 5,436,149

disclosed methods for improving DNA amplification fidelity using a DNA polymerase composition comprising a first enzyme substantially lacking 3'-5' exonuclease activity and a second enzyme comprising 3'-5' exonuclease activity. In mixtures such as these, the $exo^+$ enzyme acts to correct polymerization errors produced by the $exo^-$ DNA polymerase.

The problem inherited in the above composition comprising the mix of two DNA polymerases is that the high polymerization activity resulted from combining the two DNA polymerases may inhibit the efficiency and therefore the yield of the amplification reaction. Therefore, one can not increase fidelity by increasing the proportion of the proofreading DNA polymerase without compromising PCR product yield. It is also known that the amplification fidelity may also be affected by high DNA polymerase concentration (see for example, Mattila et al., 1991, Nucleic acid Research, 19:4967-73).

EP 1132474 A discloses mutants of the *Thermococcus aggregans* (Tag) DNA polymerase having a reduced polymerase activity, as compared to the corresponding wild-type enzyme. The amino acid residues mutated in the mutants are residues Y387 and G389.

Matilla P. et al, nucleic acids research, vol.19, no.18, January 1991, pages 4967-4973 relates to the problem of fidelity of DNA synthesis by a thermostable DNA polymerase, and shows that increasing the concentration of the polymerase negatively affects the fidelity of amplification.

WO 01/25483 A discloses a composition comprising a thermostable non-proofreading DNA polymerase and a thermostable proofreading DNA polymerase, as well as methods related thereto.

WO 01/23583 A2 discloses purified thermostable enzyme derived form the thermophilic archaebacterium *Archaeoglobus fulgidus*

There is therefore a need in the art for new methods and compositions which improve polymerization fidelity and reduce the side effects resulted from having high polymerization activity in the reaction.

**SUMMARY OF THE INVENTION**

**The present invention relates to the following embodiments defined in items 1-6:**

1. An enzyme mixture comprising a first enzyme and a second enzyme, wherein the first enzyme is a wild type Taq DNA polymerase, and the second enzyme is a mutant pfu DNA polymerase comprising a 3'-5' exonuclease activity comprising one or more mutations in the polymerization domain at amino acid positions D405E, Y410F, T542P, D543G, K593T, Y595S and within the partitioning domain including amino acids 384-389 (SYTGGF) and wherein the DNA polymerization activity is lower than that of the wild-type enzyme.

2. A method for DNA synthesis, comprising:

(a) providing an enzyme mixture according to item 1; and

(b) contacting said enzyme mixture with a nucleic acid template, wherein said enzyme mixture permits DNA synthesis.

3. A method for TA cloning of a DNA synthesis product comprising:

(a) providing an enzyme mixture according to item 1;

(b) contacting said enzyme mixture with a nucleic acid template, wherein said enzyme mixture permits DNA synthesis to generate a synthesised DNA product; and

(c) inserting said synthesised DNA product into a TA cloning vector.

4. A kit comprising the enzyme mixture of item 1, and packaging material therefore.

5. A kit for DNA synthesis according to the method of item 2 comprising a first enzyme, a second enzyme, and packaging material therefore, where the first enzyme is a wild-type Taq DNA polymerase, and the second enzyme is a mutant pfu DNA polymerase comprising 3'-5' exonuclease activity comprising one or more mutations in the polymerization domain at amino acid positions D405E, Y410F, T542P, D543G, K593T, Y595S and within the partitioning domain including amino acids 384-389 (SYTGGF) and wherein the DNA polymerization activity is lower than that of the wild-type enzyme.

6. The kit for DNA synthesis of items 4 or 5, further comprising at least one of polynucleotide precursors, primers, buffers and instructions.

The present disclosure provides an enzyme mixture comprising a first enzyme and a second enzyme, where the first enzyme comprises a DNA polymerization activity, and the second enzyme comprises a 3'-5' exonuclease activity and a reduced DNA polymerization activity.

The present disclosure also provides an enzyme mixture comprising a first enzyme and a second enzyme, where the first enzyme is a wild type Pfu DNA polymerase, the second enzyme is a mutant Pfu DNA polymerase comprising a 3'-5' exonuclease activity and a reduced DNA polymerization activity.

The present disclosure further provides an enzyme mixture comprising a first enzyme and a second enzyme, where the first enzyme is a Taq DNA polymerase, the second enzyme is a mutant Pfu DNA polymerase comprising a 3'-5' exonuclease activity and a reduced DNA polymerization activity.

The present disclosure also provides an enzyme mixture comprising a first enzyme and a second enzyme, where the first enzyme comprises a DNA polymerization activity and is a wild-type Pfu DNA polymerase or a wild-type Taq DNA polymerase, and the second enzyme is a mutant Pfu DNA polymerase comprising a 3'-5' exonuclease activity and a reduced DNA polymerization activity.

The present disclosure provides an enzyme mixture comprising two or more enzymes, where at least a first enzyme in the enzyme mixture comprises a DNA polymerization activity, and at least a second enzyme in the enzyme mixture comprises a 3'-5' exonuclease activity and a reduced DNA polymerization activity.

The present disclosure further provides a mutant Pfu DNA polymerase with reduced DNA polymerization activity, where the mutant Pfu DNA polymerase comprises one or more mutations at amino acid positions selected from the group consisting of: T542, D543, K593, Y595, Y385, G387, and G388.

The present disclosure still provides a composition comprising a mutant Pfu DNA polymerase, where the mutant DNA polymerase comprises one or more mutations at amino acid positions selected from the group consisting of: T542, D543, K593, Y595, Y385, G387, and G388.

The present disclosure provides a mutant Pfu DNA polymerase produced by introducing a mutation in to a polynucleotide encoding a wild type Pfu DNA polymerase to produce a mutant Pfu DNA polymerase comprising one or more mutations at amino acid positions selected from the group consisting of: T542, D543, K593, Y595, Y385, G387, and G388.

The present disclosure also provides a mutant Pfu DNA polymerase comprising a reduced DNA polymerization activity, where the mutant Pfu DNA polymerase is produced by the steps:

(a) providing a polynucleotide encoding a wild-type Pfu DNA polymerase;

(b) introducing one or more nucleotide mutations into the polynucleotide to produce a mutant polynucleotide encoding the mutant Pfu DNA polymerase; and

(c) expressing the mutant polynucleotide to produce the mutant Pfu DNA polymerase, where the mutant Pfu DNA polymerase comprises one or more mutations at amino acid positions selected from the group consisting of: T542, D543, K593, Y595, Y385, G387, and G388.

The present disclosure provides a composition comprising a mutant Pfu DNA polymerase produced by expressing a polynucleotide encoding a Pfu DNA polymerase with a reduced DNA polymerization activity, where the mutant Pfu DNA polymerase comprises one or more mutations at amino acid positions selected from the group consisting of: T542, D543, K593, Y595, Y385, G387, and G388.

The present disclosure also provides a composition comprising a mutant Pfu DNA polymerase comprising a reduced DNA polymerization activity, where the mutant Pfu DNA polymerase is produced by the steps: (a) introducing a mutation into a polynucleotide encoding a wild-type Pfu DNA polymerase to produce a mutant polynucleotide encoding the mutant Pfu DNA polymerase comprising one or more mutations at amino acid positions selected from the group consisting of: T542, D543, K593, Y595, Y385, G387, and G388; (b) expressing the mutant polynucleotide to produce the composition comprising the mutant Pfu DNA polymerase.

The presentdisclosure further provides a kit comprising a first enzyme and a second enzyme, where the first enzyme comprises a DNA polymerization activity, the second enzyme comprises a 3'-5' exonuclease activity and a reduced DNA polymerization activity, and packaging material therefore.

The present disclosure also provides a kit comprising a first enzyme and a second enzyme, and packaging material therefor, where the first enzyme is a wild type Pfu DNA polymerase, the second enzyme is a mutant Pfu DNA polymerase comprising a 3'-5' exonuclease activity and a reduced DNA polymerization activity.

The present disclosure further provides a kit comprising a first enzyme and a second enzyme, and packaging material therefore, where the first enzyme is a Taq DNA polymerase, and packaging material therefor, the second enzyme is a mutant Pfu DNA polymerase comprising a 3'-5' exonuclease activity and a reduced DNA polymerization activity.

The present disclosure provides a kit comprising an enzyme mixture which comprises a first enzyme and a second enzyme, where the first enzyme comprises a DNA polymerization activity and is a wild-type Pfu DNA polymerase or a wild-type Taq DNA polymerase, and the second enzyme is a mutant Pfu DNA polymerase comprising a 3'-5' exonuclease activity and a reduced DNA polymerization activity, and packaging means therefor.

The present disclosure also provides a kit comprising a mutant DNA polymerase which comprises a reduced DNA polymerization activity and packaging material therefor, where the mutant Pfu DNA polymerase comprises one or more mutations at amino acid positions selected from the group consisting of: T542, D543, K593, Y595, Y385, G387, and G388.

In one embodiment, the first enzyme of the present invention is a DNA polymerase or a reverse transcriptase.

Preferably, the DNA polymerase is selected from the group consisting of: Taq DNA polymerase, Tth DNA polymerase, UlTma DNA polymerase, Tli DNA polymerase, Pfu DNA polymerase, KOD DNA polymerase, JDF-3 DNA polymerase, PGB-D DNA polymerase and DP1/DP2 DNA polymerase.

In one embodiment of the present disclosure the second enzyme is a mutant DNA polymerase.

Preferably, the mutant DNA polymerase is derived from a DNA polymerase different from the first enzyme.

More preferably, the mutant DNA polymerase is derived from a DNA polymerase selected from the group consisting of: UlTma DNA polymerase, Tli DNA polymerase, Pfu DNA polymerase, KOD DNA polymerase, JDF-3 DNA polymerase, PGB-D DNA polymerase and DP1/DP2 DNA polymerase.

Preferably, the mutant DNA polymerase comprises a mutation in its partitioning domain or the polymerase domain.

More preferably, the mutant Pfu DNA polymerase comprises one or more mutations at amino acid positions selected from the group consisting of: D405, Y410, T542, D543, K593, Y595, Y385, G387, and G388.

More preferably, the mutant Pfu DNA polymerase comprises one or more mutations selected from the group consisting of: D405E, Y410F, T542P, D543G, K593T, Y595S, Y385Q, Y385S, Y385N, Y385L, Y385H, G387S, G387P, and G388P.

The mutant Pfu DNA polymerase may comprise a mutation of G387P.

The enzyme mixture, composition, or kit may further comprises a PCR enhancing factor and/or an additive.

Preferably, the enzyme mixture, composition, or kit comprising an enzyme mixture comprises a ratio of polymerization activity/exonuclease activity of (2.5-5U)/(0.02-5U).

More preferably, the enzyme mixture, composition, or kit comprising an enzyme mixture comprises a ratio of polymerization activity/exonuclease activity of (2.5U)/(0.04-0.08U).

In the enzyme mixture of the present invention, the first enzyme may be an enzyme of an enzyme blend, where the enzyme mixture is produced by mixing the enzyme blend with the second enzyme.

Preferably, the enzyme blend comprises a wild-type Pfu DNA polymerase and a wild-type Taq DNA polymerase.

Also preferably, the enzyme blend may further comprise a PCR enhancing factor.

The mutant Pfu DNA polymerase of the present invention may comprise one or more mutations selected from the group consisting of: T542P, D543G, K593T, Y595S, Y385Q, Y385S, Y385N, Y385L, Y385H, G387S, G387P, and G388P.

Preferably, the mutant Pfu DNA polymerase comprises one or more mutations selected from the group consisting of: T542P, D543G, K593T, Y595S, Y385Q, Y385S, Y385N, Y385L, Y385H, G387S, G387P, and G388P.

The present disclosure provides an isolated polynucleotide comprising a nucleotide sequence encoding a mutant enzyme comprises a 3'-5' exonuclease activity and a reduced DNA polymerization activity.

Preferably, the mutant enzyme comprising a 3'-5' exonuclease activity and a reduced DNA polymerization activity which is encoded by the isolated polynucleotide of the present invention is a mutant DNA polymerase or a mutant reverse transcriptase.

More preferably, the isolated polynucleotide encodes a mutant Pfu DNA polymerase.

More preferably, the isolated polynucleotide encodes a mutant Pfu DNA polymerase comprises one or more mutations at amino acid positions selected from the group consisting of: T542, D543, K593, Y595, Y385, G387, and G388.

More preferably, the isolated polynucleotide encodes a mutant Pfu DNA polymerase comprises one or more mutations selected from the group consisting of: Y410F, T542P, D543G, K593T, Y595S, Y385Q, Y385S, Y385N, Y385L, Y385H, G387S, G387P, and G388P.

The present disclosure provides a pair of isolated polynucleotides, where a first polynucleotide of the pair comprises a polynucleotide sequence encoding a first enzyme comprising a DNA polymerase activity, and a second polynucleotide of the pair comprises a polynucleotide sequence encoding an enzyme comprising a 3'-5' exonuclease activity and a reduced DNA polymerization activity.

The present disclosure also provides a pair of isolated polynucleotides, where a first polynucleotide of the pair comprises a polynucleotide sequence encoding a wild-type Pfu DNA polymerase or a Taq DNA polymerase, and a second polynucleotide of the pair comprises a polynucleotide sequence encoding an mutant Pfu DNA polymerase comprising a 3'-5' exonuclease activity and a reduced DNA polymerization activity.

Preferably, the second polynucleotide of the pair comprises a polynucleotide sequence encoding a mutant Pfu DNA polymerase which comprises one or more mutations at amino acid positions selected from the group consisting of: D405, Y410, T542, D543, K593, Y595, Y385, G387, and G388.

Also preferably, the second polynucleotide of the pair comprises a polynucleotide sequence encoding a mutant Pfu DNA polymerase which comprises one or more mutations selected from the group consisting of: D405E, Y410F, T542P, D543G, K593T, Y595S, Y385Q, Y385S, Y385N, Y385L, Y385H, G387S, G387P, and G388P.

The present disclosure provides a method for DNA synthesis comprising: (a)
providing an enzyme mixture of the present invention, the enzyme mixture comprising a first enzyme comprising a DNA polymerization activity, and a second enzyme comprising a 3'-5' exonuclease activity and a reduced DNA polymerization activity; and (b) contacting the enzyme mixture with a nucleic acid template, where the enzyme mixture permits DNA synthesis.

Preferably, the nucleic acid template is a DNA or an RNA molecule.

The present disclosure provides a method for DNA synthesis comprising: (a)
providing an enzyme mixture of the present invention, the enzyme mixture comprising a wild type Pfu DNA polymerase as a first enzyme, and a mutant Pfu DNA polymerase as a second enzyme which comprises a 3'-5' exonuclease activity and a reduced DNA polymerization activity; and (b) contacting the enzyme mixture with a nucleic acid template, where the enzyme mixture permits DNA synthesis.

The present disclosure also provides a method for TA cloning of DNA synthesis product comprising: (a) providing an enzyme mixture of the present invention, the enzyme mixture comprising a Taq DNA polymerase as a first enzyme, and a mutant Pfu DNA polymerase as a second enzyme which comprises a 3'-5' exonuclease activity and a reduced DNA polymerization activity; (b) contacting the enzyme mixture with a nucleic acid template, where the enzyme mixture permits DNA synthesis to generate a synthesized DNA product; and (c) inserting the synthesized DNA product into a TA cloning vector.

## BRIEF DESCRIPTION OF DRAWING

Figure 1 is a figure showing PCR proofreading activity assay using Pfu DNA polymerase mutants according to some embodiments of the invention.

Figure 2 is a figure showing PCR performance of Pfu plus Pfu G387P mutant blends according to some embodiments of the invention.

Figure 3 is a figure showing PCR performance of Taq plus Pfu G387P mutant blends according to some embodiments of the invention.

Figure 4 is a figure showing PCR accuracy of PfuTurbo with different amount of PfuG387P according to some embodiments of the invention.

Figure 5 is a figure showing PCR accuracy of PfuTurbo plus PfuG387P according to some embodiments of the invention.

Figure 6 is a figure showing the error rate of Taq plus PfuG387P according to some embodiments of the invention.

Figure 7 is a figure showing the polypeptide and polynucleotide sequences of wild-type DNA polymerases and mutant DNA polymerases according to some embodiments of the invention.

## DETAILED DESCRIPTION OF THE INVENTION

The subject invention provides novel composition for high fidelity polynucleotide synthesis, particularly DNA synthesis. The subject compositions comprise an enzyme mixture for DNA synthesis comprising a first enzyme and a second enzyme, where the first enzyme comprises a DNA polymerization activity, and the second enzyme comprises a 3'-5' exonuclease activity and a reduced DNA polymerization activity. In addition to providing high fidelity for DNA synthesis, the compositions of the subject invention prevent side effects of a high polymerization activity, therefore, increase the efficiency of the amplification compared to a mixture in which both DNA polymerases possess wild-type polymerization activities.

<u>Definitions</u>

As used herein, "synthesis" refers to any in vitro method for making new strand of polynucleotide or elongating existing polynucleotide (i.e., DNA or RNA). Synthesis, according to the invention, include amplification, which increases the number of copies of a polynucleotide template sequence with the use of a polymerase. Polynucleotide synthesis (e.g., amplification) results in the incorporation of nucleotides into a polynucleotide (i.e., a primer), thereby forming a new polynucleotide molecule complementary to the polynucleotide template. The formed polynucleotide molecule and its template can be used as templates to synthesize additional polynucleotide molecules.

"DNA synthesis", according to the invention, includes, but are not limited to PCR, reverse transcription, the labelling of polynucleotide (i.e., for probes and oligonucleotide primers), polynucleotide sequencing.

As used herein, the term "template dependent manner" is intended to refer to a process that involves the template dependent extension of a primer molecule (e.g., DNA synthesis by DNA polymerase). The term "template dependent manner" refers to polynucleotide synthesis of RNA or DNA wherein the sequence of the newly synthesized strand of polynucleotide is dictated by the well-known rules of complementary base pairing (see, for example, Watson, J. D. et al., In: Molecular Biology of the Gene, 4th Ed., W. A. Benjamin, Inc., Menlo Park, Calif. (1987)).

As used herein, "polynucleotide polymerase" refers to an enzyme that catalyzes the polymerization of nucleotide (i.e., the polymerase activity). Generally, the enzyme will initiate synthesis at the 3'-end of the primer annealed to a polynucleotide template sequence, and will proceed toward the 5' end of the template strand. "DNA polymerase" catalyzes the polymerization of deoxynucleotides.

As used herein, the "polymerase domain" refers to the one or more domains of a DNA polymerase which is critical for its polymerization activity. The position of the polymerase domain varies, for example, the polymerase domain for Pfu, Tgo, KDO, Tli (Vent) and PGB-D (dee Vent) are located at amino acid positions as described in Table 2B.

As used herein, the "partitioning domain" refers to a domain of a DNA polymerase which plays a critical role in coordinating the balance between synthesis and degradation of the DNA chain. Generally the partitioning domain is characterized by the YXGG motif (Truniger et al., 1996, EMBO J. 15:3430-3441). This region is located within an accessible loop connecting the 3'-5' exonuclease and polymerase domains. The position of the partitioning domain varies. For example, the partitioning domain for Pfu, Tgo, KDO, Tli (Vent) and PGB-D (dee Vent) are located at amino acid positions 384-389, 383-388, 383-388, 386-391, and 384-389 repectively.

According to the invention, another class of DNA polymerase is "reverse transcriptases", also referred to as "RT", is a critical enzyme responsible for the synthesis of cDNA from viral RNA for all retroviruses, including HIV, HTLV-I, HTLV-II, FeLV, FIV, SIV, AMV, MMTV, and MoMuLV. For review, see e.g. Levin, 1997, Cell, 88:5-8; Brosius et al., 1995, Virus Genes 11:163-79. The term "reverse transcriptase (RT) activity" means the ability to synthesize cDNA from RNA template. Methods for measuring RT activity are well known in the art, for example, the Quan-T-RT assay system is commercially available from Amersham (Arlington Heights, Ill.) and is described in Bosworth, et al., Nature 1989, 341:167-168.

As used herein, a mutant DNA polymerase with "reduced polymerization activity" is a DNA polymerase mutant comprising a DNA polymerization activity which is lower than that of the wild-type enzyme, e.g., comprising less than 10% DNA (e.g., less than 8%, 6%, 4%, 2% or less than 1%) polymerization activity of that of the wild-type enzyme.

As used herein, "exonuclease" refers to an enzyme that cleaves bonds, preferably phosphodiester bonds, between nucleotides one at a time from the end of a DNA molecule. An exonuclease can be specific for the 5' or 3' end of a DNA molecule, and is referred to herein as a 5' to 3' exonuclease or a 3' to 5' exonuclease. A useful exonulcease according to the invention is a 3' to 5' exonuclease which degrades DNA by cleaving successive nucleotides from the 3' end of the polynucleotide. During the synthesis or amplification of a polynucleotide template, a DNA polymerase with 3' to 5' exonuclease activity ($exo^+$) has the capacity of removing mispaired base (proofreading activity), therefore is less error-prone than a DNA polymerase without 3' to 5' exonuclease activity ($exo^-$). The exonuclease activity can be defined by methods well known in the art. For example, one unit of exonuclease activity may refer to the amount of enzyme required to cleave 1 $\mu$g DNA target in an hour at 37°C. Wild type Tth DNA polymerase and Taq DNA polymerase are "$exo^-$" because they do not have 3' to 5' exonuclease activities, however, wild type Pfu DNA polymerase, *E. coli* DNA polymerase I, T7 DNA polymerase, Tma DNA polymerase, Tli DNA polymerase, KOD DNA polymerase, JDF DNA polymerse, and PGB-D DNA polymerase are "$exo^+$" because they all exhibit 3' to 5' exonuclease activity.

The term "fidelity" as used herein refers to the accuracy of DNA polymerization by template-dependent DNA polymerase. The fidelity of a DNA polymerase is measured by the error rate (the frequency of incorporating an inaccurate nucleotide, i.e., a nucleotide that is not incorporated at a template-dependent manner). The accuracy or fidelity of DNA polymerization is maintained by both the polymerase activity and the 3'-5' exonuclease activity of a DNA polymerase. The term "high fidelity" refers to an error rate of $5 \times 10^{-6}$ per base pair or lower. The fidelity or error rate of a DNA polymerase may be measured using assays known to the art (see for example, Lundburg et al., 1991 Gene, 108:1-6).

As used herein, an "amplified product" refers to the double strand polynucleotide population at the end of a PCR amplification reaction. The amplified product contains the original polynucleotide template and polynucleotide synthesized by DNA polymerase using the polynucleotide template during the PCR reaction.

As used herein, "polynucleotide template" or "target polynucleotide template" refers to a polynucleotide containing an amplified region. The "amplified region," as used herein, is a region of a polynucleotide that is to be either synthesized by reverse transcription or amplified by polymerase chain reaction (PCR). For example, an amplified region of a polynucleotide template resides between two sequences to which two PCR primers are complementary to.

As used herein, the term "primer" refers to a single stranded DNA or RNA molecule that can hybridize to a polynucleotide template and prime enzymatic synthesis of a second polynucleotide strand. A primer useful according to the invention is between 10 to 100 nucleotides in length, preferably 17-50 nucleotides in length and more preferably 17-45 nucleotides in length.

"Complementary" refers to the broad concept of sequence complementarity between regions of two polynucleotide strands or between two nucleotides through base-pairing. It is known that an adenine nucleotide is capable of forming specific hydrogen bonds ("base pairing") with a nucleotide which is thymine or uracil. Similarly, it is known that a cytosine nucleotide is capable of base pairing with a guanine nucleotide.

The term "wild-type" refers to a gene or gene product which has the characteristics of that gene or gene product when isolated from a naturally occurring source. In contrast, the term "modified" or "mutant" refers to a gene or gene product which displays altered characteristics when compared to the wild-type gene or gene product. For example, a mutant DNA polymerase in the present invention is a DNA polymerase which exhibit a reduced DNA polymerization activity.

As used herein, an "enzyme mixture" according to the invention, comprises a first enzyme comprising DNA polymerization activity and a second enzyme comprising a 3'-5' exonuclease activity and a reduced DNA polymerization activity. The ratio of the DNA polymerase activity and the exonuclease activity in the enzyme mixture is about (2.5-5U of DNA polymerization activity)/(0.05-10U of 3'-5' exonulcease activity).

As used herein, the term "enzyme blend" refers to an enzyme composition comprising two or more premixed enzymes. The "enzyme blend" may further comprise other reagents, such as PCR enhancing factor, enzyme storage buffer, or reaction buffer.

Useful DNA Polymerases And Reverse Transcriptases

DNA polymerases and their properties are described in detail in, among other places, DNA Replication 2nd edition, Kornberg and Baker, W. H. Freeman, New York, N.Y. (1991).

Known conventional DNA polymerases include, for example, *Pyrococcus furiosus* (Pfu) DNA polymerase (Lundberg et al., 1991, Gene, 108:1, provided by Stratagene), *Pyrococcus woesei* (Pwo) DNA polymerase (Hinnisdaels et al., 1996, Biotechniques, 20:186-8, provided by Boehringer Mannheim), *Thermus thermophilus* (Tth) DNA polymerase (Myers and Gelfand 1991, Biochemistry 30:7661), *Bacillus stearothermophilus* DNA polymerase (Stenesh and McGowan, 1977, Biochim Biophys Acta 475:32), *Thermococcus litoralis* (Tli) DNA polymerase (also referred to as Vent DNA polymerase, Cariello et al., 1991, Polynucleotides Res, 19: 4193, provided by New England Biolabs), 9°Nm DNA polymerase (discontinued product from New England Biolabs), *Thermotoga maritima* (Tma) DNA polymerase (Diaz and Sabino, 1998 Braz J. Med. Res, 31:1239), *Thermus aquaticus* (Taq) DNA polymerase (Chien et al., 1976, J. Bacteoriol, 127: 1550), *Pyrococcus kodakaraensis* KOD DNA polymerase (Takagi et al., 1997, Appl. Environ. Microbiol. 63:4504), JDF-3 DNA polymerase (from *thermococcus sp.* JDF-3, Patent application WO 0132887), *Pyrococcus GB-D* (PGB-D) DNA polymerase (also referred as Deep-Vent DNA polymerase, Juncosa-Ginesta et al., 1994, Biotechniques, 16:820, provided by New England Biolabs), UlTma DNA polymerase (from *thermophile Thermotoga maritima*; Diaz and Sabino, 1998 Braz J. Med. Res, 31:1239; provided by PE Applied Biosystems), Tgo DNA polymerase (from *thermococcus gorgonarius*, provided by Roche Molecular Biochemicals), *E. coli* DNA polymerase I (Lecomte and Doubleday, 1983, Polynucleotide Res. 11:7505), T7 DNA polymerase (Nordstrom et al., 1981, J. Biol. Chem. 256:3112), and archaeal DP1/DP2 DNA polymerase II (Cann et al., 1998, Proc Natl Acad Sci U S A 95:14250-5). The polymerization activity of any of the above enzymes can be defined by means well known in the art. One unit of DNA polymerization activity of conventional DNA polymerase, according to the subject invention, is defined as the amount of enzyme which catalyzes the incorporation of 10 nmoles of total deoxynucleotides (dNTPs) into polymeric form in 30 minutes at optimal temperature (e.g., 72°C for Pfu DNA polymerase). Assays for DNA polymerase activity and 3'-5' exonuclease activity can be found in DNA Replication 2nd Ed., Kornberg and Baker, supra; Enzymes, Dixon and Webb, Academic Press, San Diego, Calif. (1979), as well as other publications available to the person of ordinary skill in the art.

When using the subject compositions in reaction mixtures that are exposed to elevated temperatures, e.g., during the PCR technique, use of thermostable DNA polymerases is preferred.

Reverse transcriptases useful according to the invention include, but are not limited to, reverse transcriptases from HIV, HTLV-1, HTLV-II, FeLV, FIV, SIV, AMV, MMTV, MoMuLV and other retroviruses (for reviews, see for example, Levin, 1997, Cell, 88:5-8; Verma, 1977, Biochim Biophys Acta. 473:1-38; Wu et al., 1975, CRC Crit Rev Biochem. 3:289-347).

Useful First Enzyme Comprising DNA Polymerization Activity

Enzymes comprising DNA polymerization activity according to the present disclosure include enzymes such as DNA polymerases and reverse transcriptases.

The first enzyme used in the subject composition can be any DNA polymerase, with or without a proof reading activity. Preferably, a wild type DNA polymerase is used. However, a mutant DNA polymerase can also be used so long as it provides sufficient DNA polymerization activity required for an amplification reaction.

In a preferred embodiment, the first enzyme comprising DNA polymerization activity is a wild type Pfu DNA polymerase. The enzyme mixture comprising a Pfu DNA polymerase as the first enzyme is also referred to as a Pfu blend herein after.

In preferred embodiments, a Pfu blend enzyme mixture is used for DNA synthesis reaction, e.g., PCR reaction.

In another preferred embodiment, the first enzyme comprising DNA polymerization activity is a wild type Taq DNA polymerase. The enzyme mixture comprising a Taq DNA polymerase as the first enzyme is also referred to as a Taq blend herein after.

In preferred embodiments, a Taq blend enzyme mixture is used for DNA synthesis reaction and for subsequent direct cloning, e.g., PCR reaction followed by TA cloning.

In one embodiment, the first enzyme exists in the form of an enzyme blend. This anzyme blend is mixed with a second enzyme comprising a reduced polymerization activity to produce an enzyme misture of the rpesent invention.

In a preferred embodiment, the enzyme blend is a Herculase® Enhanced or a Herculase® Hotstart DNA polymerase (Stratagene, Cat. No. 600310 or 600260). The enzyme blend can also be selected from commericially available enzyme blend, for example, from the group consisting of: EXL DNA Polymerase (Stratagene, Cat. No. 6003420/2/4), YieldAce DNA Polymerase (Stratagene, Cat. No. 600290/2/4), TaqPlus Precision PCR System (Stratagene, Cat. No. 600210/1/2), TaqPlus Long 100U (Stratagene, Cat. No. 600203/4/5), Advantage 2 PCR Enzyme System (BD Biosciences-Clontech, Cat No. 8430-1), Advantage-GC 2 (BD Biosciences-Clontech, Cat No. 8433-1), Advantage-HF 2 (BD Biosciences-Clontech, Cat No. K1914-y/1), BIO-X-ACT DNA Polymerase (Bioline, Cat. No. BIO-21049/50), TripleMaster PCR System (Brinkmann, Cat. No. 0032-008-216/24/32), FailSafe PCR System (Epicentre, Cat. No. FS99060/100/250/1K), MasterAmp Extra-Long PCR Kit (Epicentre, Cat. No. MHF9220/QU92125/QU92500QU9201K), Synergy DNA Polymerase (GeneCraft, Cat No. GC-005), SynergyN DNA Polymerase(GeneCraft, Cat No. GC-028), SynergyPlus DNA Polymerase (GeneCraft, Cat No. GC-048), Takara ExTaq DNA Polymerase (Intergen, Cat. No. RR001A/B/C), PCR SuperMix High Fidelity (Invitrogen, Cat. No. 10790020), Elongase Enzyme Mix (Invitrogen, Cat. No. 10481018), Takara ExTaq DNA Polymerase (PanVera, Cat. No. TAK RR001A/B/C), Takara LATaq DNA polymerase (PanVera, Cat. No. TAK RR002M/B/C), Expand High Fidelity PCR System (Roche Molecular Biochemicals, Cat. No. 1 732 641/650/078), Expand Long Template PCR System (Roche Molecular Biochemicals, Cat. No. 1 681 834/842; 1 7659 060), Expand 20 kb PLUS PCR System (Roche Molecular Biochemicals, Cat. No. 1 811 002), GC-RICH PCR System (Roche Molecular Biochemicals, Cat. No. 2 140 306), AccuTaq LA DNA Polymerase (Sigma-Aldrich, Cat. No. D8045), KlenTaq LA DNA Polymerase mix (Sigma-Aldrich, Cat. No. D5062), ProofSprinter DNA Polymerase Mix (Thermo Hybaid, Cat. No. PROOFMIX100/300/600) and ProofExpander PCR Kit (Thermo Hybaid, Cat. No. EXPAND100).

The enzyme mixture may comprise three DNA polymerases comprising a first, a second and a third DNA polymerases. The first DNA polymerase is a DNA polymerase with wild-type DNA polymerization activity, e.g., a wild-type Taq DNA polymerase or a wild-type Archaeal DNA polymerase. The second and the third DNA polymerases are mutant DNA polymerases comprising a 3'-5' exonuclease activity and a reduced DNA polymerization activity. Preferably, the second and the third DNA polymerases are different DNA polymerases. More preferably, the second and the third DNA polymerases are different DNA polymerases selected from the group consisting of: mutant Pfu DNA polymerase, mutant Tgo DNA polymerase, mutant KOD DNA polymerase, mutant Vent DNA polymerase, and mutant Deep Vent DNA polymerase. More preferably, the mutant Pfu DNA polymerase comprises one or more mutations at amino acid positions selected from the group consisting of: D405, Y410, T542, D543, K593, Y595, Y385, G387, and G388; the mutant Tgo DNA polymerase comprises one or more mutations at amino acid positions selected from the group consisting of: D404, Y409, T541, D542, K592, Y594, Y384, G386, and G387; the mutant KOD DNA polymerase comprises one or more mutations at amino acid positions selected from the group consisting of: D404, Y409, T541, D542, K592, Y594, Y384, G386, and G387; the mutant Vent DNA polymerase comprises one or more mutations at amino acid positions selected from the group consisting of: D407, Y412, T544, D545, K595, Y597, Y387, G389, and G390; the mutant Deep Vent DNA polymerase comprising one or more mutations at amino acid positions selected from the group consisting of: D405, Y410, T542, D543, K593, Y595, Y385, G387, and G388. The two mutant DNA polymerases comprising a 3'-5' exonuclease activity and a reduced DNA polymerization activity are preferably having different 3'-5' exonuclease specificity. For example, they may be a mutant JDF-3 DNA polymerase (e.g., G387) and a mutant Pfu DNA polymerase (e.g., G387). The mixture comprising two mutant DNA polymerases as such will enhance the proofreading activity of the mixture because JDF and Pfu have different proofreading spectra so that they can complement with each other to achieve better fidelity of the amplification reaction. It is understood that the combination of the second and the third enzymes in the mixture of the present invention is not limited to the three enzymes listed in this example. With the scope of the present invention, any two mutant Archaeal DNA polymerases can be used in the same mixture with a first DNA polymerase (Archaeal or non-Archaeal DNA polymerase). Some non-limiting examples of such three-enzyme mixtures include: a wild-type Taq DNA polymerase, a JDF-3 mutant and a Pfu mutant; a wild-type Taq DNA polymerase, a KOD mutant and a Pfu mutant; a wild-type JDF DNA polymerase, a JDF-3 mutant and a Pfu mutant; a wild-type Pfu DNA polymerase, a JDF-3 mutant and a Pfu mutant; a wild-type KOD DNA polymerase, a JDF-3 mutant and a Pfu mutant; a wild-type KOD DNA polymerase, a KOD-3 mutant and a Pfu mutant; a wild-type Pfu DNA polymerase, a JDF-3 mutant and a Pfu mutant; a wild-type Pfu DNA polymerase, a KOD mutant and a Pfu mutant.

Useful Second Enzyme Comprising 3'-5' Exonuclease Activity

Enzyme comprising 3'-5' exonuclease activity (i.e., proofreading DNA polymerase) according to the invention include, but are not limited to, DNA polymerases, E. coli exonuclease I, E. coli exonuclease III, E. coli recBCD nuclease, mung bean nuclease, and the like (see for example, Kuo, 1994, Ann N Y Acad Sci., 726:223-34).

Any proofreading DNA polymerase could be mutagenized to reduce/eliminate DNA polymerase activity and used in the enzyme reaction of the present invention. Examples can be found in many DNA polymerase families including, but are not limited to such as follows:

Family B DNA polymerases

Bacteriophage T4 DNA polymerase, ϕ29 DNA polymerase, T7 DNA polymerase; *E. coli* pol II DNA polymerase; human DNA polymerase δ, human DNA polymerase γ, archaeal DNA polymerase I (Table 1).

Eubacterial Family A DNA polymerases (with proofreading activity)

*E. coli* DNA pol I (Klenow fragment), *Thermotoga maritima* (UlTma fragment)

Family D DNA polymerases (unrelated to Families A, B, C)

Archaeal DNA polymerase II (DP1/DP2) e.g., as described in Cann et al (1998) PNAS 95:14250-5.

**Table 1. Accession Information for Cloned Family B Polymerases**

Vent Thermococcus litoralis
ACCESSION AAA72101
PID g348689
VERSION AAA72101.1 GI:348689
DBSOURCE locus THCVDPE accession M74198.1
THEST THERMOCOCCUS SP. (STRAIN TY)
ACCESSION O33845
PID g3913524
VERSION 033845 GI:3913524
DBSOURCE swissprot: locus DPOL_THEST, accession 033845
Pab Pyrococcus abyssi
ACCESSION P77916
PID g3913529
VERSION P77916 GI:3913529
DBSOURCE swissprot: locus DPOL_PYRAB, accession P77916
PYRHO Pyrococcus horikoshii
ACCESSION 059610
PID g3913526
VERSION 059610 GI:3913526
DBSOURCE swissprot: locus DPOL_PYRHO, accession 059610
PYRSE PYROCOCCUS SP. (STRAIN GE23)
ACCESSION P77932
PID g3913530
VERSION P77932 GI:3913530
DBSOURCE swissprot: locus DPOL_PYRSE, accession P77932
DeepVent Pyrococcus sp.
ACCESSION AAA67131
PID g436495
VERSION AAA67131.1 GI:436495
DBSOURCE locus PSU00707 accession U00707.1
Pfu Pyrococcus furiosus
ACCESSION P80061
PID g399403
VERSION P80061 GI:399403
DBSOURCE swissprot: locus DPOL_PYRFU, accession P80061
JDF-3 Thermococcus sp.

(continued)

Unpublished
Baross gi|2097756|pat|US|5602011|12 Sequence 12 from patent US 5602011
9degN THERMOCOCCUS SP. (STRAIN 9ON-7).
ACCESSION Q56366
PID g3913540
VERSION Q56366 GI:3913540
DBSOURCE swissprot: locus DPOL_THES9, accession Q56366
KOD Pyrococcus sp.
ACCESSION BAA06142
PID g1620911
VERSION BAA06142.1 GI:1620911
DBSOURCE locus PYWKODPOL accession D29671.1
Tgo Thermococcus gorgonarius.
ACCESSION 4699806
PID g4699806
VERSION GI:4699806
DBSOURCE pdb: chain 65, release Feb 23, 1999
THEFM Thermococcus fumicolans
ACCESSION P74918
PID g3913528
VERSION P74918 GI:3913528
DBSOURCE swissprot: locus DPOL_THEFM, accession P74918
METTH Methanobacterium thermoautotrophicum
ACCESSION 027276
PID g3913522
VERSION 027276 GI:3913522
DBSOURCE swissprot: locus DPOL_METTH, accession 027276
Metja Methanococcus jannaschii
ACCESSION Q58295
PID g3915679
VERSION Q58295 GI:3915679
DBSOURCE swissprot: locus DPOL_METJA, accession Q58295
POC Pyrodictium occultum
ACCESSION B56277
PID g1363344
VERSION B56277 GI:1363344
DBSOURCE pir: locus B56277
ApeI Aeropyrum pernix
ACCESSION BAA81109
PID g5105797
VERSION BAA81109.1 GI:5105797
DBSOURCE locus AP000063 accession AP000063.1
ARCFU Archaeoglobus fulgidus
ACCESSION 029753
PID g3122019
VERSION 029753 GI:3122019
DBSOURCE swissprot: locus DPOL_ARCFU, accession 029753
Desulfurococcus sp. Tok.
ACCESSION 6435708
PID g64357089
VERSION GT:6435708

(continued)

DBSOURCE pdb. chain 65, release Jun 2, 1999

Enzymes possessing 3'-5' exonuclease activity for use in the present compositions and methods may be isolated from natural sources or produced through recombinant DNA techniques. Preferably, the enzyme comprising 3'-5' exonuclease activity is a DNA polymerase.

A DNA polymerase comprising 3'-5' exonuclease activity (referred as $exo^+$) is capable of proofreading the incorporated nucleotides produced by its own polymerization activity. Among other applications, $exo^+$ DNA polymerases are particularly suited for cloning of PCR products, characterization of polynucleotide sequences. Useful $exo^+$ DNA polymerases include, but are not limited to, Pwo DNA polymerase; Vent DNA polymerases; Deep Vent DNA polymerase; 9°Nm DNA polymerase; UlTma DNA polymerase; Tli DNA polymerase; Pfu DNA polymerase; JDF-3 DNA polymerase; Tgo DNA polymerase; KOD DNA polymerase; and PGB-D DNA polymerase.

In preferred embodiments of the subject invention, an $exo^+$ DNA polymerase with reduced DNA polymerization activity is used as the second enzyme.

<u>Preparing $Exo^+$ DNA Polymerase With Reduced DNA Polymerization Activity</u>

The cloned wild-type $Exo^+$ DNA polymerase may be modified to generate forms exhibiting reduced polymerization activity by a number of methods. These include the methods described below and other methods known in the art. Any $exo^+$ DNA polymerase can be used to prepare for the $exo^+$ DNA polymerase with reduced DNA polymerization activity in the invention.

A. Genetic Modifications - Mutagenesis

The preferred method of preparing a DNA polymerase with reduced polymerization activity is by genetic modification (e.g., by modifying the DNA sequence of a wild-type DNA polymerase). Within the sequence of an $exo^+$ DNA polymerase, the preferred sequence for genetic modification is the DNA sequence encoding the polymerization domain. Polymerization and exonuclease domains (i.e., their crystal structures) of many DNA polymerases are known in the art (for examples, see Rodriguez et al., 2000, J. Mol. Biol. 299:447-62; Zhao et al., 1999, Structure Fold Des. 7:1189-99; Baker et al., 1998, Proc Natl Acad Sci U S A. 95:3507-12; Kiefer et al., 1997, Structure 5:95-108; Kim et al., 1995, Nature, 376:612-6; Kong et al., 1993, J Biol Chem. 268:1965-75).

General structure features of DNA polymerization domain is known in the art. For example, Blanco et al. (1991, Gene, 100:27-38) discloses that significant amino acid (aa) sequence similarity has been found in the C-terminal portion of 27 DNA-dependent DNA polymerases belonging to the two main superfamilies: (i) Escherichia coli DNA polymerase I (PolI)-like prokaryotic DNA polymerases, and (ii) DNA polymerase alpha-like prokaryotic and eukaryotic (viral and cellular) DNA polymerases. The six most conserved C-terminal regions, spanning approximately 340 amino acids, are located in the same linear arrangement and contain highly conserved motifs and critical residues involved in the polymerization function.

According to the three-dimensional model of PolIk (Klenow fragment), these six conserved regions are located in the proposed polymerization domain, forming the metal and dNTP binding sites and the cleft for holding the DNA template. Site-directed mutagenesis studies support these structural predictions.

The 3'-5' exonuclease active site of E. coli DNA polymerase I is predicted to be conserved for both prokaryotic and eukaryotic DNA polymerases based on amino acid sequence homology (Bernad et al., 1989, Cell, 59:219-28). Three amino acid regions containing the critical residues in the E. coli DNA polymerase I involved in metal binding, single-stranded DNA binding, and catalysis of the exonuclease reaction are located in the amino-terminal half and in the same linear arrangement in several prokaryotic and eukaryotic DNA polymerases. Site-directed mutagenesis at the predicted exonuclease active site of the phi 29 DNA polymerase, a model enzyme for prokaryotic and eukaryotic alpha-like DNA polymerases, specifically inactivated the 3'-5' exonuclease activity of the enzyme. These results reflect a high evolutionary conservation of this catalytic domain.

With the great availability of sequences from DNA polymerases, it has become possible to delineate a few highly conserved regions for various polymerase types (for review, see for example, Johnson, 1993, Annu Rev Biochem. 62:685-713). Delarue et al. reported an approach for unifying the structure of DNA polymerase (1990, Protein Eng., 3:461-7). The speculative hypothesis should provide a useful model to direct genetic modifications for preparing DNA polymerase with reduced polymerization activity.

Preferably, the genetic modification for preparing $exo^+$ DNA polymerase with reduced polymerization activity does not significantly reduces its 3'-5' exonuclease activity (i.e., the proof reading ativity).

Known DNA polymerase mutants that selectively reduce DNA polymerization activity can be found in the art, for example, in Blanco et al., 1995 Methods of Enzymology 262:283-294 ((Bacteriophage ϕ29); Truniger et al., 1996, EMBO J. 15:3430-3441 (Bacteriophage ϕ29); Abdus Sattar et al.,1996, Biochemistry 35:16621-9 (Bacteriophage T4); Tuske et al., 2000, J. Biological Chemistry 275:23759-68 (Klenow fragment); Bohlke et al., 2000, Nucleic Acid Research 28:3910-3917 (Thermococcus aggregans); Pisani et al., 1998, Biochemistry 37:15005-15012 (Sulfolobus solfataricus); Komori et al., 2000, Protein Eng 13:41-7 (Pyrococcus furiosus); Shen et al., 2001 J. Biological Chemistry 276:27376-83 (Pyrococcus horikoshi Family D).

Site-directed mutagenesis of bacteriophage ϕ29 DNA polymerase leads to the identification of mutations in the polymerase domain which reduce DNA polymerase activity, while having minimal effects on 3'-5' exonuclease activity (Blanco, L. and Salas, M. 1995, Methods of Enzymology 262:283-294). In one embodiment of the invention, one or more corresponding amino acids in Pfu DNA polymerases are mutated (e.g., by substitutions: D405E, Y410F, T542P, D543G, K593T, Y595S). It is understood that other amino acid side substitutions at these same sites would also selectively reduce DNA polymerase activity.

The ϕ29 DNA polymerase mutagenesis studies targeted amino acid residues within highly conserved Family B motifs (DXXSLYP [SEQ ID NO. 1], KXXXNSXYG [SEQ ID NO. 2], TXXGR [SEQ ID NO. 3], YXDTDS [SEQ ID NO. 4], and KXY [SEQ ID NO.5]), although other regions of the protein presumably can be mutagenized to selectively decrease DNA polymerase activity. One such region is the partitioning domain, characterized by the YXGG [SEQ ID NO.6] motif (Truniger et al., 1996, EMBO J. 15:3430-3441). This region is located within an accessible loop connecting the 3'-5' exonuclease and polymerase domains. The partitioning domain plays a critical role in coordinating the balance between synthesis and degradation of the DNA chain. Mutations within this region disrupt the equilibrium between polymerization and proofreading, and produce phenotypes favoring either polymerization (reduced proofreading) or proofreading (reduced polymerization).

Non-conservative (S,N) substitutions at $Y_{387}$ (equivalent to $Y_{385}$ in Pfu) in the partitioning domain of the archaeal *Thermococcus aggregans* DNA polymerase lead to a significant reduction in DNA polymerase activity and enhanced exonuclease activity, which results in improved enzyme fidelity (used alone in PCR) (Bohlke, K. et al (2000) NAR 28:3910-3917). In contrast, conservative substitutions at $Y_{387}$ (F, W, H) lead to wild-type-like fidelity and enhanced PCR performance, which may be related to improved polymerization. A G389A mutation (equivalent to Pfu G387) in *Thermococcus aggregans* DNA polymerase lead to reduced DNA polymerase activity (10% wt), increased exonuclease activity (236% wt), and loss of product synthesis in PCR (Bohlke, K. et al (2000) NAR 28:3910-3917). Analogous mutations have been investigated in bacteriophage ϕ29 DNA polymerase (Truniger, V., et al (1996) EMBO J. 15:3430-3441) and in the archaeal *Solfolobus solfataricus* (Sso) DNA polymerase (Pisani, F.M., DeFelice, M., and Rossi, M. (1998) Biochemistry 37:15005-15012), where a G→A mutation either decreases (pol/exo = 0.6 for *Sso*) or increases (pol/exo = 91 for ϕ29) DNA polymerase activity relative to exonuclease activity.

In one embodiment of the invention, Pfu DNA polymerase was mutated within the partitioning domain at amino acids 384-389 (SYTGGF [SEQ ID NO. 7]) to obtain a Pfu DNA polymerase with reduced polymerization activity. It is understood that other amino acid side substitutions within the partitioning domain, e.g., at positions Y385, G387, G388, could also selectively reduce DNA polymerase activity while having minimal effects on exonuclease activity.

In another embodiment, two or mutations are combined (e.g., by introducing additional site-directed mutations into a mutant Pfu DNA polymerase) to effectively eliminate DNA polymerase activity, while retaining high levels of proofreading activity.

U.S. Patent Nos. 5,691,142, 5,614,402 and 5,541,311 disclose methods of deriving 5'-3' nucleases from thermostable DNA polymerases for the detection of target polynucleotide molecules. These methods can be applied to the subject invention for preparing DNA polymerase comprising 3'-5' exonuclease activity with a reduced polymerization activity. Other techniques for genetic modification are well known in the art (see for example, Ausubel et. al. Short Protocols in Molecular Biology (1995) 3rd Ed. John Wiley & Sons, Inc.).

Modification to the primary structure of a wild type enzyme by deletion, addition, or alteration of the amino acids incorporated into the sequence during translation can be made without destroying the high temperature DNA polymerase activity of the protein. Such substitutions or other alterations result in proteins useful in the methods of the present invention. The availability of DNA encoding these sequences provides the opportunity to modify the codon sequence to generate mutant enzymes having reduced polymerization activity. A few methods for altering DNA sequences are provided below, any other method known in the art may also be used.

There are a number of site-directed mutagenesis methods known in the art which allow one to mutate a particular site or region in a straightforward manner, based on the sequences of the polymerization domain of a DNA polymerase. There are a number of kits available commercially for the performance of site-directed mutagenesis, including both conventional and PCR-based methods. Examples include the EXSITE™ PCR-Based Site-directed Mutagenesis Kit available from Stratagene (Catalog No. 200502) and the QUIKCHANGE™ Site-directed mutagenesis Kit from Stratagene (Catalog No. 200518), and the CHAMELEON® double-stranded Site-directed mutagenesis kit, also from Stratagene (Catalog No. 200509).

Older methods of site-directed mutagenesis known in the art relied upon sub-cloning of the sequence to be mutated into a vector, such as an M13 bacteriophage vector, that allows the isolation of single-stranded DNA template. In these methods one anneals a mutagenic primer (i.e., a primer capable of annealing to the site to be mutated but bearing one or mismatched nucleotides at the site to be mutated) to the single-stranded template and then polymerizes the complement of the template starting from the 3' end of the mutagenic primer. The resulting duplexes are then transformed into host bacteria and plaques are screened for the desired mutation.

More recently, site-directed mutagenesis has employed PCR methodologies, which have the advantage of not requiring a single-stranded template. In addition, methods have been developed that do not require sub-cloning. Several issues must be considered when PCR-based site-directed mutagenesis is performed. First, in these methods it is desirable to reduce the number of PCR cycles to prevent expansion of undesired mutations introduced by the polymerase. Second, a selection must be employed in order to reduce the number of non-mutated parental molecules persisting in the reaction. Third, an extended-length PCR method is preferred in order to allow the use of a single PCR primer set. And fourth, because of the non-template-dependent terminal extension activity of some thermostable polymerases it is often necessary to incorporate an end-polishing step into the procedure prior to blunt-end ligation of the PCR-generated mutant product.

The protocol described below accommodates these considerations through the following steps. First, the template concentration used is approximately 1000-fold higher than that used in conventional PCR reactions, allowing a reduction in the number of cycles from 25-30 down to 5-10 without dramatically reducing product yield. Second, the restriction endonuclease DpnI (recognition target sequence: 5-Gm6ATC-3, where the A residue is methylated) is used to select against parental DNA, since most common strains of E. coli Dam methylate their DNA at the sequence 5-GATC-3. Third, Taq Extender is used in the PCR mix in order to increase the proportion of long (i.e., full plasmid length) PCR products. Finally, Pfu DNA polymerase is used to polish the ends of the PCR product prior to intramolecular ligation using T4 DNA ligase.

A non-limiting example for the method is described in detail as follows:

> Plasmid template DNA (approximately 0.5 pmole) is added to a PCR cocktail containing: 1x mutagenesis buffer (20 mM Tris HCl, pH 7.5; 8 mM $MgCl_2$; 40 $\mu$g/ml BSA); 12-20 pmole of each primer (one of skill in the art may design a mutagenic primer as necessary, giving consideration to those factors such as base composition, primer length and intended buffer salt concentrations that affect the annealing characteristics of oligonucleotide primers; one primer must contain the desired mutation, and one (the same or the other) must contain a 5' phosphate to facilitate later ligation), 250 $\mu$M each dNTP, 2.5 U Taq DNA polymerase, and 2.5 U of Taq Extender (Available from Stratagene; See Nielson et al. (1994) Strategies 7: 27, and U.S. Patent No. 5,556,772). Primers can be prepared using the triester method of Matteucci et al., 1981, J. Am. Chem. Soc. 103:3185-3191. Alternatively automated synthesis may be preferred, for example, on a Biosearch 8700 DNA Synthesizer using cyanoethyl phosphoramidite chemistry.

The PCR cycling is performed as follows: 1 cycle of 4 min at 94°C, 2 min at 50°C and 2 min at 72°C; followed by 5-10 cycles of 1 min at 94°C, 2 min at 54°C and 1 min at 72°C. The parental template DNA and the linear, PCR-generated DNA incorporating the mutagenic primer are treated with DpnI (10 U) and Pfu DNA polymerase (2.5U). This results in the DpnI digestion of the in vivo methylated parental template and hybrid DNA and the removal, by Pfu DNA polymerase, of the non-template-directed Taq DNA polymerase-extended base(s) on the linear PCR product. The reaction is incubated at 37°C for 30 min and then transferred to 72°C for an additional 30 min. Mutagenesis buffer (115 ul of 1x) containing 0.5 mM ATP is added to the DpnI-digested, Pfu DNA polymerase-polished PCR products. The solution is mixed and 10 ul are removed to a new microfuge tube and T4 DNA ligase (2-4 U) is added. The ligation is incubated for greater than 60 min at 37°C. Finally, the treated solution is transformed into competent E. coli according to standard methods.

Methods of random mutagenesis which will result in a panel of mutants bearing one or more randomly-situated mutations exist in the art. Such a panel of mutants may then be screened for those exhibiting reduced polymerization relative to the wild-type polymerase (e.g., by measuring the incorporation of 10nmoles of dNTPs into polymeric form in 30 minutes at the optimal temperature for a given DNA polymerase). An example of a method for random mutagenesis is the so-called "error-prone PCR method". As the name implies, the method amplifies a given sequence under conditions in which the DNA polymerase does not support high fidelity incorporation. The conditions encouraging error-prone incorporation for different DNA polymerases vary, however one skilled in the art may determine such conditions for a given enzyme. A key variable for many DNA polymerases in the fidelity of amplification is, for example, the type and concentration of divalent metal ion in the buffer and the inherited fidelity of the PCR enzyme. The use of manganese ion and/or variation of the magnesium or manganese ion concentration may therefore be applied to influence the error rate of the polymerase.

In a preferred embodiment, the second enzyme with reduced polymerization activity is derived from Pfu DNA polymerase.

The DNA coding sequence of a wild-type Pfu DNA polymerase can be found in the art, for example, from Genbank (accession No. U84155). A detailed description of the structure and function of Pfu DNA polymerase can be found, among other places in U.S. Patent Nos. 5,948,663; 5,866,395; 5,545,552; 5,556,772. A not-limiting detailed procedure for preparing Pfu DNA polymerase with reduced polymerization activity is provided in Example 1.

A person of average skill in the art having the benefit of this disclosure will recognize that polymerases with reduced polymerization activity derived from other exo$^+$ DNA polymerases including Vent DNA polymerase, JDF-3 DNA polymerase, Tgo DNA polymerase and the like may be suitably used in the subject compositions.

The first or the second enzyme of the subject composition may comprise DNA polymerases that have not yet been isolated. Assays for both DNA polymerization activity and 3'-5' exonuclease activity can be found in the subject description and in DNA Replication 2nd Ed., Kornberg and Baker, supra; Enzymes, Dixon and Webb, Supra, as well as other publications available to the person of ordinary skill in the art.

In preferred embodiments, mutant Pfu DNA polymerase comprises one or more mutations at amino acid positions selected from the group consisting of: D405, Y410, T542, D543, K593, Y595, Y385, G387, and G388.

More preferably, the mutant Pfu DNA polymerase comprises one or more mutations selected from the group consisting of: D405E, Y410F, T542P, D543G, K593T, Y595S, Y385Q, Y385S, Y385N, Y385L, Y385H, G387S, G387P, and G388P.

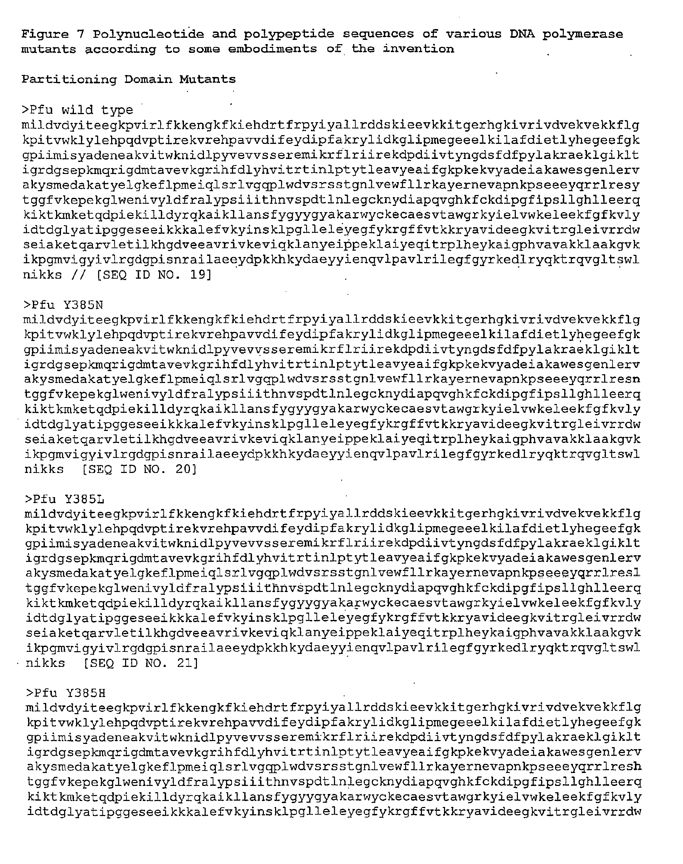

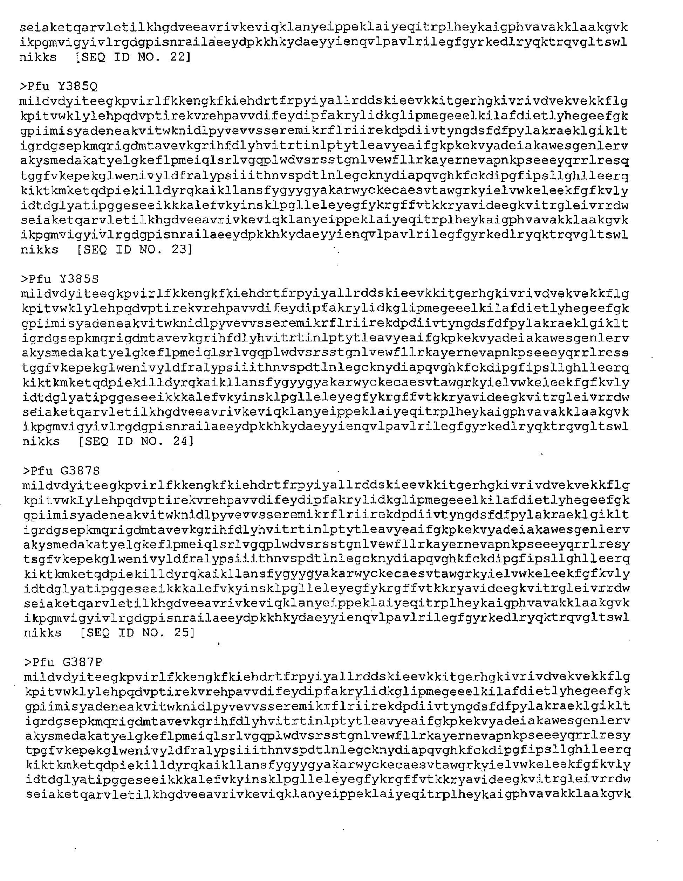

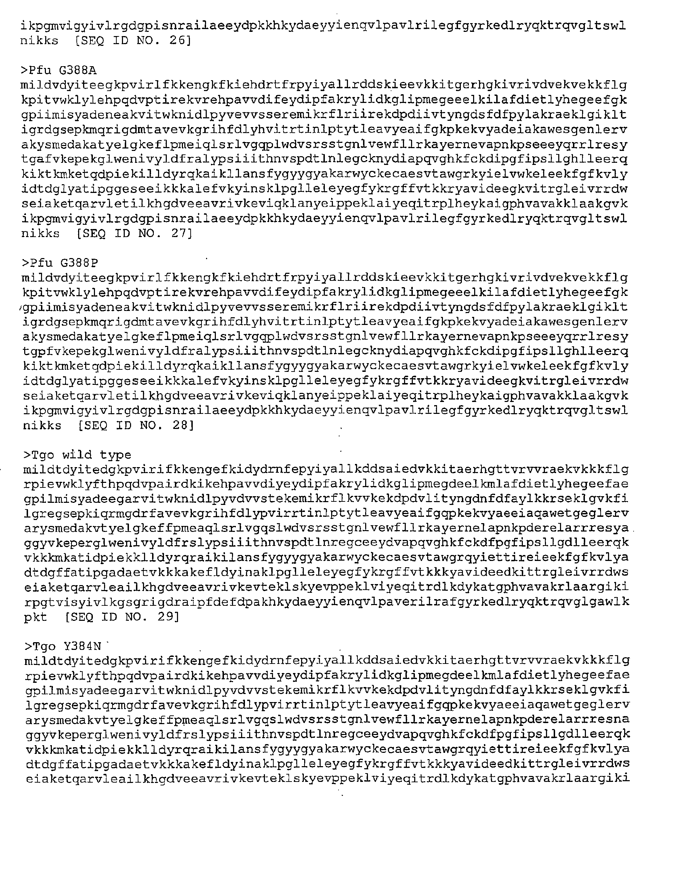

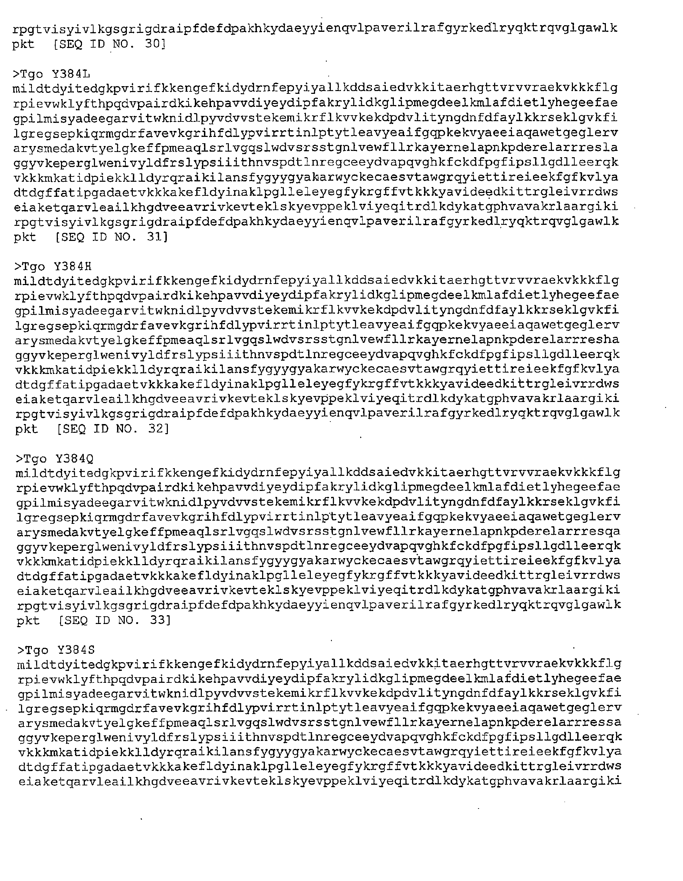

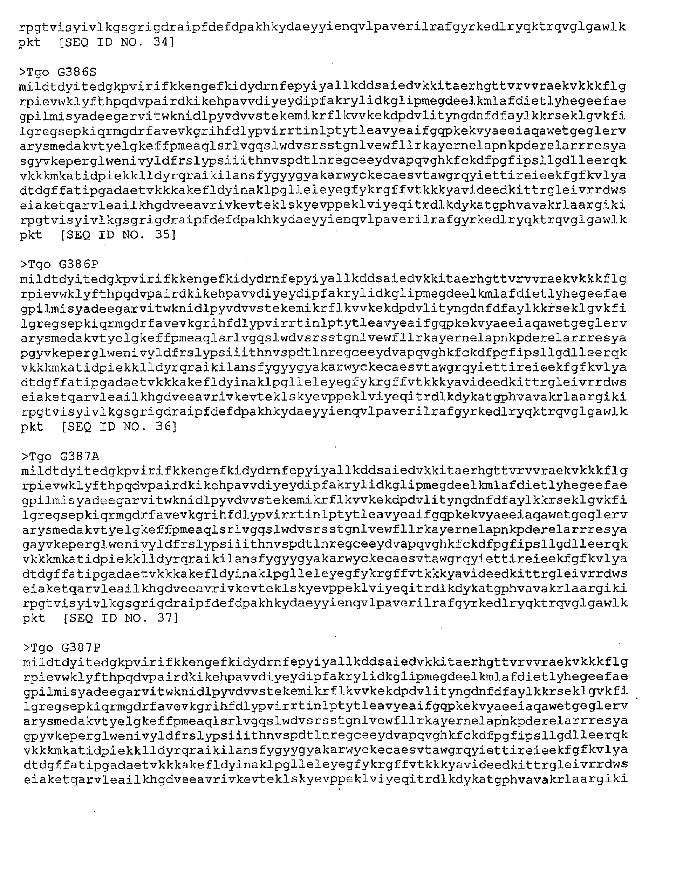

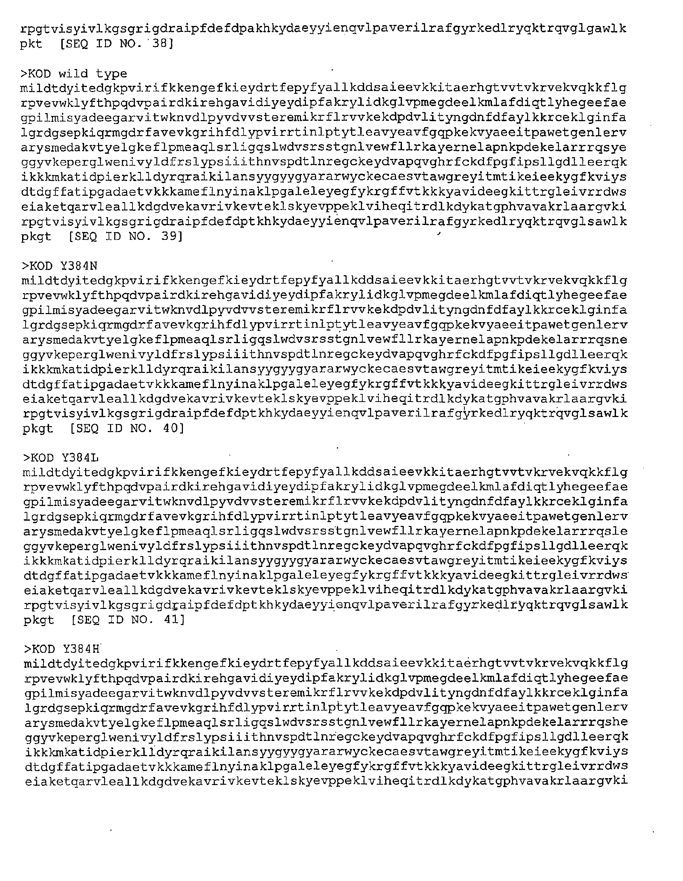

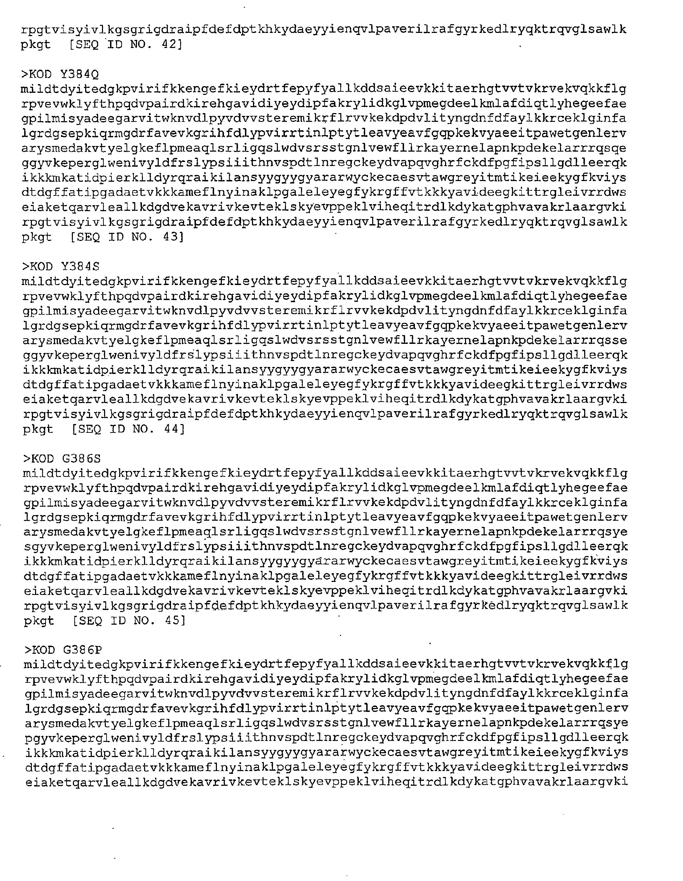

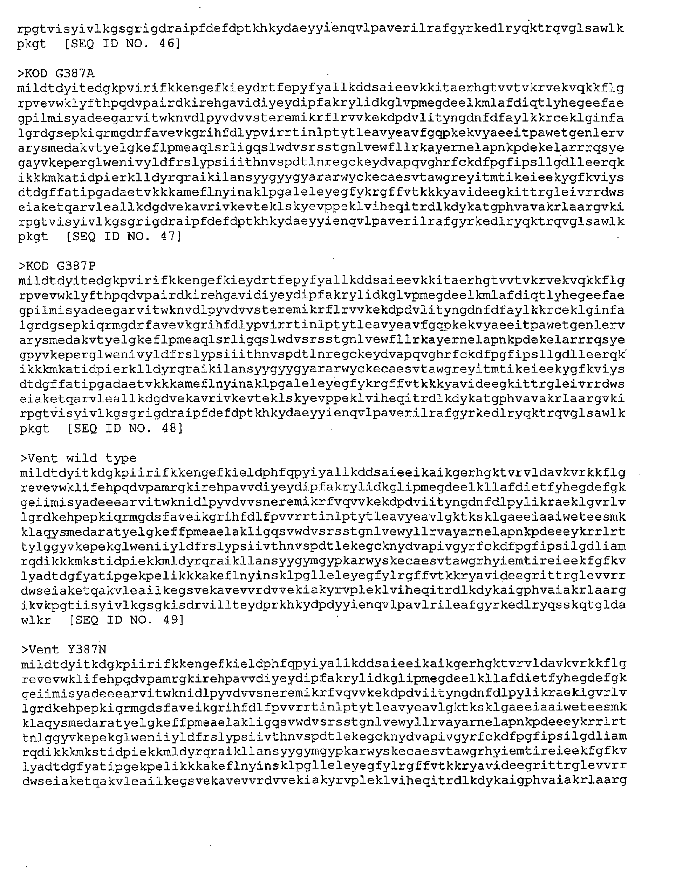

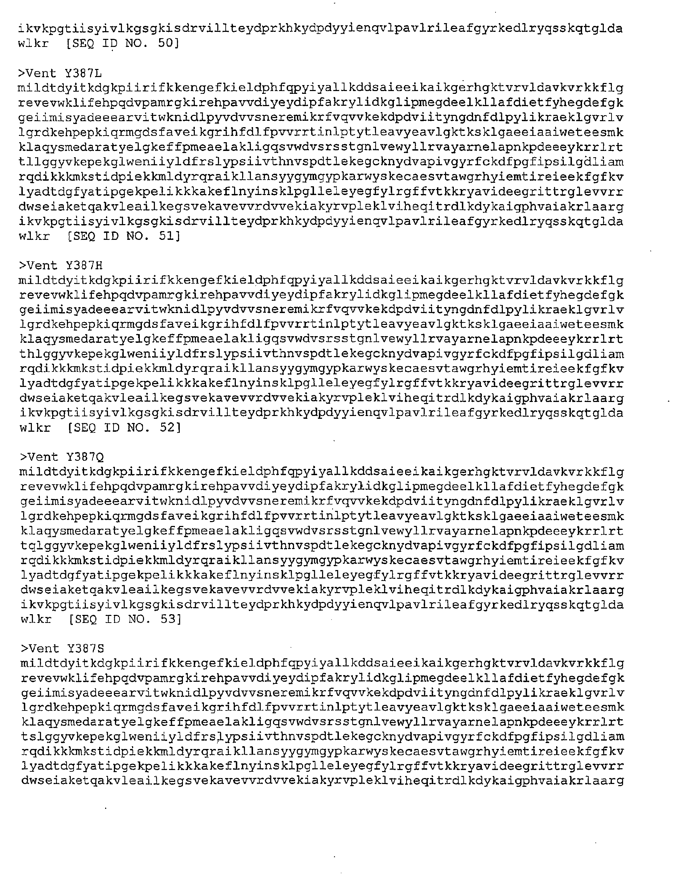

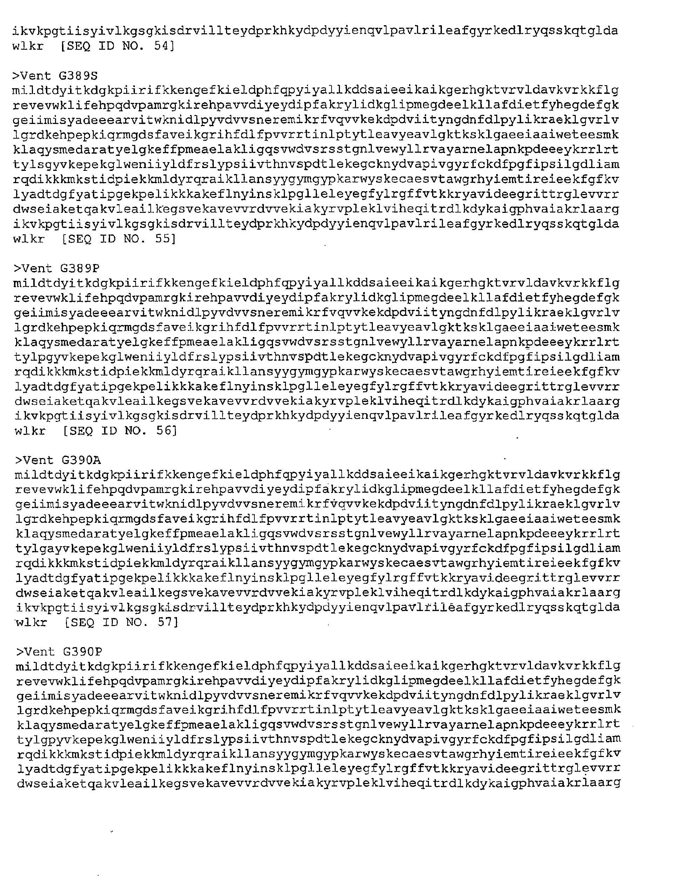

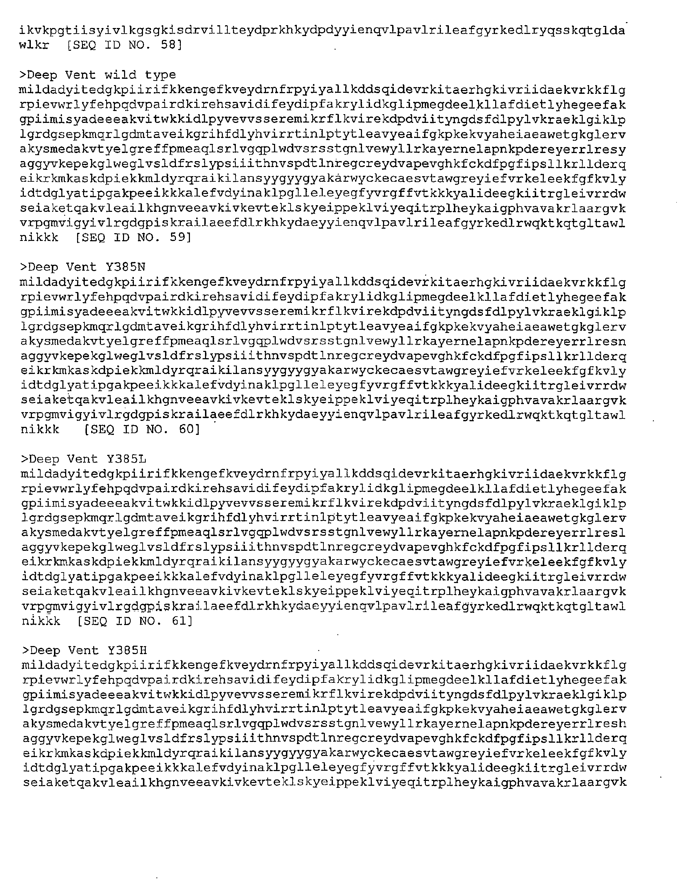

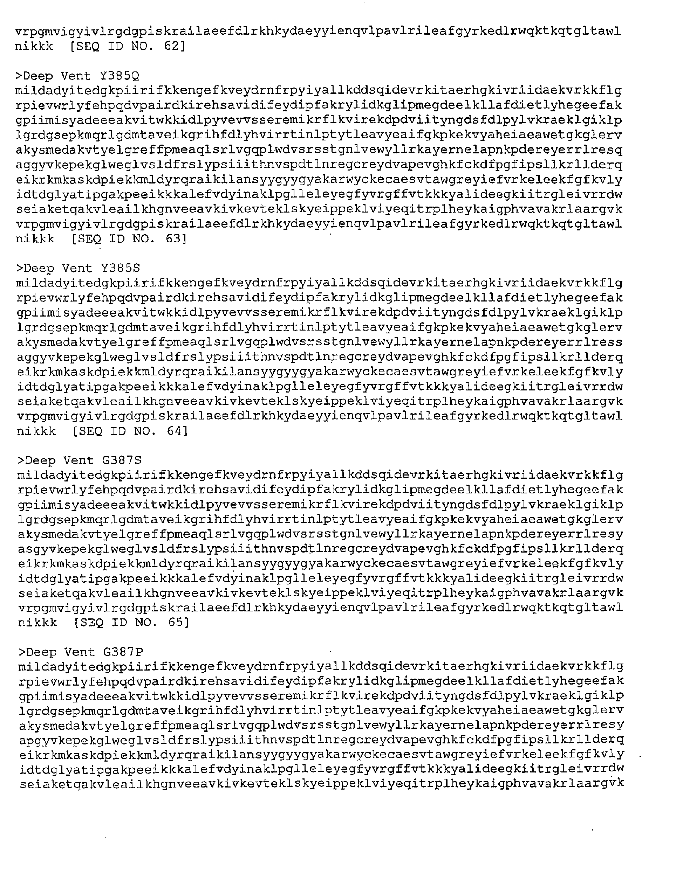

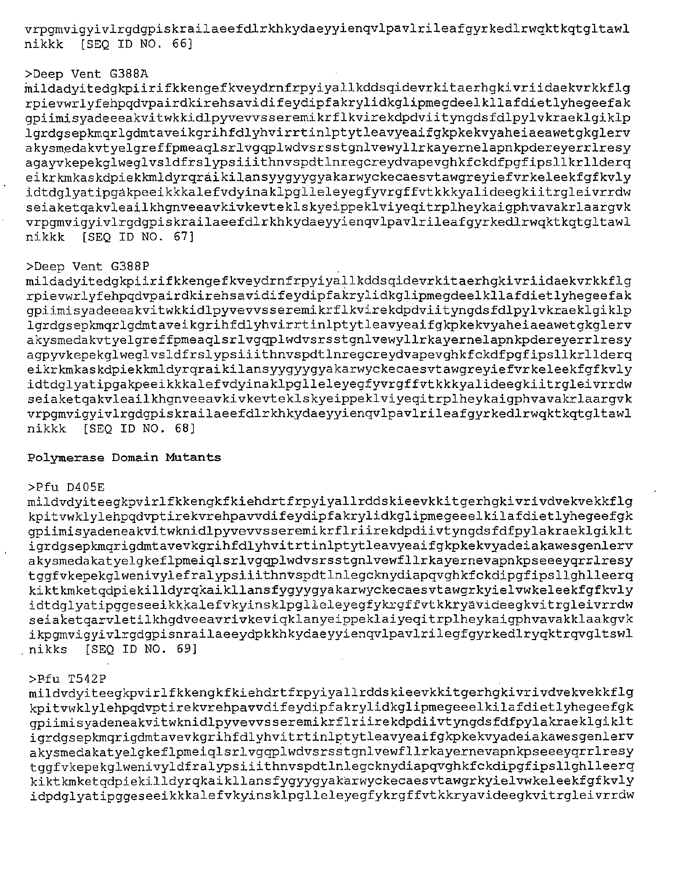

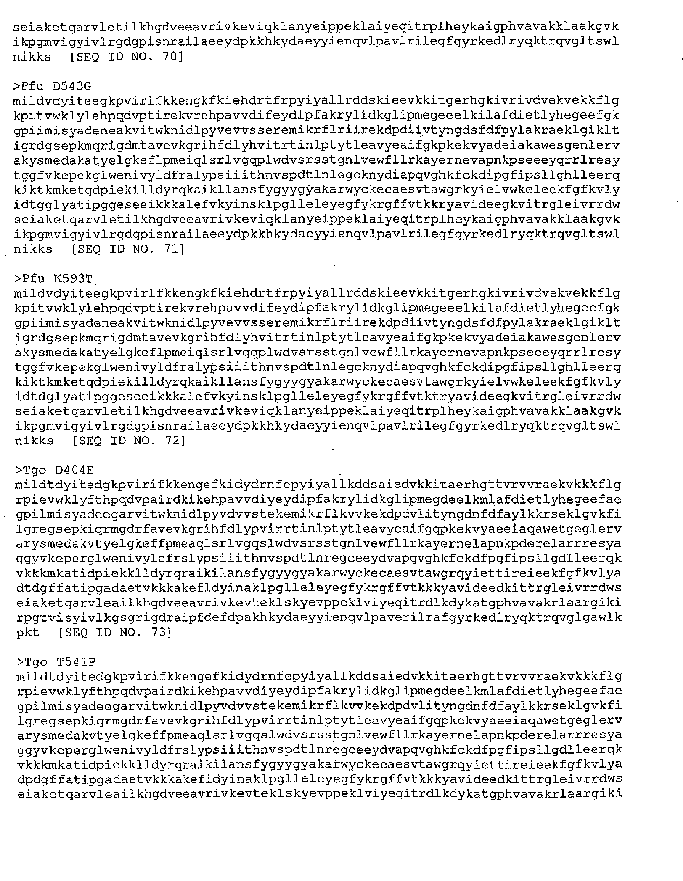

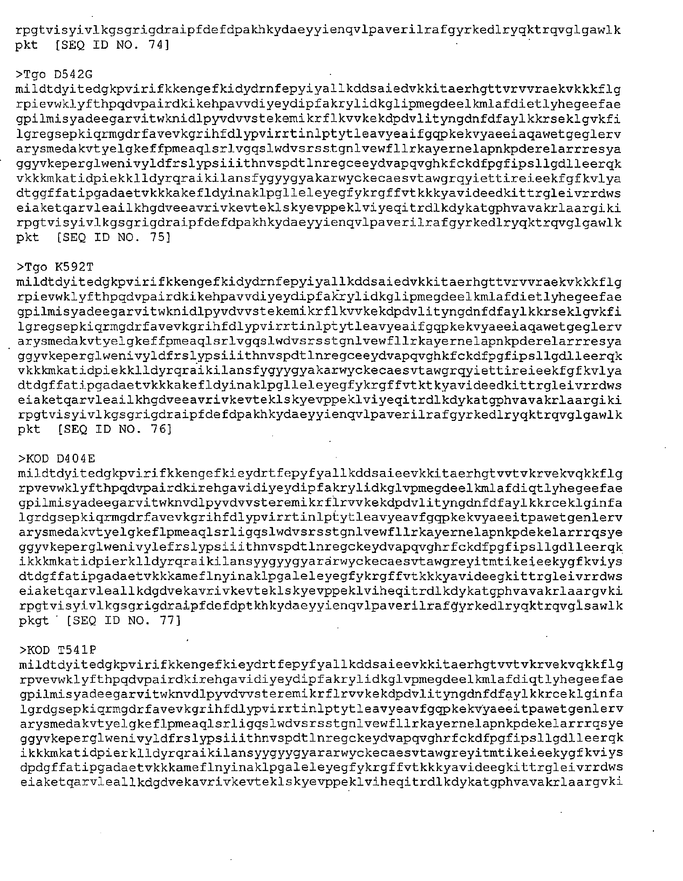

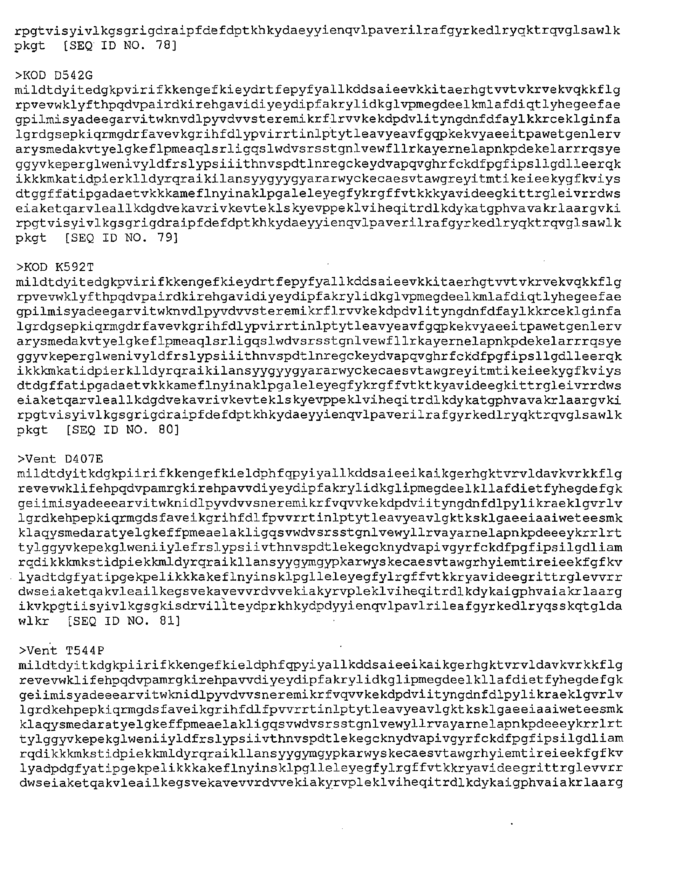

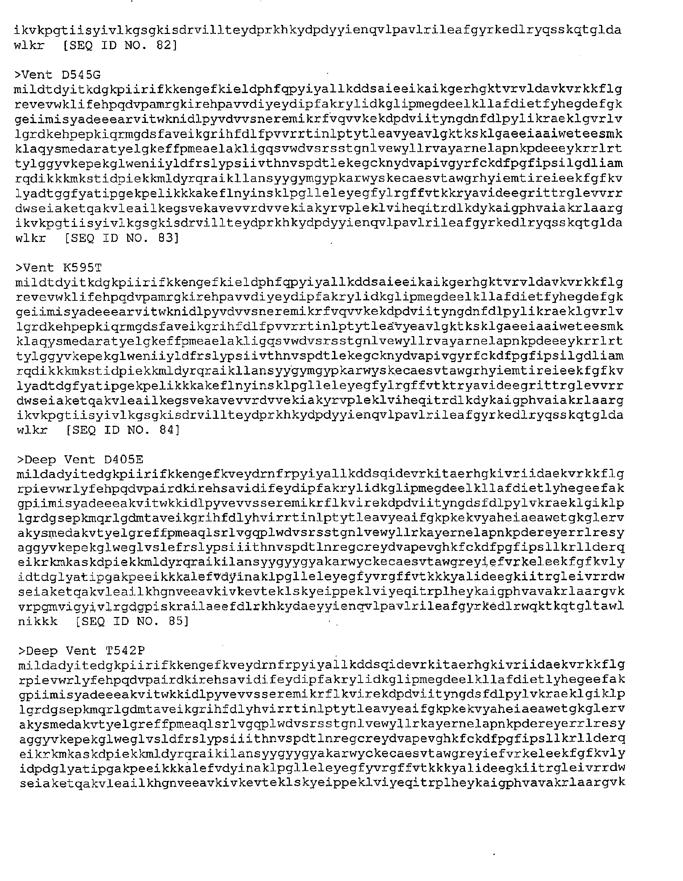

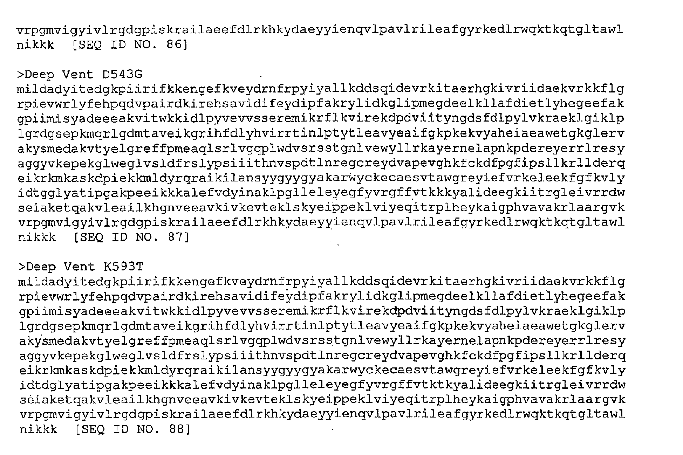

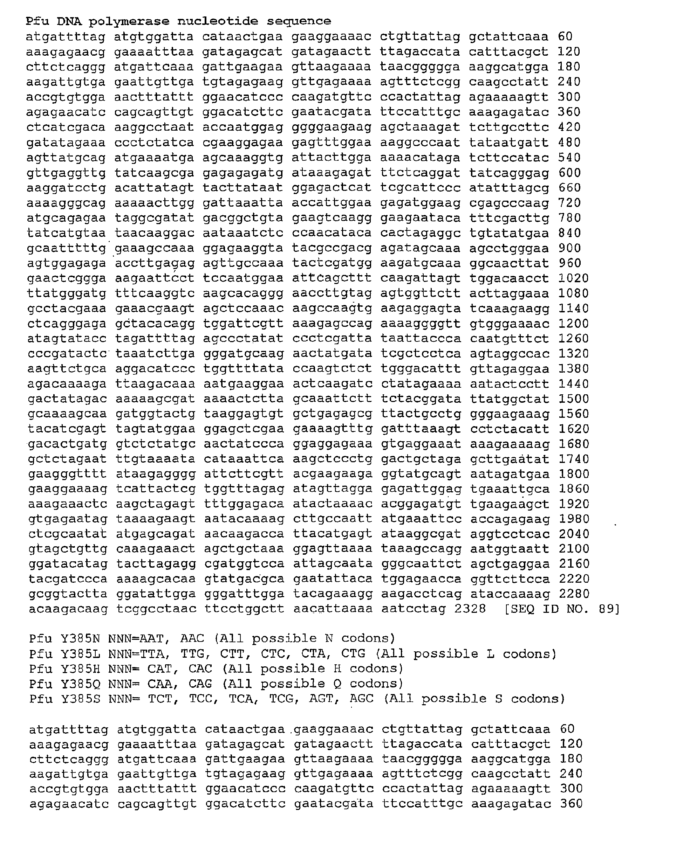

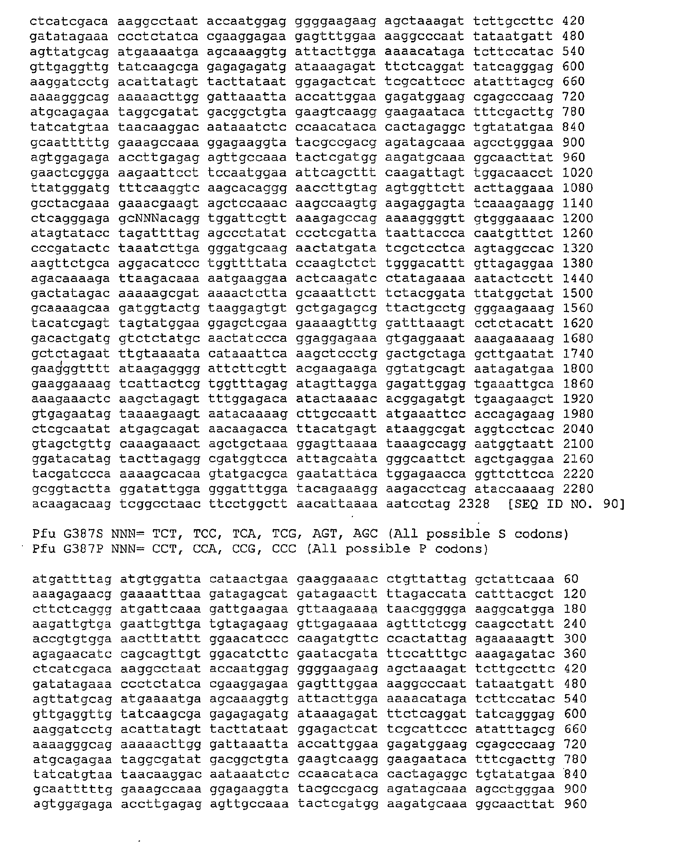

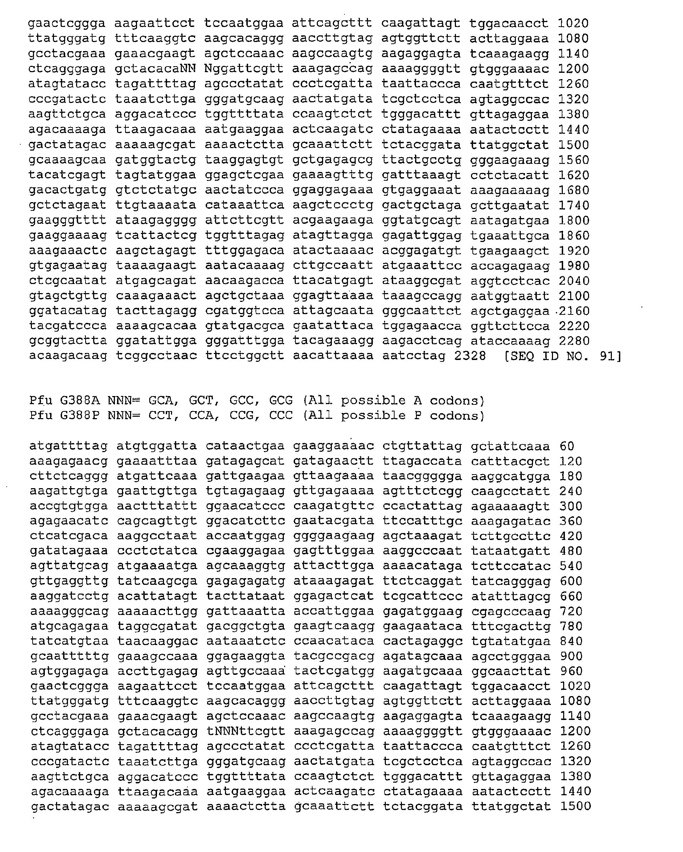

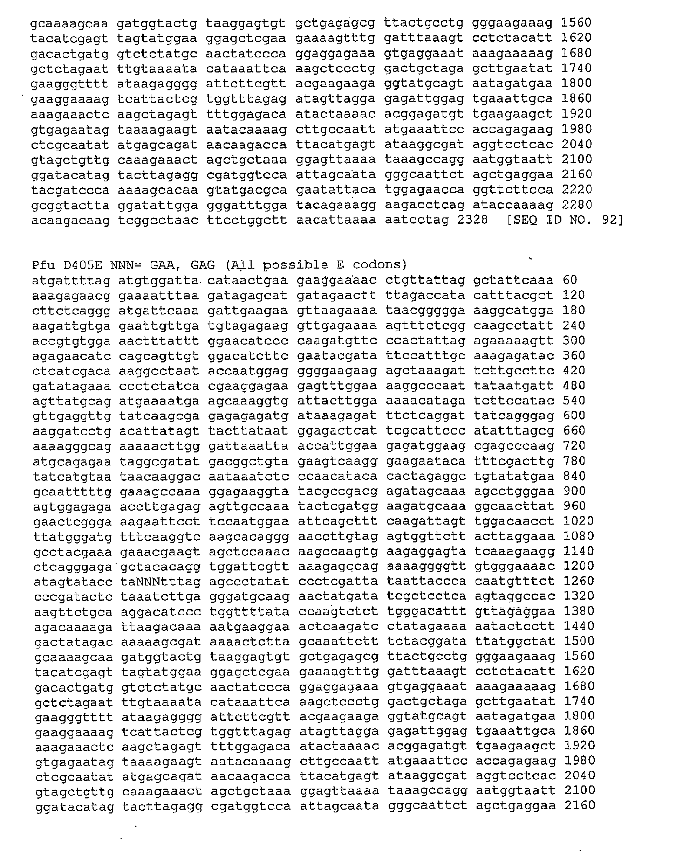

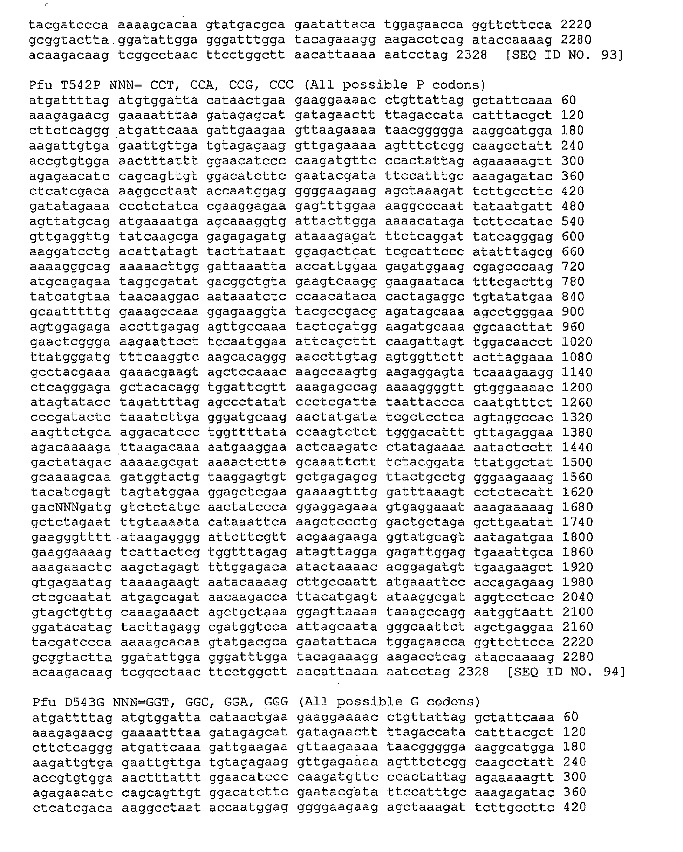

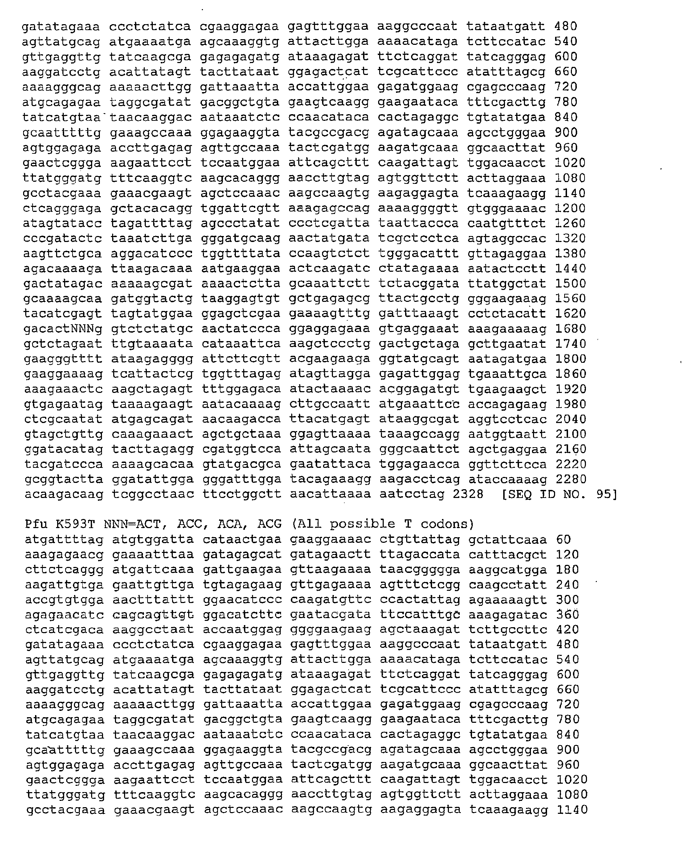

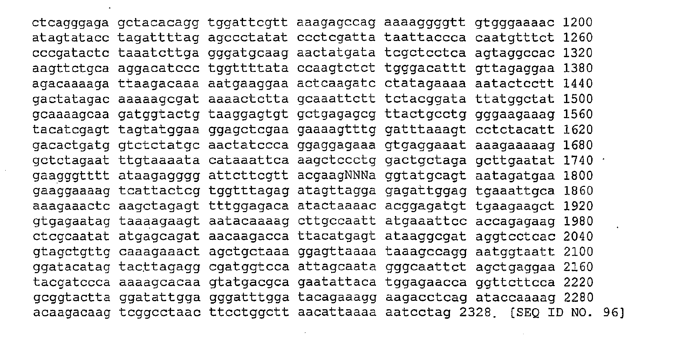

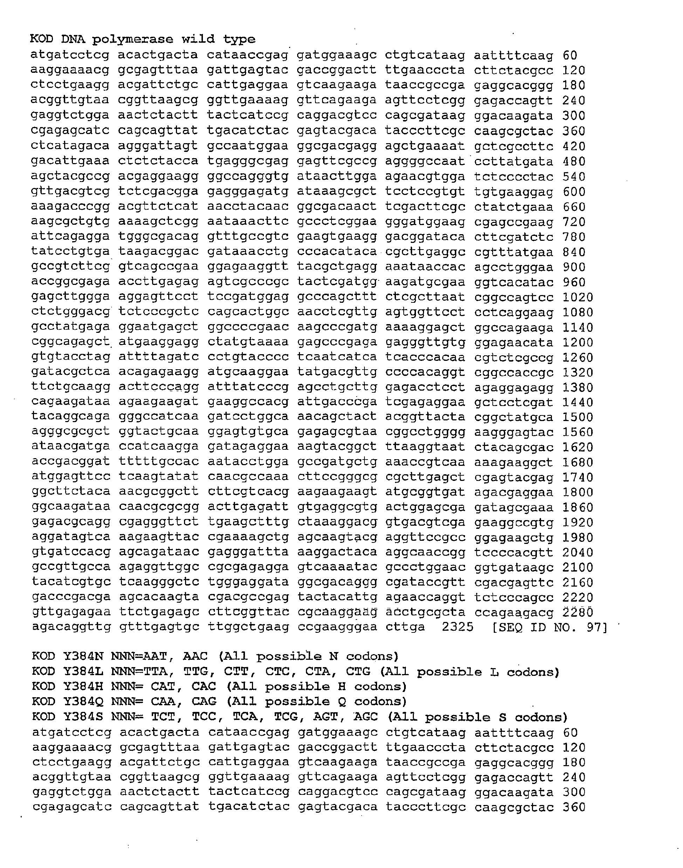

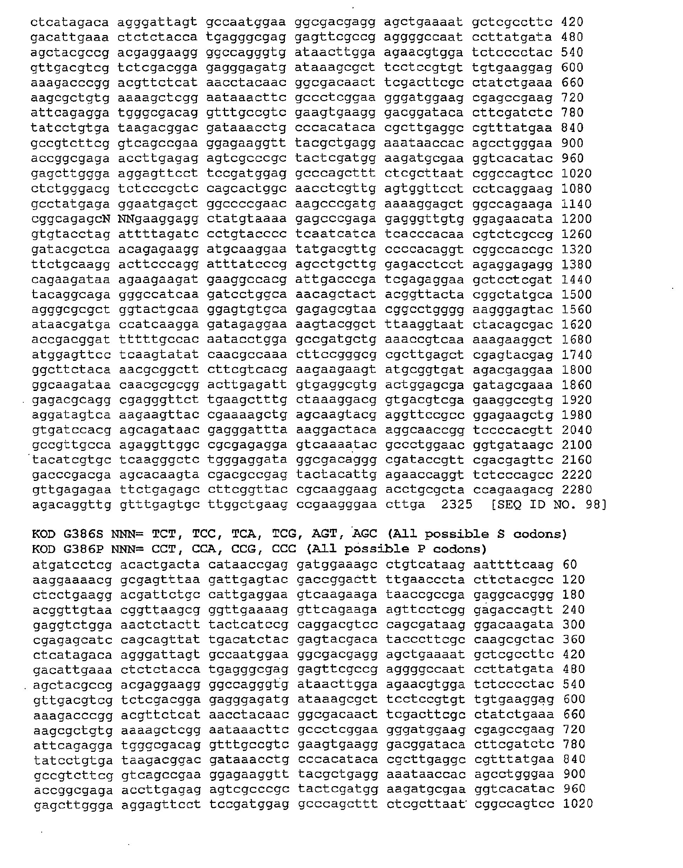

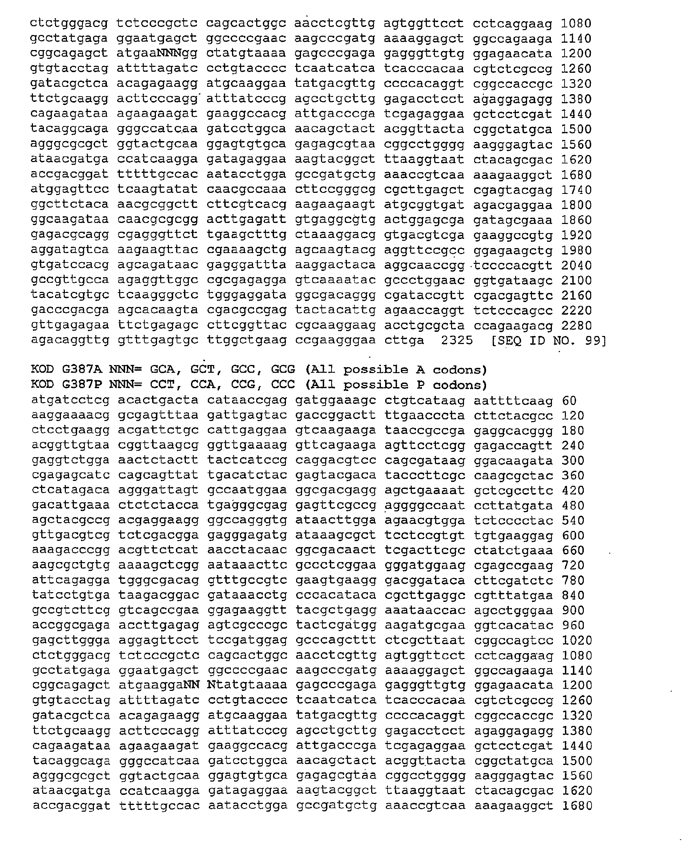

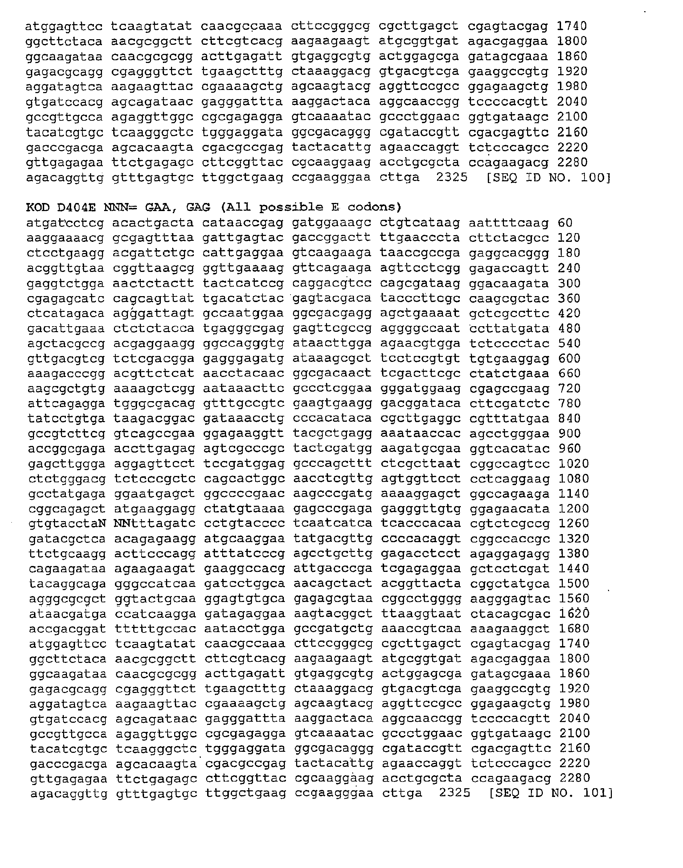

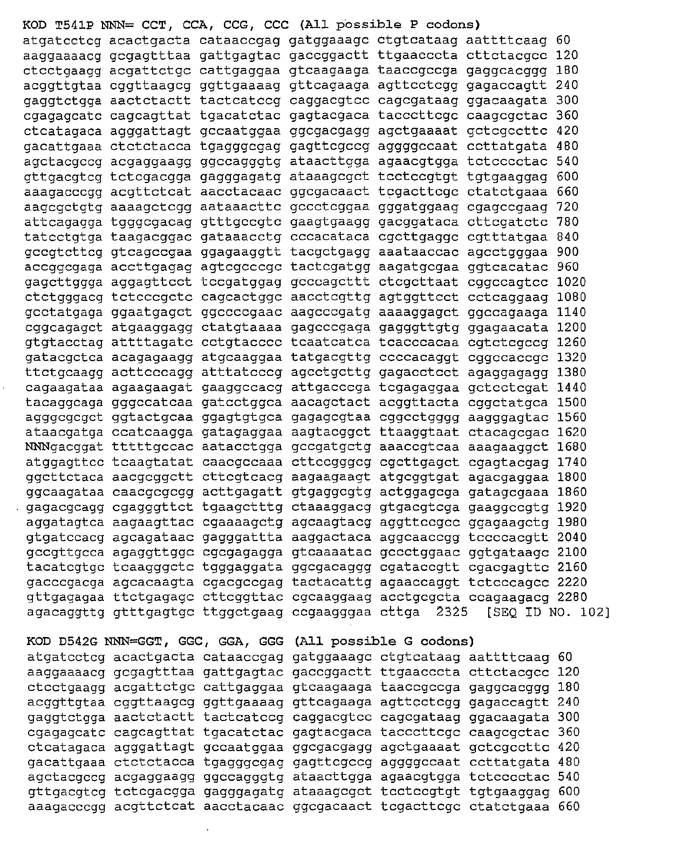

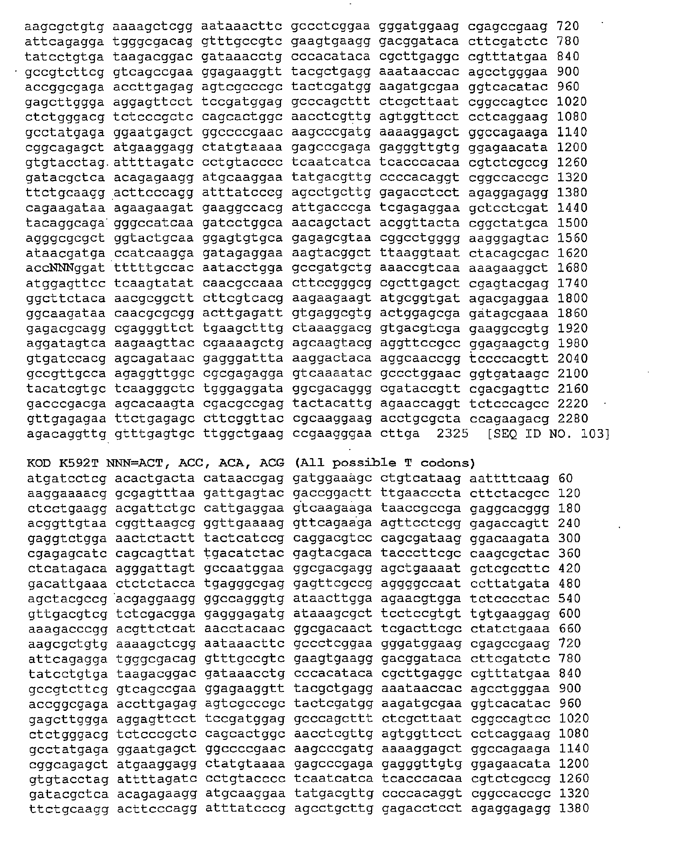

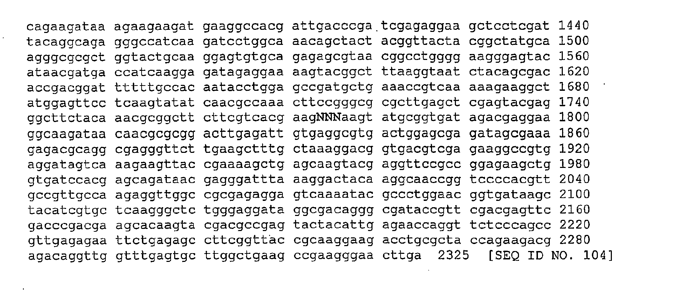

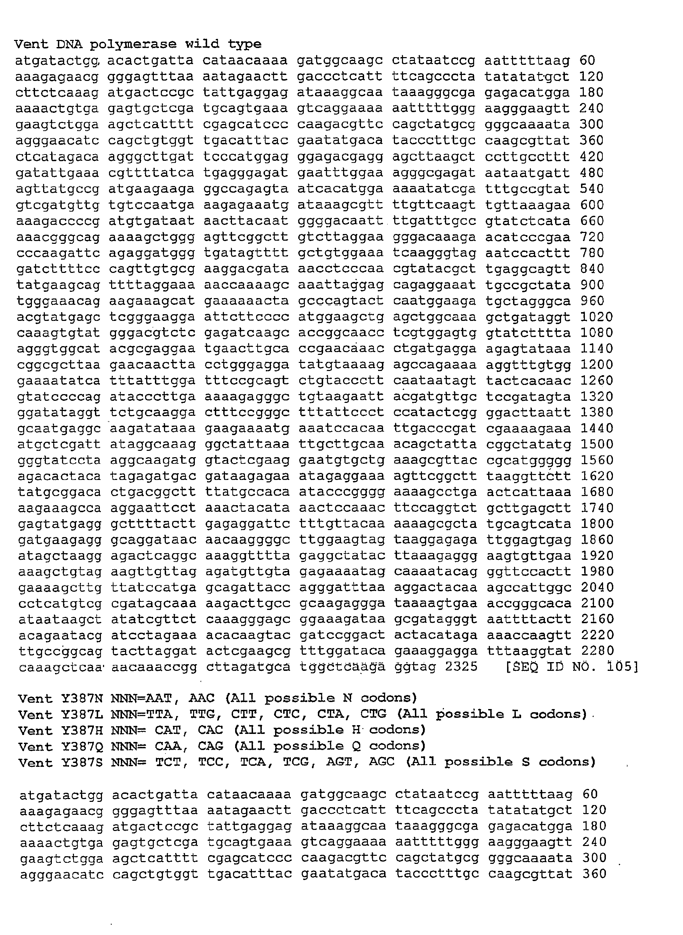

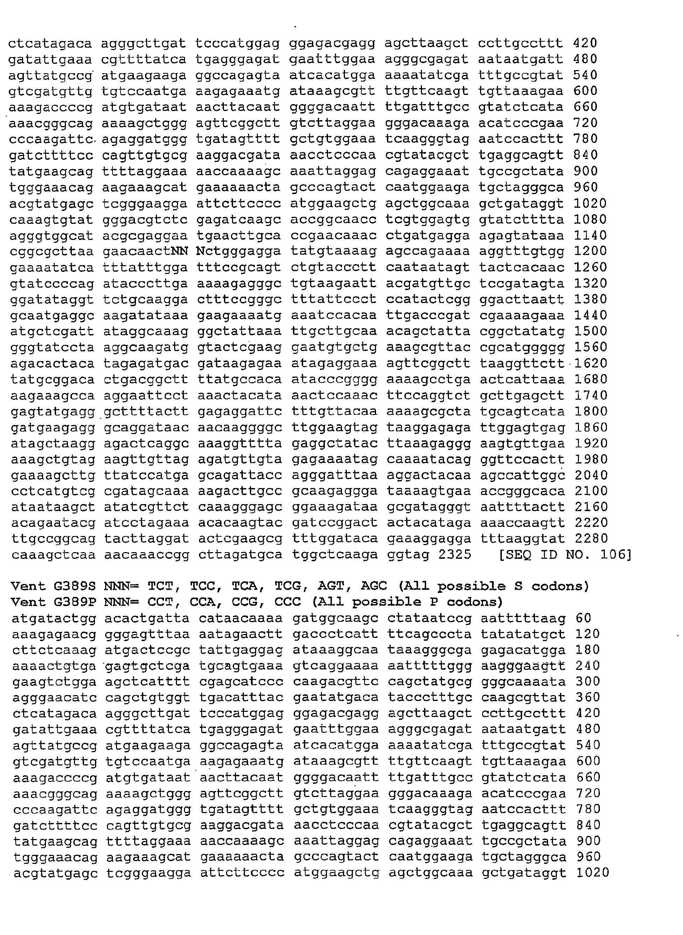

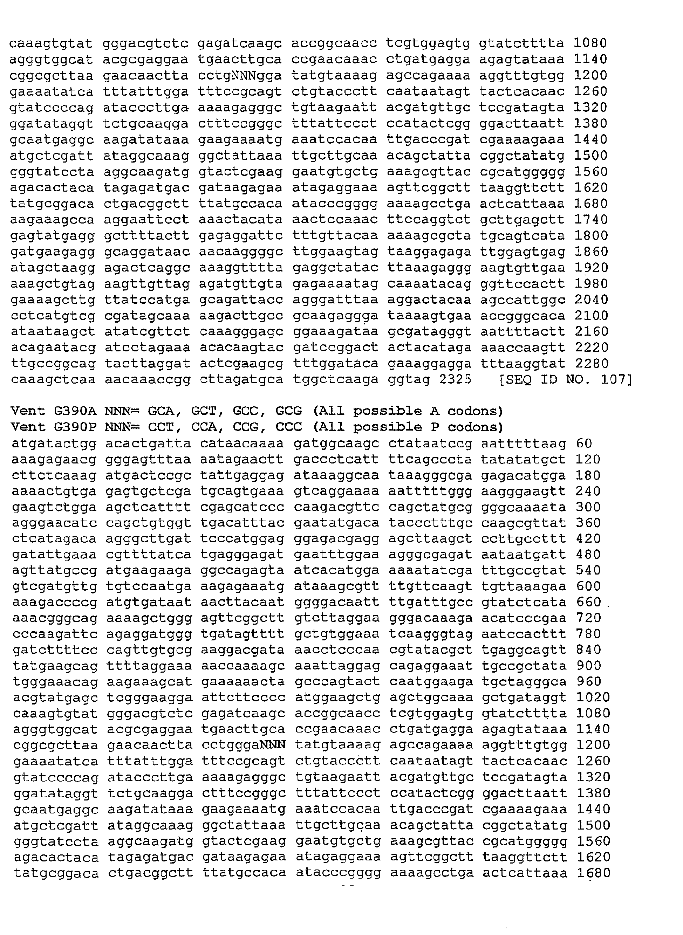

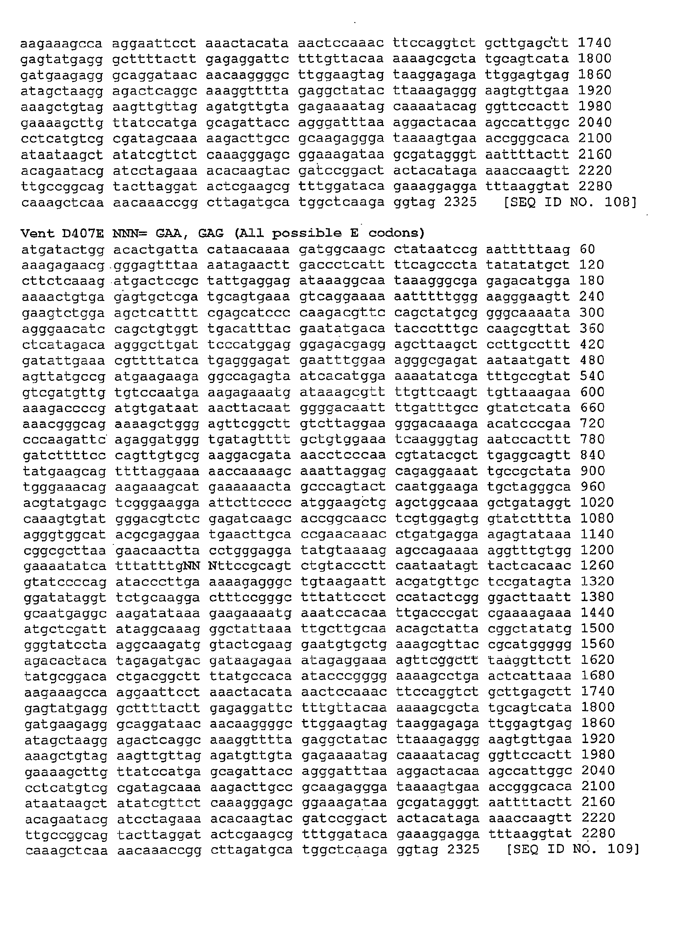

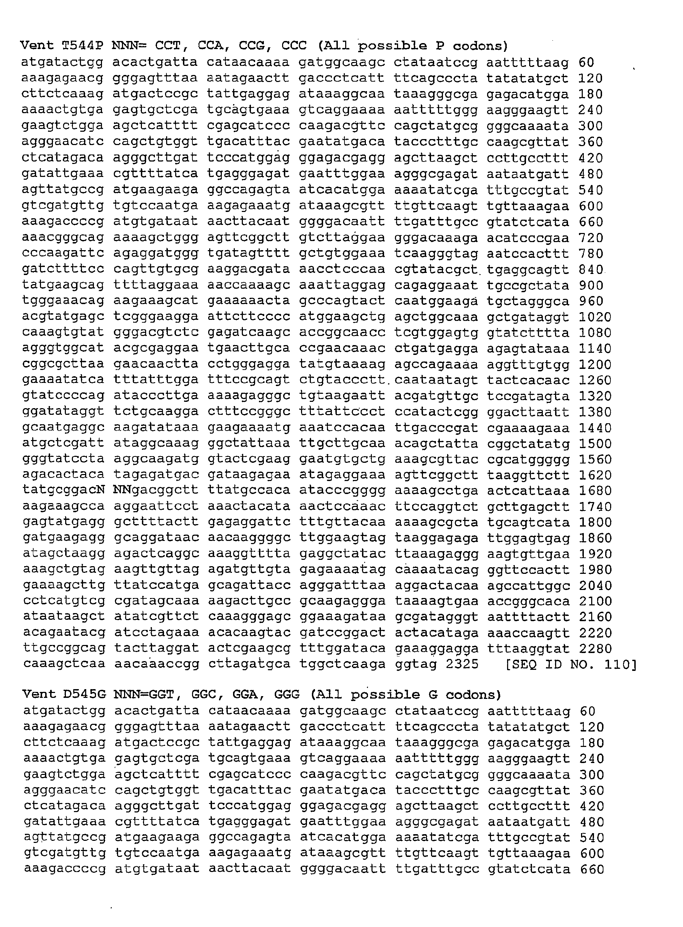

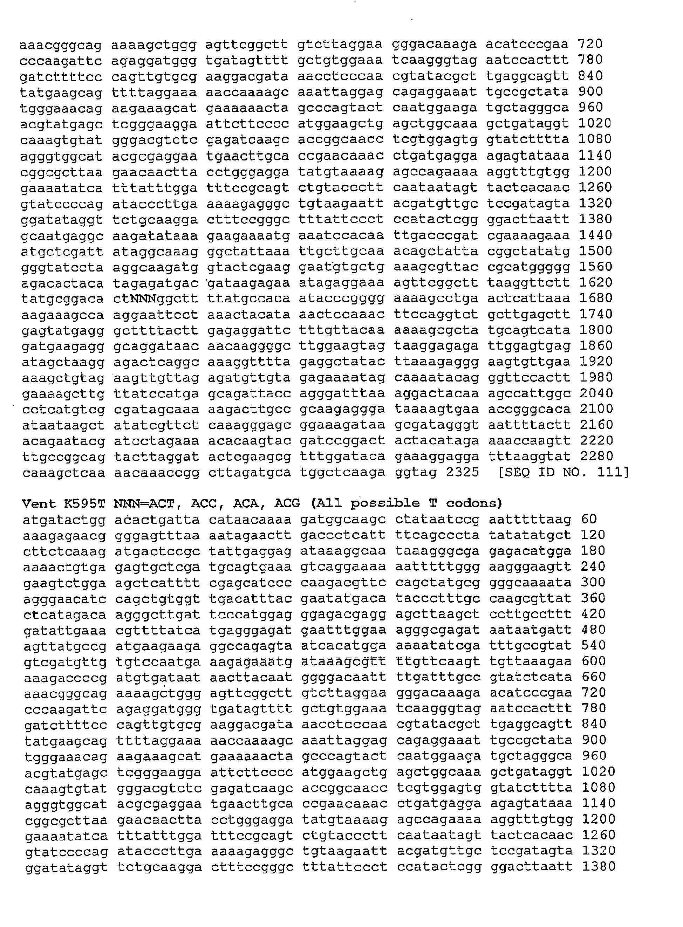

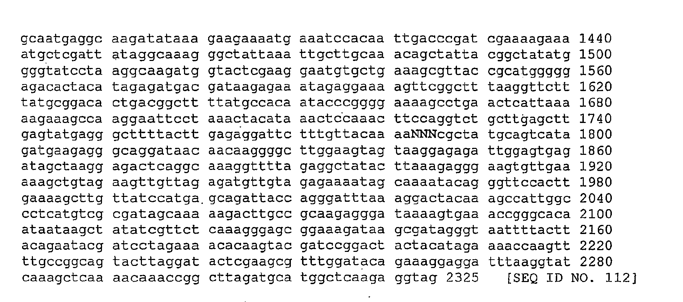

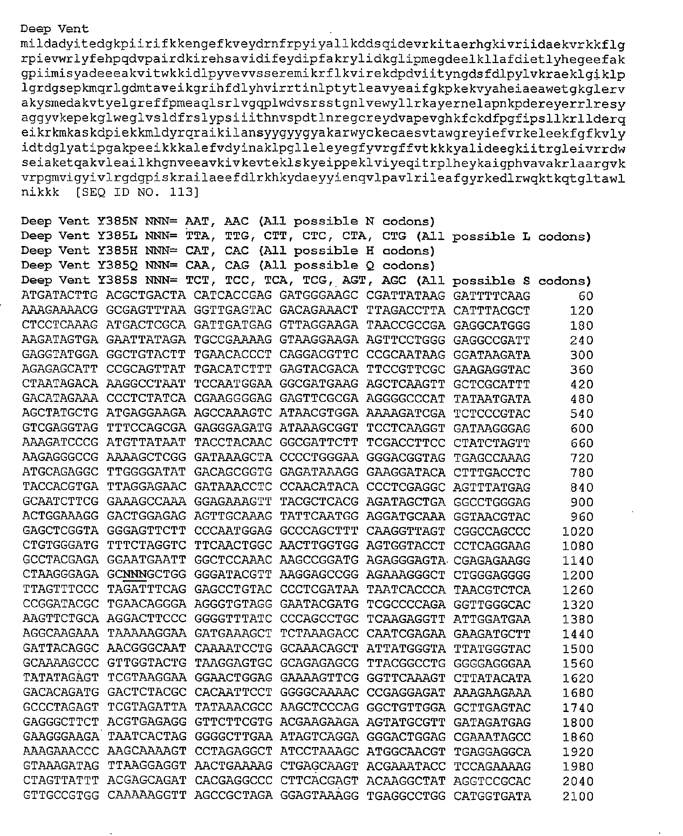

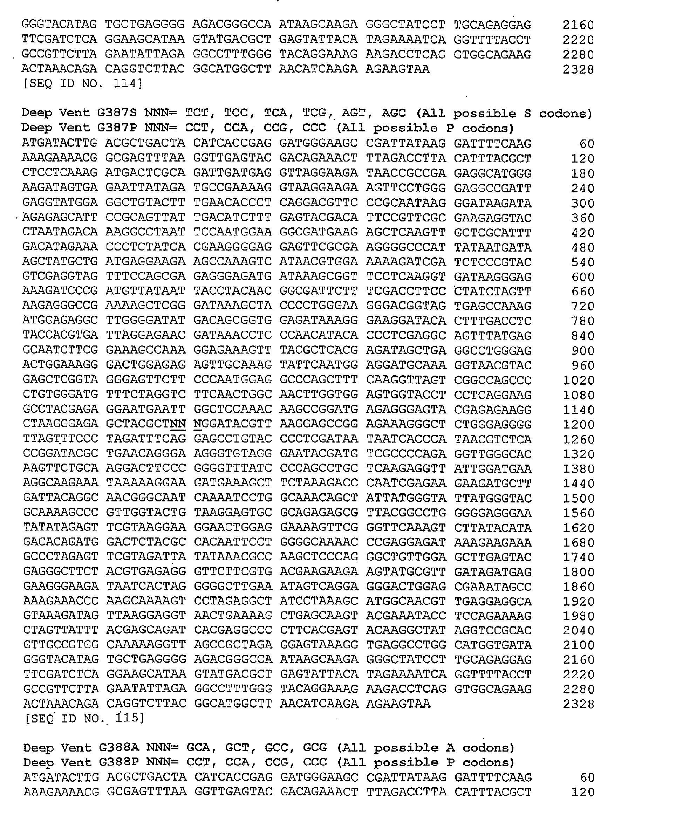

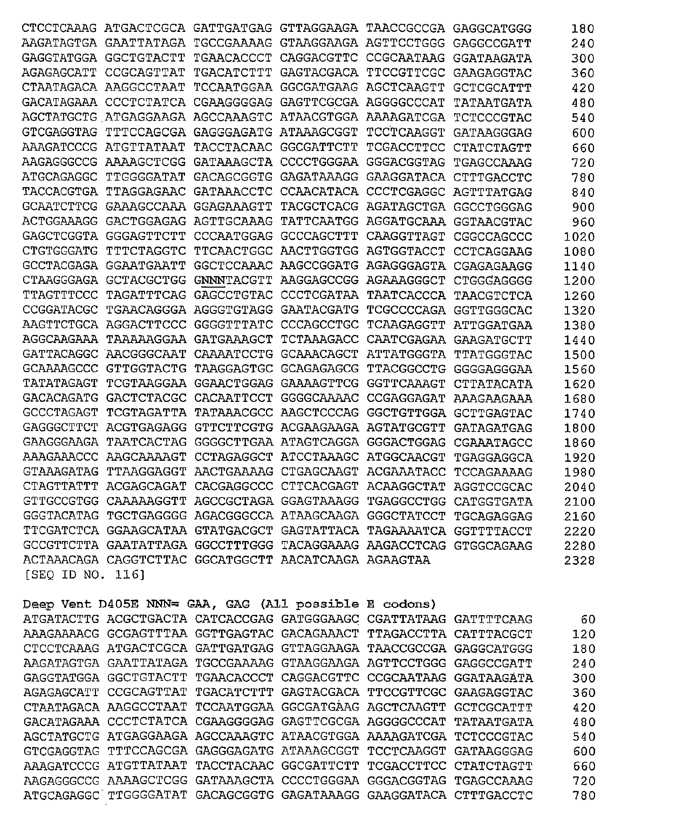

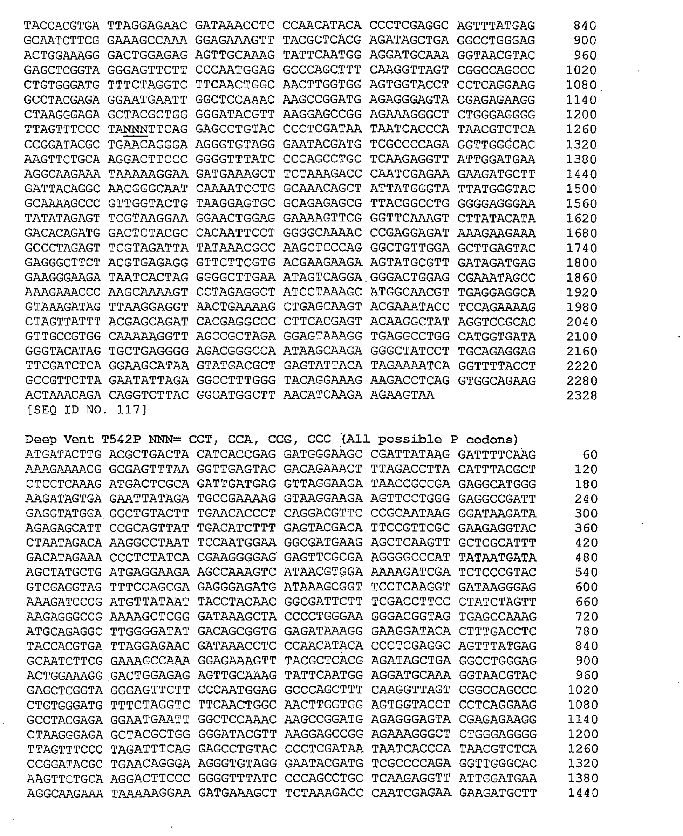

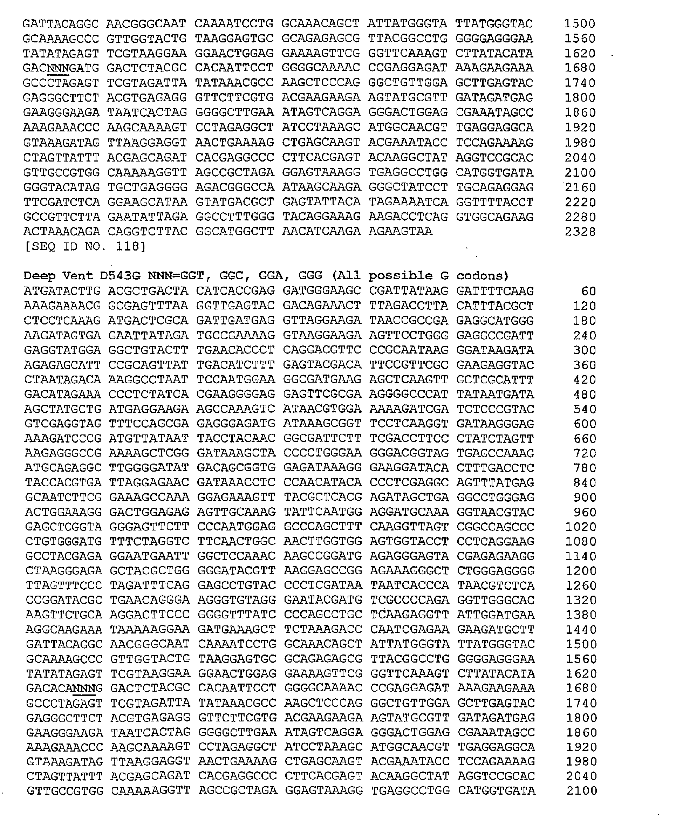

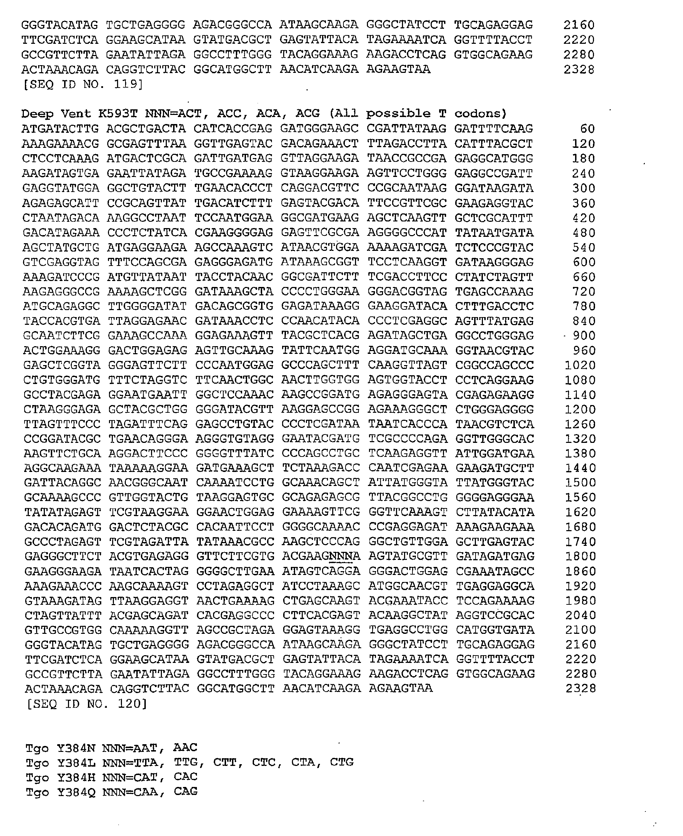

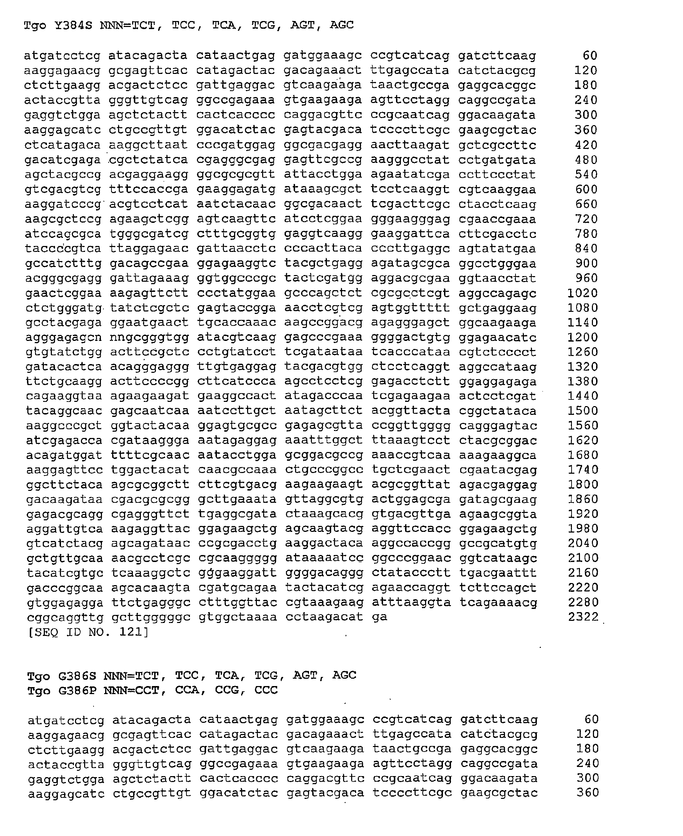

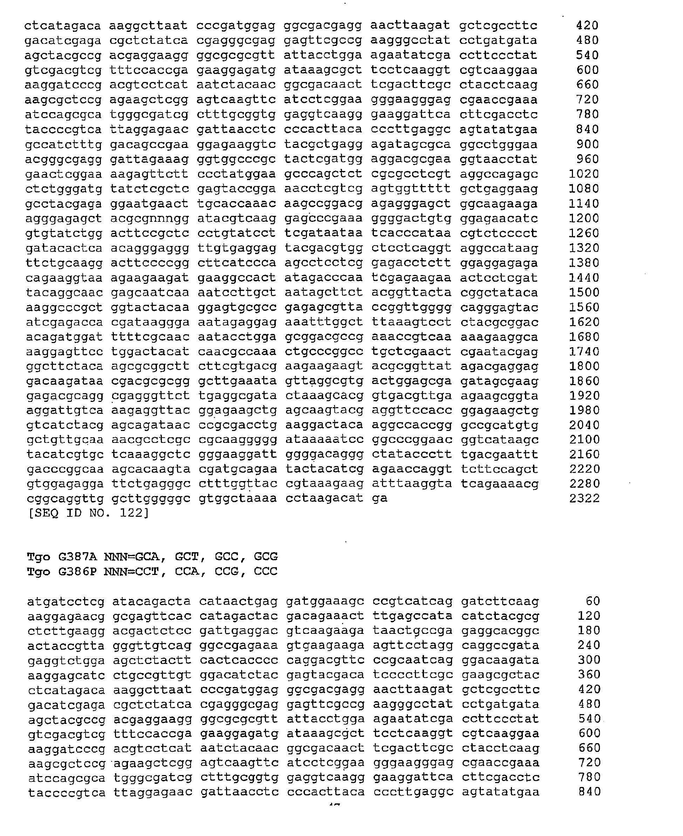

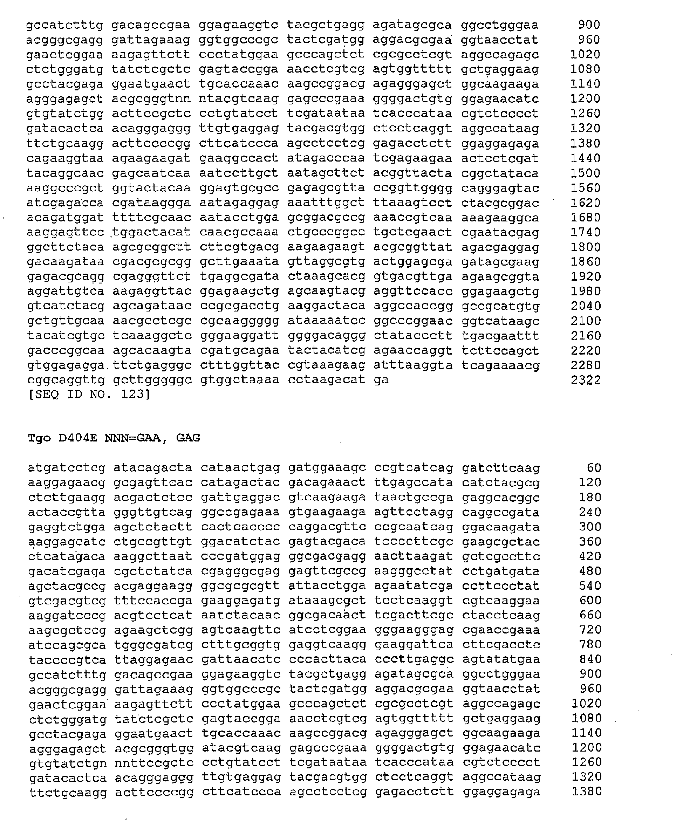

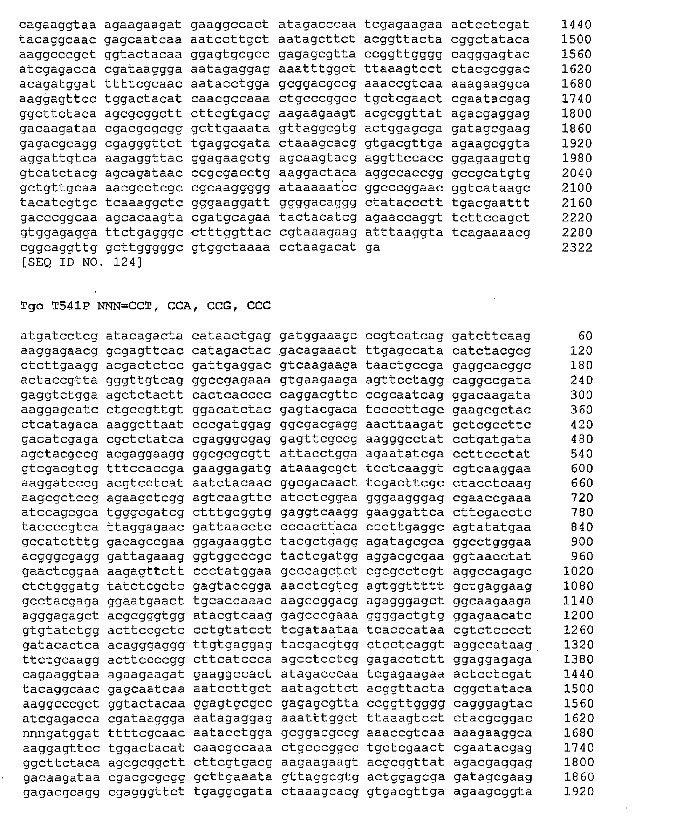

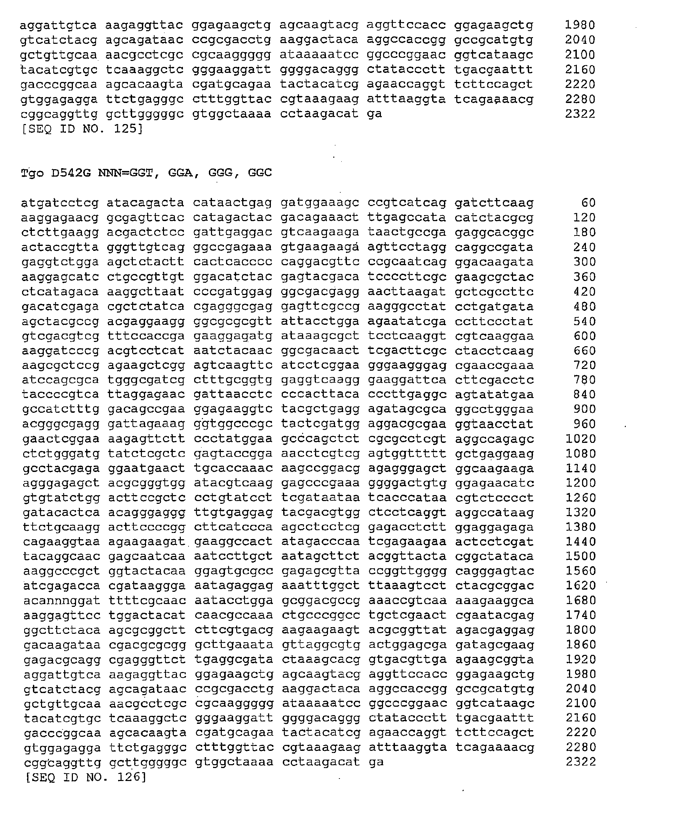

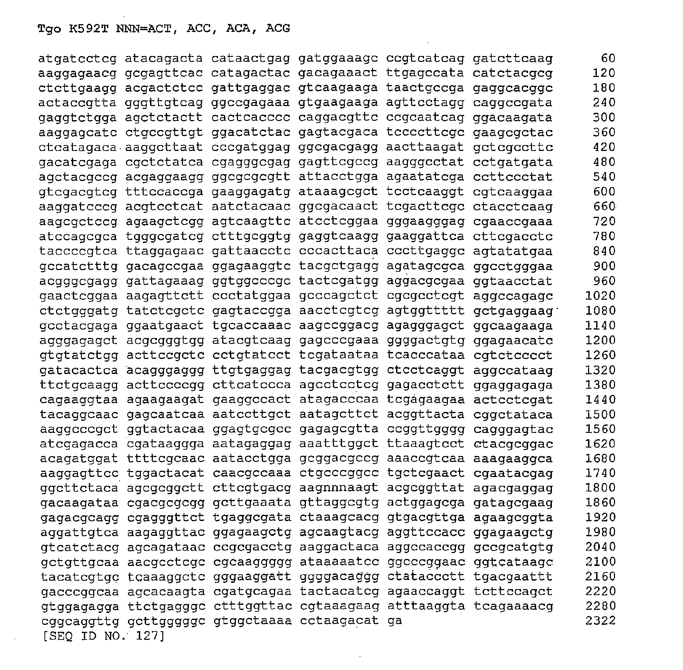

The disclosure encompasses compositions and methods in which a mutant of a related archaeal DNA polymerase is with reduced (e.g., deficient in) polymerase activity, while retaining proofreading activity. Such mutations may be within the partitioning domain or the polymerase domain of the DNA polymerases. Table 2 (A and B) and Figure 7 provides an unlimited example of such mutations in various DNA polynerases. A mutant DNA polymerase of the invention may comprise a single mutation as indicted in Table 2, or a combination of any two or more mutations.

**Table 2A Partitioning Domain Mutations in Various DNA Polymerases**

| Enzyme | Domain (bp) | Domain sequence | Predicted Mutations for Reducing DNA Polymerase Activity* | Preferred mutation |
|---|---|---|---|---|
| Pfu | 384-389 | SYTGGF | Y385, G387, G388 (Y385N, Y385L, Y385H, Y385Q, Y385S; G387S, G387P; G388A, G388P) | G387P |
| Tgo | 383-388 | SYAGGY [SEQ ID NO. 10] | Y384, G386, G387 (Y384N, Y384L, Y384H, Y384Q, Y384S; G386S, G386P; G387A, G387P) | G386P |
| KOD | 383-388 | SYEGGY [SEQ ID NO. 11] | Y384, G386, G387 (Y384N, Y384L, Y384H, Y384Q, Y384S; G386S, G386P; G387A, G387P) | G386P |
| Vent | 386-391 | TYLGGY [SEQ ID NO. 12] | Y387, G389, G390 (Y387N, Y387L, Y387H, Y387Q, Y387S; G389S, G389P; G390A, G390P) | G389P |
| DeepVent | 384-389 | SYAGGY | Y385, G387, G388 (Y385N, Y385L, Y385H, Y385Q, Y385S; G387S, G387P; G388A, G388P) | G387P |

**Table 2B Polymerase Domain Mutations in Various DNA Polymerases**

| Enzyme | Domain (bp) | Domain sequence | Predicted Mutations for Reducing DNA Polymerase Activity# |
|---|---|---|---|
| | DXXSLYP | | |
| Pfu | 405-411 | DFRALYP [SEQ ID NO. 13] | D405 (D405E) |
| Tgo | 404-410 | DFRSLYP | D404 (D404E) |
| KOD | 404-410 | DFRSLYP | D404 (D404E) |
| Vent | 407-413 | DFRSLYP | D407 (D404E) |
| Deep Vent | 405-411 | DFRSLYP | D405 (D404E) |
| | YXDTDS | | |
| Pfu | 539-544 | YIDTDG [SEQ ID NO. 14] | T542, D543 (T542P; D543G) |
| Tgo | 538-543 | | T541, D542 (T541P; D542G) |
| KOD | 538-543 | YADTDG [SEQ ID NO. 15] | T541, D542 (T541P; D542G) |

(continued)

| Enzyme | Domain (bp) | Domain sequence | Predicted Mutations for Reducing DNA Polymerase Activity# |
|---|---|---|---|
| Vent<br><br>Deep Vent | 541-546<br><br>539-544 | YSDTDG [SEQ ID NO. 16]<br>YADTDG<br>YIDTDG | T544, D545 (T544P; D545G)<br><br>T542, D543 (T542P; D543G) |
| <br>Pfu<br>Tgo<br>KOD<br>Vent<br>Deep Vent | KXY<br>593-595<br>592-594<br>592-594<br>595-597<br>593-595 | <br>KRY [SEQ ID NO. 17]<br>KKY [SEQ ID<br><br>NO. 18]<br>KKY<br>KRY<br>KKY | <br>K593 (K593T)<br>K592 (K592T)<br>K592 (K592T)<br>K595 (K595T)<br>K593 (K593T) |
| #alternative side chain substitutions at key positions are also expected to reduce polymerase activity | | | |

B. Methods of Evaluating Mutants for Reduced Polymerization

Random or site-directed mutants generated as known in the art or as described herein and expressed in bacteria may be screened for reduced polymerization by several different assays. Embodiments for the expression of mutant and wild type enzymes is described herein below in section C. In one method, $exo^+$ DNA polymerase proteins expressed in lytic lambda phage plaques generated by infection of host bacteria with expression vectors based on, for example, Lambda ZapII®, are transferred to a membrane support. The immobilized proteins are then assayed for polymerase activity on the membrane by immersing the membranes in a buffer containing a DNA template and the unconventional nucleotides to be monitored for incorporation.

Mutant polymerase libraries may be screened using a variation of the technique used by Sagner et al (Sagner, G., Ruger, R., and Kessler, C. (1991) Gene 97:119-123). For this approach, lambda phage clones are plated at a density of 10-20 plaques per square centimeter. Proteins present in the plaques are transferred to filters and moistened with polymerase screening buffer (50mM Tris (pH 8.0), 7mM MgCl2, 3mM β-ME). The filters are kept between layers of plastic wrap and glass while the host cell proteins are heat-inactivated by incubation at 65°C for 30 minutes. The heat-treated filters are then transferred to fresh plastic wrap and approximately 351 of polymerase assay cocktail are added for every square centimeter of filter. The assay cocktail consists of 1X cloned Pfu (cPfu) magnesium free buffer (1X buffer is 20 mM Tris-HCl (pH 8.8), 10 mM KCl, 10 mM $(NH4)_2SO_4$, 100 μg/ml bovine serum albumin (BSA), and 0.1% Triton X-100; Pfu Magnesium-free buffer may be obtained from Stratagene (Catalog No. 200534)), 125 ng/ml activated calf thymus or salmon sperm DNA, 1.29 μCi/ml $\alpha$-$^{33}$P ddNTP. The filters are placed between plastic wrap and a glass plate and then incubated at 65°C for one hour, and then at 70°C for one hour and fifteen minutes. Filters are then washed three times in 2X SSC for five minutes per wash before rinsing twice in 100% ethanol and vacuum drying. Filters are then exposed to X-ray film (approximately 16 hours), and plaques that incorporate label are identified by aligning the filters with the original plate bearing the phage clones. Plaques identified in this way are re-plated at more dilute concentrations and assayed under similar conditions to allow the isolation of purified plaques.

In assays such as the one described above, the signal generated by the label is a direct measure of the polymerization activity of the polymerase. A plaque comprising a mutant DNA polymerase with reduced DNA polymerization activity compared to that of the wild-type enzyme can be selected.

Incorporation of nucleotides may also be measured in extension reactions by adding, for example, 1 μl of appropriately diluted bacterial extract (i.e., heat-treated and clarified extract of bacterial cells expressing a cloned polymerase or mutated cloned polymerase) to 10 μl of each nucleotide cocktail, followed by incubation at the optimal temperature for 30 minutes (e.g., 73°C for Pfu DNA polymerase), for example, as described in Hogrefe et al., 2001, Methods in Enzymology, 343:91-116. Extension reactions are quenched on ice, and then 5μl aliquots are spotted immediately onto DE81 ion-exchange filters (2.3cm; Whatman #3658323). Unincorporated label is removed by 6 washes with 2xSCC (0.3M NaCl, 30mM sodium citrate, pH 7.0), followed by a brief wash with 100% ethanol. Incorporated radioactivity is then measured by scintillation counting. Reactions that lack enzyme are also set up along with sample incubations to determine "total cpms" (omit filter wash steps) and "minimum cpms"(wash filters as above). Cpms bound is proportional to the amount of polymerase activity present per volume of bacterial extract.

A Non-limiting method for determining polymerization activity of a DNA polymerase mutant relative to wild type (wt) is provided as follows. Relative percent radioactivity incorporated which indicates the relative polymerization activity of a DNA polymerase mutant can be determined as:

$$\frac{\text{(corrected cpms for mutant DNA polymerase) x (ng wt DNA polymerase)}}{\text{(corrected cpms for wt DNA polymerase) x (ng mutant DNA polymerase)}}.$$

To more precisely quantify % activity, one should covert cpms incorporated into units of DNA polymerase activity. One unit of polymerase activity is defined as the amount of enzyme that catalyzes the incorporation of 10nmoles of total dNTP into polymeric form (e.g., binds to DE-81 paper) in 30 minutes at optimal temperature. Units of DNA polymerase activity can be calculated using the following equation:

$$\frac{\text{(corrected sample cpms)}}{\text{total cpms}} \text{ x } \frac{\text{(8nmoles dNTPs)}}{\text{reaction}} \text{ x } \frac{\text{(1 unit)}}{\text{(10nmoles dNTPs incorporated)}}$$

Polymerase specific activity (U/mg) can be extrapolated from the slope of the linear portion of units versus enzyme amount plots. Protein concentrations can be determined relative to a BSA standard (Pierce) in a colorimetric assay (e.g. Pierce's Coomassie Plus Protein Assay). Alternatively, when protein amounts are limiting (or for preparations of limited purifty), relative protein concentrations can be verified by SDS-PAGE analysis. Several aliquots of each DNA polymerase preparation, ranging from 1-20 ng of total protein, are subject to SDS-PAGE electrophoresis and the intensity of silver- and/or Sypro orange (Molecular Probes)-stained bands are compared to standards. Finally, % activity can be determined as:

$$\frac{\text{specific polymerase activity (U/mg) of mutant DNA polymerase}}{\text{specific polymerase activity (U/mg) of wt DNA polymerase}}$$

It is preferred that the polymerases with reduced polymerization activity of the present invention maintain their proofreading activities (i.e., 3'-5' exonuclease activities). The mutant DNA polymerases with reduced DNA polymerization activities, therefore, are also assayed for 3'-5- exonuclease activities.

Suitable exonuclease activity assays include one described in Hogrefe et al (supra, and as described in Example 3). Another assay employs double-stranded λ DNA, which has been uniformly labeled with $^3H$ S-adenosyl methionine (NEN #NET-155) and *Sss* I methylase (NEB), and then restriction digested with *Pal* I (Kong et al., 1993, J. Biol. Chem. 268:1965). Using double-stranded labeled DNA templates, one can determine specificity by measuring whether cpms decrease (3'-5' exonuclease) with the addition of dNTPs (10-100μM). A typical exonuclease reaction cocktail consists of 1x reaction buffer and 20μg/ml $^3H$-labeled digested double-stranded λ DNA (~$10^6$ cpms/ml), prepared as described (Kong et al., supra). Exonuclease activity can be measured in the appropriate PCR buffer or in a universal assay buffer such as 70mM Tris HCl (pH 8.8), 2mM $MgCl_2$, 0.1% Triton-X, and 100μg/ml BSA.

Percent exonuclease activity can be determined as: (corrected cpms for mutants)/(corrected cpms for wt DNA polymerase). To more precisely quantify % activity, cpms released can be converted into units of exonuclease activity. One unit of exonuclease activity is defined as the amount of enzyme that catalyzes the acid-solubilization of 10nmoles of total dNMPs in 30 minutes at a defined temperature. To determine units, background (average "minimum cpms" value) is first subtracted from the average sample cpms. Nmoles dNMPs released is calculated using the following equation:

$$\frac{\text{(corrected sample cpms)}}{\text{total cpms}} \text{ x } \frac{\text{(ng DNA)}}{\text{reaction}} \text{ x } \frac{\text{(1nmole dNMP)}}{\text{(330ng dNMP)}}$$

Units of exonuclease activity (in 30 minutes) can then be determined as:

$$\frac{(\text{nmoles dNMPs released per hr})}{2} \times \frac{(1\ \text{unit})}{(10\text{nmoles dNMPs released})}$$

.

Exonuclease specific activity (U/mg) can be extrapolated from the slope of the linear portion of units versus enzyme amount plots. Finally, % activity can be determined as:

$$\frac{\text{specific exonuclease activity (U/mg) of mutant DNA polymerase}}{\text{specific exonuclease activity (U/mg) of wt DNA polymerase}}$$

.

In addition to the substrate described above, exonuclease activity can be also be quantified using [$^3$H]-*E. coli* genomic DNA (NEN #NET561; 5.8μCi/μg), a commercially-available substrate. A typical exonuclease reaction cocktail consists of 25ng/ml $^3$H-labeled *E. coli* genomic DNA and 975 ng/ml cold E. coli genomic DNA in 1x reaction buffer. Assays are performed as described above.

Genes for desired mutant DNA polymerases generated by mutagenesis may be sequenced to identify the sites and number of mutations. For those mutants comprising more than one mutation, the effect of a given mutation may be evaluated by introduction of the identified mutation to the wild-type gene by site-directed mutagenesis in isolation from the other mutations borne by the particular mutant. Screening assays of the single mutant thus produced will then allow the determination of the effect of that mutation alone.

In one embodiment, the Pfu mutant is G387P, which reduces the error rate of wild type Pfu DNA polymerase by 3-fold in a Pfu blend when added at 5-25ng/50μl reaction. The Pfu G387P mutant also reduces the error rate of Taq by approximately 5- to 10-fold in a blend when added at 6/60ng/50μl reaction. Pfu G387P exhibited 0.4% DNA polymerase activity and 57% exonuclease activity (i.e., relative to wild type Pfu) in a preliminary screen of partially purified (~50% purity) His-tagged proteins, eluted from nickel columns (Table 1). After column chromatography (~95% purity), the His-tagged Pfu G387P mutant was found to be devoid of detectable DNA polymerase activity (<0.01% activity relative to wild type Pfu) (Table 3).

C. Expression of Wild-type or Mutant enzymes According to the Invention

Methods known in the art may be applied to express and isolate the mutated forms of DNA polymerase (i.e., the second enzyme) according to the invention. The methods described here can be also applied for the expression of wild-type enzymes useful (e.g., the first enzyme) in the invention. Many bacterial expression vectors contain sequence elements or combinations of sequence elements allowing high level inducible expression of the protein encoded by a foreign sequence. For example, as mentioned above, bacteria expressing an integrated inducible form of the T7 RNA polymerase gene may be transformed with an expression vector bearing a mutated DNA polymerase gene linked to the T7 promoter. Induction of the T7 RNA polymerase by addition of an appropriate inducer, for example, isopropyl-β-D-thiogalactopyranoside (IPTG) for a lac-inducible promoter, induces the high level expression of the mutated gene from the T7 promoter.

Appropriate host strains of bacteria may be selected from those available in the art by one of skill in the art. As a non-limiting example, E. coli strain BL-21 is commonly used for expression of exogenous proteins since it is protease deficient relative to other strains of E. coli. BL-21 strains bearing an inducible T7 RNA polymerase gene include WJ56 and ER2566 (Gardner & Jack, 1999, supra). For situations in which codon usage for the particular polymerase gene differs from that normally seen in E. coli genes, there are strains of BL-21 that are modified to carry tRNA genes encoding tRNAs with rarer anticodons (for example, argU, ileY, leuW, and proL tRNA genes), allowing high efficiency expression of cloned protein genes, for example, cloned archaeal enzyme genes (several BL21-CODON PLUSTM cell strains carrying rare-codon tRNAs are available from Stratagene, for example).

There are many methods known to those of skill in the art that are suitable for the purification of a modified DNA polymerase of the invention. For example, the method of Lawyer et al. (1993, PCR Meth. & App. 2: 275) is well suited for the isolation of DNA polymerases expressed in E. coli, as it was designed originally for the isolation of Taq polymerase. Alternatively, the method of Kong et al. (1993, J. Biol. Chem. 268:1965) may be used, which employs a heat denaturation step to destroy host proteins, and two column purification steps (over DEAE-Sepharose and heparin-Sepharose columns) to isolate highly active and approximately 80% pure DNA polymerase. Further, DNA polymerase mutants may be isolated by an ammonium sulfate fractionation, followed by Q Sepharose and DNA cellulose columns, or by adsorption of contaminants on a HiTrap Q column, followed by gradient elution from a HiTrap heparin column.

In one embodiment, the Pfu mutants are expressed and purified as described in U.S. Patent No. 5,489,523.

D. Other Methods For Reducing Polymerization Activity

In order to prevent the side effects of having a high DNA polymerization activity in an amplification reaction, the polymerization activity of the composition of the invention may also be reduced by physical and/or chemical modification and/or inhibition.

The polymerization activity of the subject composition may be reduced by chemical and/or physical means. Conditions which preferentially inhibit the polymerization activity of a DNA polymerase is known in the art (for reviews, see Johnson, 1993, supra; Wright, 1996, Acta Biochim Pol. 43:115-24; Elion, 1982, Am J Med., 73:7-13). The level ofpolymerization activity need only be reduced to that level of activity which does not interfere with amplification reactions (e.g., does not significantly affect the $exo^+$ activity of the composition or the efficiency yield of the amplification reaction).

Concentrations of $Mg^{2+}$ greater than 5 mM inhibit the polymerization activity of the Pfu DNA polymerase. The effect of a given concentration of $Mg^{2+}$ for a given DNA polymerase may be determined by quantitation of the efficiency and specificity of polymerization.

The inhibitory effect of other ions, polyamines, denaturants, such as urea, formamide, dimethylsulfoxide, glycerol and non-ionic detergents (Triton X-100 and Tween-20), polynucleotide binding chemicals such as, actinomycin D, ethidium bromide and psoralens, may be tested by their addition to the standard reaction buffers for polynucleotide amplificaiton (e.g., PCR). Those compounds having a preferential inhibitory effect on the polymerization activity but not significantly affecting the 3'-5' exonuclease activity of a DNA polymerase are then used to create reaction conditions under which 3'-5' nuclease activity is retained while polymerization activity is reduced.

Physical means may be used to preferentially inhibit the polymerization activity of a polymerase. For example, the polymerization activity of thermostable polymerases is destroyed by exposure of the polymerase to extreme heat (typically 96°C to 100°C) for extended periods of time (greater than or equal to 20 minutes). While there are minor differences with respect to the specific heat tolerance for each of the enzymes, these are readily determined. The polymerase mixture of the invention or the $exo^+$ DNA polymerase used as the second enzyme with reduced polymerization activity can be treated with heat for various periods of time and the effect of the heat treatment upon the polymerization and 3'-5' nuclease activities is determined. Conditions reducing DNA polymerase activity but not significantly affecting the 3'-5' exonuclease activity may be used to pretreat the polymerase mixture or the $exo^+$ DNA polymerase used as second enzyme with reduced polymerization activity in the present invention.

Enzyme Mixture

The subject enzyme mixture composition comprises a first enzyme comprising DNA polymerization activity and a second enzyme comprising 3'-5' exonuclease activity with reduced DNA polymerase activity.

In one embodiment, the first enzyme is a DNA polymerase with 3'-5' exonuclease activity. The fidelity of the first enzyme for DNA amplification is increased by the use of a second enzyme which also possesses 3'-5' exonuclease activity. A preferred DNA polymerase with 3'-5' exonuclease activity as the first enzyme is a wild type Pfu DNA polymerase.

In another embodiment, the first enzyme is a DNA polymerase without 3'-5' exonuclease activity. The fidelity of an amplification reaction is provided by the second enzyme of the subject invention, which possesses 3'-5' exonuclease activity. A preferred DNA polymerase without 3'-5' exonuclease activity as the first enzyme is a Taq DNA polymerase.

In yet another embodiment, the first enzyme may is a reverse transcriptase with DNA polymerization activity. The fidelity of the reverse transcriptase in cDNA synthesis is increased by the use of a second enzyme which possesses 3'-5' exonuclease activity.

A. Selection of the first and the second enzyme pair

In the subject method for DNA synthesis, any enzyme comprising DNA polymerization activity may be mixed with a second enzyme comprising 3'-5' exonuclease activity and reduced polymerization activity.

When both first and second enzymes in the mixture comprise 3'-5' exonuclease activity, it may be desirable to combine two enzymes with different proofreading activities. By "different proofreading activity", it means that two 3'-5' exonucleases exhibits different proofreading preference for a nucleotide. For example, one 3'-5' exonuclease may proofread a G-T mispair more efficiently than an A-A mispair, another exonuclease having a different proofreading preference may proofread an A-A mispair more efficiently than a G-T mispair. By using a second enzyme with a different proofreading preference from the first enzyme of the subject composition, one can enhance proofreading of the first enzyme by providing proofreading to mispairs which the first enzyme is not capable of recognizing and excising efficiently.

Another factor to consider when selecting the first and the second enzymes of the subject invention is the compatibility of reaction conditions (e.g., pH, buffer composition, temperature requirement, etc.) required by each enzyme.

In a preferred embodiment, the subject composition comprises a wild-type Pfu DNA polymerase as the first enzyme and a mutant Pfu DNA polymerase with reduced DNA polymerization activity as the second enzyme. Preferably, the mixture comprises a ratio of 2.5-5U Pfu DNA polymerase plus an amount of a polymerase reduced mutant corresponding to <0.01U DNA polymerase activity and 0.007U to 0.04U of 3'-5' exonuclease activity (or the amount of exonuclease activity containing within approximately 0.5 to 10U wild type Pfu). More preferably, the mixture comprises a ratio of 2.5-5U Pfu DNA polymerase plus an amount of a polymerase reduced mutant corresponding to <0.01U DNA polymerase activity and 0.02U of 3'-5' exonuclease activity (or the amount of exonuclease activity contained within 2-3U wild type Pfu). In a preferred embodiment, the enzyme mixture composition comprises a wild-type Pfu DNA polymerase with 2.5U DNA polymerization activity and 0.02U 3'-5' exonuclease activity as the first enzyme and a mutant DNA polymerase with reduced DNA polymerization activity (e.g., G387P) with 0.02U 3'-5' exonuclease activity as the second enzyme.

In another preferred embodiment, the subject composition comprises a wild-type Taq DNA polymerase as the first enzyme and a mutant Pfu DNA polymerase with reduced DNA polymerization activity as the second enzyme. Preferably, the enzyme mixture comprises a ratio of 2.5U Taq DNA polymerase plus an amount of a polymerase deficient mutant corresponding to <0.1U DNA polymerase activity and 0.01 to 0.2U of 3'-5' exonuclease activity (or the amount of exonuclease activity contained within 1-20U wild type Pfu). More preferably, the enzyme mixture comprises a ratio of 2.5U Taq DNA polymerase plus an amount of a polymerase deficient mutant corresponding to <0.01U DNA polymerase activity and 0.08U of 3'-5' exonuclease activity (or the amount of exonuclease activity contained within 10-12U wild type Pfu). In a preferred embodiment, the enzyme misture composition comprises a wild-type Taq DNA polymerase with 2.5U polymerization activity as the first enzyme and a mutant Pfu DNA polymerase with reduced polymerization activity (e.g., G387P) with 0.08U 3'-5' exonuclease activity.

Preferably the mutant Pfu DNA polymerase with reduced DNA polymerization activity comprises one or more mutations at amino acid positions selected from the group consisting of: D405, Y410, T542, D543, K593, Y595, Y385, G387, and G388.

More preferably, the mutant Pfu DNA polymerase comprises one or more mutations selected from the group consisting of: D405E, Y410F, T542P, D543G, K593T, Y595S, Y385Q, Y385S, Y385N, Y385L, Y385H, G387S, G387P, and G388P.

B. The Ratio Of Polymerization To Exonuclease Activity In The Enzyme Mixture

In a variety of DNA synthesis and amplification procedures, the compositions of the present invention provide superior synthesis results (e.g., higher fidelity and efficiency), as compared with the synthesis results obtained with a single DNA polymerase or with a mixture comprising two wild type DNA polymerases. When using the subject composition, the ratio of total polymerization activity and total exonuclease activity in the enzyme mixture may be critical for optimal efficiency and fidelity of DNA synthesis.

In the enzyme mixture disclosed herein, when DNA polymerases are used as the first and second enzymes, both enzymes may contribute to the polymerization and/or 3'-5' exonuclease activity. When an enzyme other than a conventional DNA polymerase is used as the first enzyme (e.g., a reverse transcriptase), both enzymes may contribute to DNA polymerization activity, but only the second enzyme contribute to the 3'-5' exonuclease activity. When an enzyme other than a DNA polymerase is used as the second enzyme (e.g., E. coli exonuclease I), both enzymes may contribute to the 3'-5' exonuclease activity, but only the first enzyme contribute to the polymerization activity of the enzyme mixture.

The ratio of the first and the second enzyme in the subject composition may be varied with respect to one another. The ratio of the DNA polymerization activity to 3'-5' exonuclease activity present in the subject composition employed in a given synthesis procedure may be readily optimized by performing a series of simple experiments in which the ratio of the DNA polymerization activity to the exonuclease activity in the reaction mixture are systematically varied with respect to one another and the synthesis results compared.

3'-5' exonuclease activity has been shown to degrade unannealed primers. The degraded primers would not be available in subsequent rounds of DNA amplification and would therefore effect the efficiency of the PCR reaction. In applications requiring very high product yield, it may therefore be desirable to have a low concentration of the exonuclease activity relative to the DNA polymerization activity to decrease this effect and to increase the product yield. However, when fidelity is more important than yield, it may be desirable to have a high concentration of the exonuclease activity relative to the DNA polymerization activity to increase the accuracy of the synthesis or amplification so long as the level of polymerization activity does not significantly inhibit the efficiency of the amplification.

In a preferred embodiment, the ratio of the DNA polymerase activity and the exonuclease activity in the enzyme mixture is about (2.5-5U of DNA polymerization activity)/(0.02-5U of 3'-5' exonulcease activity), for example, about (2.5U of DNA polymerization activity)/(0.04-0.08U of 3'-5' exonulcease activity).

Applications of The Subject Invention

In one aspect, the disclosure provides a method for DNA synthesis using the compositions of the subject invention. The subject compositions may be used in various methods of polynucleotide synthesis in essentially the same manner as the DNA polymerase or other synthetic enzyme present in the subject composition. Typically, synthesis of a polynucleotide requires a synthesis primer, a synthesis template, polynucleotide precursors for incorporation into the newly synthesized polynucleotide, (e.g. dATP, dCTP, dGTP, dTTP), and the like. Detailed methods for carrying out polynucleotide synthesis are well known to the person of ordinary skill in the art and can be found, for example, in Molecular Cloning second edition, Sambrook et al., Cold Spring Harbor Laboratory Press, Cold Spring Harbor, N.Y. (1989).

A. Application In Amplification Reactions

"Polymerase chain reaction" or "PCR" refers to an in vitro method for amplifying a specific polynucleotide template sequence. The technique of PCR is described in numerous publications, including, PCR: A Practical Approach, M. J. McPherson, et al., IRL Press (1991), PCR Protocols: A Guide to Methods and Applications, by Innis, et al., Academic Press (1990), and PCR Technology: Principals and Applications for DNA Amplification, H. A. Erlich, Stockton Press (1989). PCR is also described in many U.S. Patents, including U.S. Patent Nos. 4,683,195; 4,683,202; 4,800,159; 4,965,188; 4,889,818; 5,075,216; 5,079,352; 5,104,792; 5,023,171; 5,091,310; and 5,066,584.

For ease of understanding the advantages provided by the present invention, a summary of PCR is provided. The PCR reaction involves a repetitive series of temperature cycles and is typically performed in a volume of 50-100 μl. The reaction mix comprises dNTPs (each of the four deoxynucleotides dATP, dCTP, dGTP, and dTTP), primers, buffers, DNA polymerase, and polynucleotide template. PCR requires two primers that hybridize with the double-stranded target polynucleotide sequence to be amplified. In PCR, this double-stranded target sequence is denatured and one primer is annealed to each strand of the denatured target. The primers anneal to the target polynucleotide at sites removed from one another and in orientations such that the extension product of one primer, when separated from its complement, can hybridize to the other primer. Once a given primer hybridizes to the target sequence, the primer is extended by the action of a DNA polymerase. The extension product is then denatured from the target sequence, and the process is repeated.

In successive cycles of this process, the extension products produced in earlier cycles serve as templates for DNA synthesis. Beginning in the second cycle, the product of amplification begins to accumulate at a logarithmic rate. The amplification product is a discrete double-stranded DNA molecule comprising: a first strand which contains the sequence of the first primer, eventually followed by the sequence complementary to the second primer, and a second strand which is complementary to the first strand.

Due to the enormous amplification possible with the PCR process, small levels of DNA carryover from samples with high DNA levels, positive control templates or from previous amplifications can result in PCR product, even in the absence of purposefully added template DNA. If possible, all reaction mixes are set up in an area separate from PCR product analysis and sample preparation. The use of dedicated or disposable vessels, solutions, and pipettes (preferably positive displacement pipettes) for RNA/DNA preparation, reaction mixing, and sample analysis will minimize cross contamination. See also Higuchi and Kwok, 1989, Nature, 339:237-238 and Kwok, and Orrego, in: Innis et al. eds., 1990, PCR Protocols: A Guide to Methods and Applications, Academic Press, Inc., San Diego, Calif.

1. Thermostable Enzymes

For PCR amplifications, the enzymes used in the invention are preferably thermostable. As used herein, "thermostable" refers to an enzyme which is stable to heat, is heat resistant, and functions at high temperatures, e.g., 50 to 90°C. The thermostable enzyme according to the present invention must satisfy a single criterion to be effective for the amplification reaction, i.e., the enzyme must not become irreversibly denatured (inactivated) when subjected to the elevated temperatures for the time necessary to effect denaturation of double-stranded polynucleotides. By "irreversible denaturation" as used in this connection, is meant a process bringing a permanent and complete loss of enzymatic activity. The heating conditions necessary for denaturation will depend, e.g., on the buffer salt concentration and the length and nucleotide composition of the polynucleotides being denatured, but typically range from 85°C, for shorter polynucleotides, to 105°C for a time depending mainly on the temperature and the polynucleotide length, typically from 0.25 minutes for shorter polynucleotides, to 4.0 minutes for longer pieces of DNA. Higher temperatures may be tolerated as the buffer salt concentration and/or GC composition of the polynucleotide is increased. Preferably, the enzyme will not become irreversibly denatured at 90 to 100°C. An enzyme that does not become irreversibly denatured, according to the invention, retains at least 10%, or at least 25%, or at least 50% or more function or activity during the amplification reaction.

2. PCR Reaction Mixture

In addition to the subject enzyme mixture, one of average skill in the art may also employ other PCR parameters to increase the fidelity of synthesis/amplification reaction. It has been reported PCR fidelity may be affected by factors such as changes in dNTP concentration, pH, units of enzyme used per reaction, and the ratio of $Mg^{2+}$ to dNTPs present in the reaction (Mattila et al., 1991, supra).

$Mg^{2+}$ concentration affects the annealing of the oligonucleotide primers to the template DNA by stabilizing the primer-template interaction, it also stabilizes the replication complex of polymerase with template-primer. It can therefore also increases non-specific annealing and produced undesirable PCR products (gives multiple bands in gel). When non-specific amplification occurs, $Mg^{2+}$ may need to be lowered or EDTA can be added to chelate $Mg^{2+}$ to increase the accuracy and specificity of the amplification.

Other divalent cations such as $Mn^{2+}$, or $Co^{2+}$ can also affect DNA polymerization. Suitable cations for each DNA polymerase are known in the art (e.g., in DNA Replication 2nd edition, supra). Divalent cation is supplied in the form of a salt such $MgCl_2$, $Mg(OAc)_2$, $MgSO_4$, $MnCl_2$, $Mn(OAc)_2$, or $MnSO_4$. Usable cation concentrations in a Tris-HCl buffer are for $MnCl_2$ from 0.5 to 7 mM, preferably, between 0.5 and 2 mM, and for $MgCl_2$ from 0.5 to 10 mM. Usable cation concentrations in a Bicine/KOAc buffer are from 1 to 20 mM for $Mn(OAc)_2$, preferably between 2 and 5 mM.

Monovalent cation required by DNA polymerase may be supplied by the potassium, sodium, ammonium, or lithium salts of either chloride or acetate. For KCl, the concentration is between 1 and 200 mM, preferably the concentration is between 5 and 100 mM, although the optimum concentration may vary depending on the polymerase used in the reaction.

Deoxyribonucleotide triphosphates (dNTPs) are added as solutions of the salts of dATP, dCTP, dGTP, dUTP, and dTTP, such as disodium or lithium salts. In the present methods, a final concentration in the range of 1 $\mu$M to 2 mM each is suitable, and 100-600 $\mu$M is preferable, although the optimal concentration of the nucleotides may vary in the reverse transcription reaction depending on the total dNTP and divalent metal ion concentration, and on the buffer, salts, particular primers, and template. For longer products, i.e., greater than 1500 bp, 500 $\mu$M each dNTP may be preferred when using a Tris-HCl buffer.

dNTPs chelate divalent cations, therefore amount of divalent cations used may need to be changed according to the dNTP concentration in the reaction. Excessive amount of dNTPs (e.g., larger than 1.5 mM) can increase the error rate and possibly inhibits DNA polymerases.

Lowering the dNTP (e.g., to 10-50 $\mu$M) may therefore reduce error rate. PCR reaction for amplifying larger size template may need more dNTPs.

One suitable buffering agent is Tris-HCl, preferably pH 8.3, although the pH may be in the range 8.0-8.8. The Tris-HCl concentration is from 5-250 mM, although 10-100 mM is most preferred. A preferred buffering agent is Bicine-KOH, preferably pH 8.3, although pH may be in the range 7.8-8.7. Bicine acts both as a pH buffer and as a metal buffer.

PCR is a very powerful tool for DNA amplification therefore very little template DNA is needed. However, in some embodiments, to reduce the likelihood of error, a higher DNA concentration may be used, though too many templates may increase the amount of contaminants and reduce efficiency.

Usually, up to 3 $\mu$M of primers may be used, but high primer to template ratio can results in non-specific amplification and primer-dimer formation. Therefore it is usually necessary to check primer sequences to avoid primer-dimer formation. In a preferred embodiment, 0.1-0.5 $\mu$M of primers are used.

3. Cycling Parameters

Denaturation time may be increased if template GC content is high. Higher annealing temperature may be needed for primers with high GC content or longer primers. Gradient PCR is a useful way of determining the annealing temperature. Extension time should be extended for larger PCR product amplifications. However, extension time may need to be reduced whenever possible to limit damage to enzyme.

The number of cycle can be increased if the number of template DNA is very low, and decreased if high amount of template DNA is used.

4. PCR Enhancing Factors And Additives

PCR enhancing factors may also be used to improve efficiency of the amplification. As used herein, a "PCR enhancing factor" or a "Polymerase Enhancing Factor" (PEF) refers to a complex or protein possessing polynucleotide polymerase enhancing activity (Hogrefe et al., 1997, Strategies 10::93-96; and U.S. Patent No. 6,183,997).

For Pfu DNA polymerase, PEF comprises either P45 in native form (as a complex of P50 and P45) or as a recombinant protein. In the native complex of Pfu P50 and P45, only P45 exhibits PCR enhancing activity. The P50 protein is similar in structure to a bacterial flavoprotein. The P45 protein is similar in structure to dCTP deaminase and dUTPase, but it functions only as a dUTPase converting dUTP to dUMP and pyrophosphate. PEF, according to the present invention, can also be selected from the group consisting of: an isolated or purified naturally occurring polymerase enhancing protein obtained from an archeabacteria source (e.g., *Pyrococcus furiosus*); a wholly or partially synthetic protein having the same amino acid sequence as Pfu P45, or analogs thereof possessing polymerase enhancing activity; polymerase-enhancing mixtures of one or more of said naturally occurring or wholly or partially synthetic proteins; polymerase-enhancing protein complexes of one or more of said naturally occurring or wholly or partially synthetic proteins; or polymerase-enhancing partially purified cell extracts containing one or more of said naturally occurring proteins (U.S. Patent No. 6,183,997, supra). The PCR enhancing activity of PEF is defined by means well known in the art. The unit definition for PEF is based on the dUTPase activity of PEF (P45), which is determined by monitoring the production of pyrophosphate (PPi) from dUTP. For example, PEF is incubated with dUTP (10mM dUTP in 1x cloned Pfu PCR buffer) during which time PEF hydrolyzes dUTP to dUMP and PPi. The amount of PPi formed is quantitated using a coupled enzymatic assay system that is commercially available from Sigma (#P7275). One unit of activity is functionally defined as 4.0 nmole of PPi formed per hour (at 85°C).

Other PCR additives may also affect the accuracy and specificity of PCR reaction. EDTA less than 0.5 mM may be present in the amplification reaction mix. Detergents such as Tween-20™ and Nonidet™ P-40 are present in the enzyme dilution buffers. A final concentration of non-ionic detergent approximately 0.1% or less is appropriate, however, 0.01-0.05% is preferred and will not interfere with polymerase activity. Similarly, glycerol is often present in enzyme preparations and is generally diluted to a concentration of 1-20% in the reaction mix. Glycerol (5-10%), formamide (1-5%) or DMSO (2-10%) can be added in PCR for template DNA with high GC content or long length (e.g., > 1kb). These additives change the Tm (melting temperature) of primer-template hybridization reaction and the thermostability of polymerase enzyme. BSA (up to 0.8 $\mu$g/$\mu$l) can improve efficiency of PCR reaction. Betaine (0.5-2M) is also useful for PCR over high GC content and long fragments of DNA. Tetramethylammonium chloride (TMAC, >50mM), Tetraethylammonium chloride (TEAC), and Trimethlamine N-oxide (TMANO) may also be used. Test PCR reactions may be performed to determine optimum concentration of each additive mentioned above.

Various specific PCR amplification applications are available in the art (for reviews, see for example, Erlich, 1999, Rev Immunogenet., 1:127-34; Prediger 2001, Methods Mol. Biol. 160:49-63; Jurecic et al., 2000, Curr. Opin. Microbiol. 3:316-21; Triglia, 2000, Methods Mol. Biol. 130:79-83; MaClelland et al.,1994, PCR Methods Appl. 4:S66-81; Abramson and Myers, 1993, Current Opinion in Biotechnology 4:41-47).

The subject invention can be used in PCR appliciations include, but are not limited to, i) hot-start PCR which reduces non-specific amplification; ii) touch-down PCR which starts at high annealing temperature, then decreases annealing temperature in steps to reduce non-specific PCR product; iii) nested PCR which synthesizes more reliable product using an outer set of primers and an inner set of primers; iv) inverse PCR for amplification of regions flanking a known sequence. In this method, DNA is digested, the desired fragment is circularized by ligation, then PCR using primer complementary to the known sequence extending outwards; v) AP-PCR (arbitrary primed)/RAPD (random amplified polymorphic DNA). These methods create genomic fingerprints from species with little-known target sequences by amplifying using arbitrary oligonucleotides; vi) RT-PCR which uses RNA-directed DNA polymerase (e.g., reverse transcriptase) to synthesize cDNAs which is then used for PCR. This method is extremely sensitive for detecting the expression of a specific sequence in a tissue or cells. It may also be use to quantify mRNA transcripts; vii) RACE (rapid amplification of cDNA ends). This is used where information about DNA/protein sequence is limited. The method amplifies 3' or 5' ends of cDNAs generating fragments of cDNA with only one specific primer each (plus one adaptor primer). Overlapping RACE products can then be combined to produce full length cDNA; viii) DD-PCR (differential display PCR) which is used to identify differentially expressed genes in different tissues. First step in DD-PCR involves RT-PCR, then amplification is performed using short, intentionally nonspecific primers; ix) Multiplex-PCR in which two or more unique targets of DNA sequences in the same specimen are amplified simultaneously. One DNA sequence can be use as control to verify the quality of PCR; x) Q/C-PCR (Quantitative comparative) which uses an internal control DNA sequence (but of different size) which compete with the target DNA (competitive PCR) for the same set of primers; xi) Recusive PCR which is used to synthesize genes. Oligonucleotides used in this method are complementary to stretches of a gene (>80 bases), alternately to the sense and to the antisense strands with ends overlapping (-20 bases); xii) Asymmetric PCR; xiii) In Situ PCR; xiv) Site-directed PCR Mutagenesis.

It should be understood that this invention is not limited to any particular amplification system. As other systems are developed, those systems may benefit by practice of this invention. A recent survey of amplification systems was published in.

B. Applications In Reverse Transcription

The term "reverse transcriptase" describes a class of polymerase characterized as RNA-dependent DNA polymerases. All known reverse transcriptases require a primer to synthesize a DNA transcript from an RNA template. Historically, reverse transcriptase has been used primarily to transcribe mRNA into cDNA which can then be cloned into a vector for further manipulation (e.g., PCR amplification by a DNA-dependent DNA polymerase).

Avian myoblastosis virus (AMV) reverse transcriptase was the first widely used RNA-dependent DNA polymerase (Verma, 1977, Biochem.Biophys.Acta 473:1). The enzyme has 5'-3' RNA-directed DNA polymerase activity, 5'-3' DNA-directed DNA polymerase activity, and RNase H activity. RNase H is a processive 5' and 3' ribonuclease specific for the RNA strand of RNA-DNA hybrids (Perbal, 1984, A Practical Guide to Molecular Cloning, Wiley & Sons New York). Errors in transcription cannot be corrected by reverse transcriptase because known viral reverse transcriptases lack the 3'-5' exonuclease activity necessary for proofreading (Saunders and Saunders, 1987, Microbial Genetics Applied to Biotechnology, Croom Helm, London). The use of the second enzyme in the subject composition provides proofreading for the reverse transcription reaction. A detailed study of the activity of AMV reverse transcriptase and its associated RNase H activity has been presented by Berger et al., 1983, Biochemistry 22:2365-2372.

The reaction mixture for reverse transcription usually includes enzymes, aqueous buffers, salts, oligonucleotide primers, target polynucleotide, and nucleoside triphosphates. Depending upon the context, the mixture can be either a complete or incomplete reverse transcription reaction mixture. The reaction mixture can be modified according to the conditions required by the second enzyme of the subject composition. It is known that cDNAs can be obtained from mRNAs in vitro using a reverse transcriptase (RNA-dependent DNA polymerase). The full length cDNA strands produced in turn may be used as a template for subsequent amplification reaction (e.g., PCR) and the like.

Reverse transcription in combination with PCR (RT-PCT) is utilized to detect the presence of one or many specific RNA molecules which may be present in a sample. The method can be used to detect, for example, RNA from different organisms (such as viruses, bacteria, fungi, plants, and animals), or RNA indicative of an infection, a disease state, or predisposition to a disease. For example, mRNA specific to tumor cells can be detected. The method is also useful for detecting a class of microorganisms or a group of related disease conditions.

Reverse transcription can generally be performed at any temperature within the functional temperature range of the reverse transcriptase. Preferably, the temperature of incubation is any temperature at which the reverse transcriptase is functional and the primer remains hybridized to the RNA molecule. For non-thermostable reverse transcriptases, preferred temperatures are those temperatures that are at or around the optimum temperature for the reverse transcriptase. For most non-thermostable reverse transcriptases this temperature will be between about 25°C and 45°C

U.S. Patent No. 5,994,079 discloses thermostable reverse transcriptases. $Mn^{2+}$ is preferred as the divalent cation and is typically included as a salt, for example, manganese chloride ($MnCl_2$), manganese acetate ($Mn(OAc)_2$), or manganese sulfate ($MnSO_4$). If $MnCl_2$ is included in a reaction containing 10 mM Tris buffer, for example, the $MnCl_2$ is generally present at a concentration of 0.5-7.0 mM; 0.8-1.4 mM is preferred when 200 $\mu$M of each dGTP, dATP, dUTP, and, dCTP are utilized; an 1.2 mM $MnCl_2$ is most preferred.

A thermostable reverse transcriptase may retain at least 5% of its maximum activity at any temperature above 50°C or has an optimal temperature of at least 50°C. The highest temperature at which a thermostable reverse transcriptase is functional can be quite high. For this reason, preferred temperature ranges for reverse transcription when a thermostable reverse transcriptase is used are most conveniently described in terms of the calculated melting temperature of a hybrid between the RNA molecule of interest and the primer. Such a melting temperature is referred to herein as the RNA/primer melting temperature (R/P Tm). Preferred ranges include a temperature from 20°C below the melting temperature of a hybrid between the RNA molecule of interest and the primer and 5°C above the melting temperature of a hybrid between the RNA molecule of interest and the primer. In general, the closer the temperature is to the R/P Tm, the greater the degree of discrimination there will be between specific and non-specific hybrids of the RNA and primer. If the temperature is close to the R/P Tm, however, decreased stability of specific hybrids may cause priming to be less efficient.

R/P Tm can be determined either by calculation or by empirical measurement. For calculating R/P Tm, any established formula for calculating stability of polynucleotide hybrids can be used. A preferred formula for calculating R/P Tm is $Tm=81.5+16.6(\log M)^{+}0.41(\%\ G^{+}C)-0.72(\%\ \text{formamide})$, which was derived from studies on the stability of perfectly-matched DNA:DNA hybrids. For RNA:DNA hybrids, incorporating formamide concentration in the formula does not hold because the relationship between formamide concentration and the depression of Tm is not linear. At 80% formamide, RNA:DNA hybrids are more stable than DNA:DNA hybrids, increasing the Tm by about 10 to 30°C depending on the sequence (Hames & Higgins, Polynucleotide Hybridisation: A Practical Approach (IRL Press Limited, Oxford, England. 1985)). Carrying out the reaction in 80% formamide can therefore also be used to suppress formation of DNA:DNA duplexes, to preferentially select RNA:DNA hybrids, and to estimate the Tm for R/P. Because the empirically derived formulas for the estimation of RNA:DNA hybrid Tm may not be as accurate for short DNA primers, the hybridization temperature is preferably determined by assessing hybrid stability in 0.1-0.4 M monovalent cation at temperatures ranging from 40 to 60°C R/P Tm can also be determined empirically (Lesnick and Freier, 1995, Biochemistry 34:10807-10815, McGraw et al., 1990, Biotechniques 8:674-678; and Rychlik et al., 1990, Polynucleotides Res. 18:6409-6412).

The fidelity of viral reverse transcriptases, such as AMV-RT and MoMuLV-RT, may be compared to thermoactive reverse transcriptases by a straightforward assay procedure described in U.S. Patent No. 5,994,079 (supra). Plasmid BS+ (Stratagene) can be used for such an assay. The plasmid encodes an α-complementing β-galactosidase activity and can be linearized with NdeI. T3 RNA polymerase is used to prepare a cRNA transcript of the α-donor region. After treatment of the cRNA with RNase-free DNase and isolation of the cRNA, the cRNA is used as a template for a reverse transcription/amplification reaction. A reverse transcription primer complementary to the 3' end of the cDNA containing an NdeI sequence at its 5' terminus, and an upstream PCR primer comprising a PstI sequence at the 5' termini provide a 752 bp PCR product. The PCR product and the pBS+ vector are then digested with NdeI and PstI followed by ligation of the PCR product into the vector and transformation into a suitable host. The presence of white colonies indicates that a mutation had occurred during the RT or PCR amplification. The assay provides means for assigning a relative value to the fidelity of the reverse transcriptase activity of various enzymes. Specific mutations can be determined by sequence analysis.

Following reverse transcription of RNA, the RNA can be removed from the RNA/cDNA hybrid by heat denaturation or by a number of other known means such as alkali, heat, or enzyme treatment. Enzyme treatment may consist of, for example, treating the RNA/cDNA hybrid with RNase H. RNase H is specific for RNA strands within an RNA/DNA double-stranded molecule.

The subject composition is suitable for high fidelity transcribing and amplifying RNA from a number of sources. The RNA template may be contained within a polynucleotide preparation from an organism, for example, a viral or bacterial polynucleotide preparation. The preparation may contain cell debris and other components, purified total RNA, or purified mRNA. The RNA template may be a population of heterogeneous RNA molecules in a sample or a specific target RNA molecule.

RNA suitable for use in the present methods may be contained in a biological sample suspected of containing a specific target RNA. The biological sample may be a heterogeneous sample in which RNA is a small portion of the sample, as in for example, a blood sample or a biopsied tissue sample. Thus, the subject composition is useful for clinical detection and diagnosis. The RNA target may be indicative of a specific disease or infectious agent.

RNA may be prepared by any number of methods known in the art; the choice may depend on the source of the sample and availability. Methods for preparing RNA are described in Davis et al., 1986, Basic Methods in Molecular Biology, Elsevier, NY, Chapter 11; Ausubel et al., 1987, Current Protocols in Molecular Biology, Chapter 4, John Wiley and Sons, NY; Kawasaki and Wang, 1989, PCR Technology, ed. Erlich, Stockton Press NY; Kawasaki, 1990, PCR Protocols: A Guide to Methods and Applications, Innis et al. eds. Academic Press, San Diego

C. Detection Of Amplified Product

Detection of amplified polynucleotide product can be accomplished by any of a variety of well known techniques. In a preferred embodiment, the amplified product is separated on the basis of molecular weight by gel electrophoresis, and the separated products are then visualized by the use of polynucleotide specific stains which allow one to observe the discrete species of resolved amplified product present in the gel. Although numerous polynucleotide specific stains exist and would be suitable to visualize the electrophoretically separated polynucleotides, ethidium bromide is preferred.

Alternative methods suitable to detect the amplified polynucleotide product include hybridization-based detection means that use a labeled polynucleotide probe capable of hybridizing to the amplified product. Exemplary of such detection means include the Southern blot analysis, ribonuclease protection analysis using in vitro labeled polyribonucleotide probes, and similar methods for detecting polynucleotides having specific nucleotide sequences. See, for example, Ausubel et al., Current Protocols in Molecular Biology, John Wiley & Sons, 1987.

Amplified products (e.g., by PCR or RT-PCR) using the subject composition of the invention can be used for subsequent analysis such as sequencing or cloning.

D. Application In Direct Cloning of PCR Amplified Product

While it is understood that the amplified product using subject composition can be cloned by any method known in the art. In one embodiment, the invention provides a composition which allows direct cloning of PCR amplified product.

The most common method for cloning PCR products involves incorporation of flanking restriction sites onto the ends of primer molecules. The PCR cycling is carried out and the amplified DNA is then purified, restricted with an appropriate endonuclease(s) and ligated to a compatible vector preparation.

A method for directly cloning PCR products eliminates the need for preparing primers having restriction recognition sequences and it would eliminate the need for a restriction step to prepare the PCR product for cloning. Additionally, such method would preferably allow cloning PCR products directly without an intervening purification step.

U.S. Patent Nos. 5,827,657 and 5,487,993
discloses method for direct cloning of PCR products using a DNA polymerase which takes advantage of the single 3'-deoxy-adenosine monophosphate (dAMP) residues attached to the 3' termini of PCR generated nucleic acids. Vectors are prepared with recognition sequences that afford single 3'-terminal deoxy-thymidine monophosphate (dTMP) residues upon reaction with a suitable restriction enzyme. Thus, PCR generated copies of genes can be directly cloned into the vectors without need for preparing primers having suitable restriction sites therein.

Taq DNA polymerase exhibits terminal transferase activity that adds a single dATP to the 3' ends of PCR products in the absence of template. This activity is the basis for the TA cloning method in which PCR products amplified with Taq are directed ligated into vectors containing single 3'dT overhangs. Pfu DNA polymerase, on the other hand, lacks terminal transferase activity, and thus produces blunt-ended PCR products that are efficiently cloned into blunt-ended vectors.

In one embodiment, the subject invention comprises a Taq DNA polymerase as the first enzyme and a mutant Pfu DNA polymerase with reduced polymerization activity as the second enzyme. Taq DNA polymerase in the composition produces amplified DNA product with 3'-dAMP and allows direct cloning of the amplified product, while the mutant Pfu DNA polymerase provides fidelity for the amplification.

Kits

The invention herein also contemplates a kit format which comprises a package unit having one or more containers of the subject composition and in some embodiments including containers of various reagents used for polynucleotide synthesis, including synthesis in PCR. The kit may also contain one or more of the following items: polynucleotide precursors, primers, buffers, instructions, and controls. Kits may include containers of reagents mixed together in suitable proportions for performing the methods in accordance with the invention. Reagent containers preferably contain reagents in unit quantities that obviate measuring steps when performing the subject methods.

## EXAMPLES

The following examples are offered for the purpose of illustrating, not limiting, the subject invention.

### Example 1. Constructing Mutants Of Pfu DNA Polymerase With Reduced DNA Polymerase Activity

We introduced mutations into Pfu DNA polymerase that were likely to reduce or eliminate DNA polymerase activity, while having minimal effects on proofreading activity. The mutations selected were identified from previous mutagenesis studies carried out using related Family B DNA polymerases. We made the same amino acid side chain substitutions in the polymerization domain at the following residues in Pfu (D405E, Y410F, T542P, D543G, K593T, Y595S) (Table 1).

Mutations were also introduced within the partitioning domain at amino acids 384-389 (SYTGGF) in Pfu DNA polymerase (Table 1).

The DNA template used for mutagenesis contained the Pfu pol gene, cloned into pBluescript (pF72 clone described in US 5,489,523) and expressed with an N-terminal $His_6$ tag for affinity purification. A modified QuikChange (Stratagene) protocol was used to insert the $His_6$ tag at the 5' end of the Pfu *pol* gene, just after the initiator ATG. The insertion reaction was carried out in two steps. In the first step, a standard QuikChange reaction was carried out in the presence of *Tth* ligase (10U/RXN) using only the $His_6$ forward primer. After 18 cycles, the reaction was *Dpn*I-digested for one hour at 37°C and then purified with the StrataPrep® Plasmid Miniprep Kit (Stratagene). The purified material served as the template in the second QuikChange reaction, which employed only the $His_6$ reverse primer. After 18 cycles, the second reaction was *Dpn*I-digested for one hour at 37°C, and then transformed. The $His_6$-Pfu *pol* construct was confirmed by both PCR amplification and sequencing using the Big Dye sequencing kit.

Point mutations were introduced into the Pfu *pol* gene using the QuikChange Site-Directed Mutagenesis Kit (Stratagene). Clones were sequenced to verify incorporation of the desired mutations.

Table 1: Activity of partially-purified His-tagged *Pfu* mutants (Nickel-resin eluates):

| Mutation | Polymerase activity | | Exonuclease activity | | Rel. exo/pol vs. wt (1.0)[$] |
|---|---|---|---|---|---|
| | Cpms @ 50ng (500ng) | % wild type @ 50ng* | Cpms (50 ng) | % wild type[@] | |
| **Partitioning** | | | | | |
| S384G | 46920 | 71 | 1425 | ≥100 | 2.3 |
| S384K | 66545 | 100 | 554 | 63 | 0.6 |
| Y385N | 1123 | 2 | 158 | 18 | 10.6 |

(continued)

| Mutation | Polymerase activity | | Exonuclease activity | | Rel. exo/pol vs. wt (1.0)$ |
|---|---|---|---|---|---|
| | Cpms @ 50ng (500ng) | % wild type @ 50ng* | Cpms (50 ng) | % wild type@ | |
| **Partitioning** | | | | | |
| Y385W | 10515 (24519) | 16 | 36 | 4 | 0.3 |
| Y385L | 2383 | 4 | 180 | 21 | 5.7 |
| Y385H | 4276 | 6 | 91 | 10 | 1.6 |
| **Y385Q** | 386 (5431) | 0.6 | 252 | 29 | **49.2** |
| Y385S | 1095 (4206) | 2 | 578 | 66 | 39.8 |
| Y385F | 80685 (21580) | 100 | 1008 | ≥100 | 0.9 |
| T386E | 48296 | 73 | 263 | 30 | 0.4 |
| T386Y | 47318 | 72 | 1112 | ≥100 | 1.8 |
| T386G | 46289 | 70 | 1011 | ≥100 | 1.6 |
| G387S | 648 | 1 | 169 | 19 | 19.7 |
| **G387P** | 258 (66) | 0.4 | 500 | 57 | **146.2** |
| G388A | 2560 | 4 | 73 | .008 | 2.2 |
| G388S | 74551 | 100 | 670 | 76 | 0.7 |
| G388P | 1222 | 2 | 202 | 23 | 12.5 |
| F389Y | 43455 (29809) | 66 | 37 | 4 | 06 |
| F389L | 72647 | 100 | 1054 | ≥100 | 1.1 |
| F389V | 30641 | 46 | 614 | 70 | 1.5 |
| F389S | 17998 | 27 | 1335 | ≥100 | 5.6 |
| F389H | 19623 | 30 | 543 | 62 | 2.1 |
| | Polymerase activity | | Exo/pol activity | | |
| **Polymerase** | Cpms @ 5ng | % wild type @ 5ng# | Cpms exonuclease | Cpms Polymerase | Rel. exo/pol vs. wt (1.0)& |
| **DXXSLYP** | | | | | |
| **D405E** | 69 (500ng) | <0.2 | 321 | 0 | **>396** |
| Y410F | 10181 | 27 | 698 | 16189 | 5.3 |
| **YXDTDS** | | | | | |
| **T542P** | 27 | .07 | 1105 | 0 | **>1364** |
| D543G | 10 | .03 | 704 | 687 | 127 |
| **T542P/D543G** | 23 | .06 | 505 | 0 | **>623** |
| **KXY** | | | | | |
| **K593T** | 155 | .4 | 668 | 0 | **>825** |
| Y595S | 6107 | 16 | 1072 | 2684 | 49 |
| 100% for wt *Pfu* equals: *66146 cpms; #38014 cpms; @877cpms exo/pol for wt *Pfu* equals: $0.01326; &0.0081 | | | | | |

**Example 2. Affinity Purification Of His-Tagged Pfu DNA Polymerase Mutants**

**Bacterial expression of Pfu mutants.** Plasmid DNA was purified with the StrataPrep® Plasmid Miniprep Kit (Stratagene), and used to transform XL-10 Gold cells. Ampicillin resistant colonies were grown up in 1-5 liters of LB media containing Turbo Amp™ antibiotic (100μg/μl) at 37°C with moderate aeration. The cells were collected by centrifugation and stored at -20°C.

**Purification (His$_6$ tag protocol/batch binding method):** Cells pellets were resuspended in native binding buffer (20mM phosphate (pH 7.8), 500mM NaCl). Egg white lysozyme (100μg/ml) was added and the cells were incubated for 15 minutes on ice. Cell suspensions were subjected to sonication three times with a Bronson Sonifier 250 at a duty cycle of 80% and an output level of 5 for 45 seconds. The suspensions were left on ice to cool between sonication events. The lysate was cleared by centrifugation at 26,890g. The cleared lysates were added to 5mls of ProBond Ni resin (Invitrogen), equilibrated in native binding buffer, and the slurry was incubated for two hours with gentle agitation at 4°C. The resin was settled by low speed centrifugation (800Xg). The resin was washed three times with 4ml of native binding buffer (pH 7.8) by resuspending the resin, rocking the slurry for two minutes, and then separating the resin from the supernatant by gravity centrifugation. The resin was then washed in the same fashion with native wash buffer (20mM phosphate (pH 6.0), 500mum NaCl). Protein was eluted with two 5-ml additions of 350mM Imidazole elution buffer (20mM phosphate, 500mM NaCl, 350mM Imidazole (pH 6.0)) by resuspending the resin, rocking the slurry for five minutes, and then separating the resin from the supernatant by gravity centrifugation. Eluted proteins were spin concentrated using Centricon 30 centrifugal filter devices (Amicon). Protein samples were evaluated for size and purity by SDS-PAGE using Tris-Glycine 4-20% acrylamide gradient gels. Gels were stained with silver stain or Sypro Orange (Molecular Probes).

**Alternative expresssion/purification:** Alternatively, Pfu mutants were subcloned into the pCAL-n-EK vector (Affinity™ Protein Expression and Purification System) which contains an upstream, in-frame calmodulin binding peptide (CBP) tag for purifying fusion proteins with calmodulin agarose. Plasmid DNA was purified with the StrataPrep® Plasmid Miniprep Kit (Stratagene), and used to transform BL21 (DE3) CodonPlus® cells. Ampicillin resistant colonies were grown up in 1-5 liters of LB media containing Turbo Amp™ antibiotic (100μg/μl) at 30°C with moderate aeration. When cultures reached an absorbance at $OD_{600}$ of 0.6 to 1.0, the cells were induced with 1mM IPTG and incubated in the same manner for 2 hours to overnight (16 hours). The cells were collected by centrifugation and stored at -20°C.

Cells pellets were resuspended to an approximate concentration of 0.25g/ml in buffers identical or similar to calcium binding buffer (50mM Tris-HCL (pH 8.0), 150 mM Nad, 1mM magnesium acetate and 2mM CaCl). Egg white lysozyme (100μg/ml) was added and the cells were incubated for 15 minutes on ice. Cell suspensions were subjected to sonication three times with a Bronson Sonifier 250 at a duty cycle of 80% and an output level of 5 for 45 seconds. The suspensions were left on ice to cool between sonication events. The lysate was cleared by centrifugation at 26,890g.

The cleared lysates were added to 1ml of calmodulin agarose (CAM agarose), equilibrated in buffer, and the slurry was incubated with gentle agitation at 4°C. After two hours the reactions were centrifuged at 3000g for 5 minutes to collect the CAM agarose and recombinant protein. The lysate supematent was removed and the CAM agarose was washed at least once by resuspending the resin in 50ml of calcium binding buffer followed by collection of the CAM agarose by centrifugation as described above. The CAM agarose was transferred to a disposable 15m1 column, packed, then washed with at least 200ml of calcium binding buffer. Recombinant proteins were eluted from the column by using a buffer similar or identical to 50mM Tris-HCl (pH 8.0), 1M NaCl, 2mM EGTA.

Protein samples were evaluated for size and purity by SDS-PAGE using Tris-Glycine 4-20% acrylamide gradient gels. Gels were stained with silver stain or Sypro Orange (Molecular Probes).

### Example 3. Assaying DNA Polymerase And 3'-5' Exonuclease Activities Of Pfu DNA Polymerase Mutants

Pfu mutant preparations were assayed for DNA polymerase and 3'-5' exonuclease activities as follows.

**DNA polymerase.** DNA polymerase activity was measured by monitoring incorporation of radiolabelled TTP into activated calf thymus DNA. A suitable DNA polymerase reaction cocktail contained: 1x PCR reaction buffer, 200μM each dATP, dCTP, and dGTP, 195μM TTP, 5μM [$^3$H]TTP (NEN #NET-221H, 20.5Ci/mmole; partially evaporated to remove EtOH), and 250μg/ml of activated calf thymus DNA (e.g., Pharmacia #27-4575-01). DNA polymerases (wt Pfu or Pfu mutants) were diluted in Pfu storage buffer and 1μl of each enzyme dilution was added to 10μl aliquots of polymerase cocktail. Polymerization reactions were conducted in duplicate or triplicate for 30 minutes at 72°C. The extension reactions were quenched on ice, and then 5μl aliquots were spotted immediately onto DE81 ion-exchange filters (2.3cm; Whatman #3658323). Unincorporated [$^3$H]TTP was removed by 6 washes with 2xSCC (0.3M NaCl, 30mM sodium citrate, pH 7.0), followed by a brief wash with 100% ethanol. Incorporated radioactivity was measured by scintillation counting.

Reactions that lack enzyme were set up along with sample incubations to determine "total cpms" (omit filter wash steps) and "minimum cpms"(wash filters as above). Sample cpms were subtracted by minimum cpms to determine "corrected cpms" for each DNA polymerase.

To determine percent (%) activity relative to wild type Pfu, -50-500ng of purified Pfu mutants were assayed in a nucleotide incorporation assay, alongside wild type Pfu diluted serially over the linear range of the assay (50-500pg; 0.003-0.03U).

**Exonuclease assays.** Exonuclease reactions were performed (in triplicate) by adding 4μl aliquots of diluted DNA polymerases (0.25-10U wt Pfu; 5-200ng) to 46μl of reaction cocktail. Reactions were incubated for 1 hour at 72°C. Reactions lacking DNA polymerase were also set up along with sample incubations to determine "total cpms" (no TCA precipitation) and "minimum cpms" (TCA precipitation, see below).

Exonuclease reactions were stopped by transferring the tubes to ice. Sonicated salmon sperm DNA (150μl; 2.5 mg/ml stock) and TCA (200μl; 10% stock) were added to all but the "total cpms" tubes. The precipitation reactions were incubated for ≥15 minutes on ice, and then spun in a microcentrifuge at 14,000rpm for 10 minutes. 200μl of the supernatant was removed, being careful not to disturb the pellet, and transferred to scintillation fluid (Bio-Safe II™, Research Products International Corp.). The samples were thoroughly mixed by inversion and then counted in a scintillation counter.

To determine percent (%) exonuclease activity relative to wild type Pfu, equivalent amounts of Pfu and purified Pfu mutants (which fall in the linear range of the assay; ~5-200ng Pfu) are assayed in an exonuclease assay.

**Results:** Several Pfu mutants exhibited reductions in DNA polymerase activity compared to wild type Pfu, when tested as partially purified (~50% purity) preparations eluted from nickel resins (Table 1). Pfu mutants showing <10% DNA polymerase activity and at least 10% exonuclease activity include the partitioning domain mutants: Y385QSNLH, G387SP, and G388P and the polymerase domain mutants: D405E, T542P, D543G, and K593T. The initial measurements of % DNA polymerase activity shown in Table 1 was considered as approximate estimates, due to the purity of the protein samples tested and uncertainties as to whether all protein amounts tested were in the linear range of the assay.

**Example 4. Purification Of Pfu DNA Polymerase Mutants By Conventional Column Chromatography**

The untagged or affinity-tagged fusions of Pfu K593T and G387P mutants were purified as follows. Cells pellets (12-24 grams) were resuspended in 3 volumes of lysis buffer (buffer A: 50mM Tris HCl (pH 8.2), 1mM EDTA, and 10mM βME). Lysozyme (1 mg/g cells) and PMSF (1mM) were added and the cells were lysed for 1 hour at 4°C. The cell mixture was sonicated, and the debris removed by centrifugation at 15,000 rpm for 30 minutes (4°C). Tween 20 and Igepal CA-630 were added to final concentrations of 0.1% and the supernatant was heated at 72°C for 10 minutes. Heat denatured *E. coli* proteins were then removed by centrifugation at 15,000 rpm for 30 minutes (4°C).

The supernatant was chromatographed on a Q-Sepharose™ Fast Flow column (~5ml column), equilibrated in buffer B (buffer A plus 0.1 % (v/v) Igepal CA-630, and 0.1 % (v/v) Tween 20). Flow-through fractions were collected and then loaded directly onto a P11 Phosphocellulose column (1.6 x 10cm), equilibrated in buffer C (same as buffer B, except pH 7.5). The column was washed and then eluted with a 0-0.7M KCl gradient/Buffer C. Fractions containing Pfu DNA polymerase mutants (95kD by SDS-PAGE) were dialyzed overnight against buffer D (50mM Tris HC1 (pH 7.5), 5mM βME, 5% (v/v) glycerol, 0.2% (v/v) Igepal CA-630, 0.2% (v/v) Tween 20, and 0.5M NaCl) and then applied to a Hydroxyapatite column (1.0 x 1.3 cm; ~1ml), equilibrated in buffer D. The column was washed and Pfu DNA polymerase mutants were eluted with buffer D2 containing 400 mM $KPO_4$, (pH 7.5), 5mM βME, 5% (v/v) glycerol, 0.2% (v/v) Igepal CA-630, 0.2% (v/v) Tween 20, and 0.5 M NaCl. Purified proteins were spin concentrated using Centricon YM30 devices, and exchanged into Pfu final dialysis buffer (50mM Tris-HCl (pH 8.2), 0.1mM EDTA, 1mM dithiothreitol (DTT), 50% (v/v) glycerol, 0.1% (v/v) Igepal CA-630, and 0.1% (v/v) Tween 20).

**Results:** His-tagged and untagged Pfu G387P and K593T mutants were purified by ion exchange/hydroxyappetite (IE/HA) chromotography. The purified protein preps were analyzed by SDS-PAGE and determined to be of ≥95% purity. The IE/HA purified mutants were tested in a nucleotide incorporation assay to more precisely quantify percent remaining DNA polymerase activity. As shown in Table 3, the Pfu G387P mutant exhibits no significant DNA polymerase activity (<100 cpms above background) when up to 1.2μg of protein was assayed. These results indicate that the Pfu G387P mutant exhibits <0.01% of the DNA polymerase activity exhibited by wild type Pfu DNA polymerase. In comparison, the Pfu K593T mutant retains approximately 1-2% of the DNA polymerase activity of wild type Pfu.

Table 3. Residual Polymerase Activity in IE/HA Purified *Pfu* Mutant Preps:

| *Pfu* DNA Polymerase | Amount Assayed (ng) | Corrected cpms | Relative (%) Polymerase Activity | Mean Relative Polymerase Activity |
|---|---|---|---|---|
| **$His_6$-tagged mutant enzyme preps** | | | | |
| Wild type | 25 | 16,661 | 100 | 100 |
| G387P | 240 | 42 | 0.026 | Cpms not significantly (<100cpms) above background; therefore, assume <100/16661 x 25/1200 = <0.01% |
| | 600 | 0 | - | |
| | 1200 | 16 | 0.002 | |
| K593T | 80 | 1228 | 2.3 | 1.8 |
| | 200 | 1774 | 1.3 | |

(continued)

| Untagged mutant enzyme prep | | | | |
|---|---|---|---|---|
| Wild type | 2 | 6134 | 100 | 100 |
| G387P Prep J | 8.4 | 60 | 0.23 | Cpms not significantly (<100cpms) above background; therefore, assume <100/6134 x 2/420 = <0.008% |
| | 42 | 0 | - | |
| | 420 | 8 | 0.0006 | |

**Example 5. Verifying The Presence Of Proofreading Activity In Pfu Mutants Under PCR Conditions**

A qualitative assay was used to verify that $His_6$-tagged Pfu mutants retained 3'-5' exonuclease activity under PCR conditions. In this assay, the 900bp Hα1AT target is amplified with exo- Pfu DNA polymerase (2.5U/50μl) using a forward primer containing a 3'dG, which produces a dG/dG mismatch upon annealing to the DNA template. The amplicon is amplified from human genomic DNA using the forward primer: 5'-GAG.GAG.AGC.AGG.AAA.GGT.GGA.A**G**-3' [SEQ ID NO. 8] (100ng/50μl rxn) and the reverse primer: 5'-GAG.GTA.CAG.GGT.TGA.GGC.TACT.G - 3' [SEQ ID NO. 9] (100ng/50μl rxn). Amplification is carried out in the absence or presence of varying amounts of $His_6$-tagged Pfu mutants on a Perkin/Elmer 9600 thermal cycler with the following program: (1 cycle) 95°C for 2.5 minutes; (30 cycles) 95°C for 40 seconds, 61°C for 10 seconds, 72°C for 2.5 minutes; (1 cycle) 72°C for 7 minutes. In the absence of proofreading activity, $exo^-$ Pfu produces low yields of product, presumably because the enzyme can not efficiently extend a dG/dG mismatch. In the presence of Pfu mutants with proofreading activity, the 3'dG should be excised from the primer, thereby allowing $exo^-$ Pfu to amplify the target in high yields. This PCR assay was used to verify that Pfu mutants tested in fidelity assays retained sufficient proofreading activity under PCR conditions to excise mismatched PCR primers. Moreover, the assay allowed us to determine the range of protein concentrations that could be added to PCR reactions without inhibition of amplification.

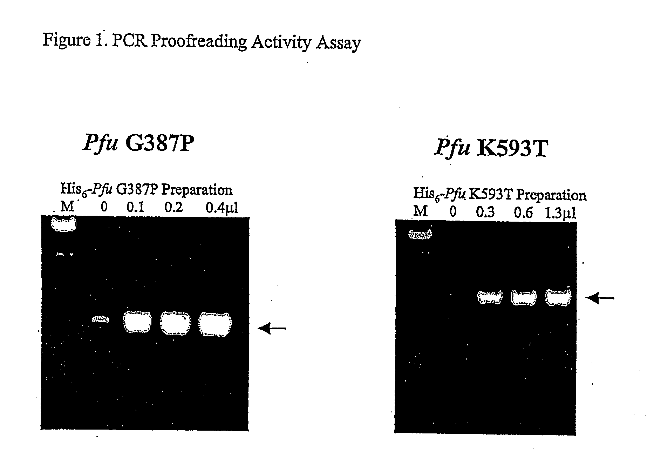

**Results:** As shown in Figure 1, amplifications conducted with $exo^-$ Pfu alone produced low yields of product due to poor extension of the dG/dG mismatch. Product yields were significantly higher in the presence of the $His_6$-tagged Pfu G387P and K593T mutants, presumably because these mutants excise the 3'dG from the primer, thereby allowing $exo^-$ Pfu to efficiently amplify the target. Additional experiments showed that the polymerase deficient Pfu G387P and K593T mutants were unable to amplify the target in the absence of $exo^-$ Pfu (or wild type Pfu).

**Example 6. PCR Amplification With Pfu Or Taq DNA Polymerase Blends Containing** Pfu **Mutants**

**Pfu blends.** PCR reactions were conducted under standard conditions in cloned Pfu PCR buffer (10mM KC1, 10mM $(NH_4)_2SO_4$, 20mM Tris HC1 (pH 8.8), 2mM Mg $SO_4$, 0.1% Triton X-100, and 100μg/ml BSA) with 2.5-SU Pfu*Turbo* DNA polymerase (2.5U/μl cloned Pfu DNA polymerase plus 1U/μl native or 2U/μl cloned *Pyrococcus furiosus* dUTPase (PEF)) and varying concentrations of polymerase deficient Pfu mutants. For genomic targets 0.3-9kb in length, PCR reactions contained 2.5U Pfu*Turbo* DNA polymerase, 100ng of human genomic DNA, 200μM each dNTP, and 100ng of each primer. For genomic targets 11.9kb and 17kb in length, PCR reactions contained 5U Pfu*Turbo* DNA polymerase, 250ng of human genomic DNA, 500μM each dNTP, and 200ng of each primer.

**Taq blends.** PCR reactions were conducted under standard conditions in Herculase PCR buffer (50mM Tricine (pH 9.1), 8mM $(NH_4)_2SO_4$, 2.3mM $MgCl_2$, 0.1% Tween-20, and 75μg/ml BSA) with 2.5U cloned Taq DNA polymerase, 1U of native or 2U cloned *Pyrococcus furiosus* dUTPase (PEF)), and varying concentrations of polymerase deficient Pfu mutants.

**Cycling Conditions (Table 4):**

| Target size (kb) | Target gene | Cycling Parameters |
|---|---|---|
| 0.3 | Aldolase B | (1 cycle) 95°C 2 min<br>(30 cycles) 95°C 40 sec, 58°C 30 sec, 72°C 1 min<br>(1 cycle) 72°C 7 min |
| 0.9 | Hα1AT | (1 cycle) 95°C 2 min<br>(30 cycles) 95°C 40 sec, 58°C 30 sec, 72°C 1 min<br>(1 cycle) 72°C 7 min |

(continued)

| Target size (kb) | Target gene | Cycling Parameters |
|---|---|---|
| 2.3 | Pfu pol (5ng plasmid DNA) | (1 cycle) 95°C 2 min<br>(30 cycles) 95°C 40 sec, 58°C 30 sec, 72°C 3 min<br>(1 cycle) 72°C 7 min |
| 2.6 | Hα1AT | (1 cycle) 95°C 2 min<br>(30 cycles) 95°C 40 sec, 58°C 30 sec, 72°C 3 min<br>(1 cycle) 72°C 7 min |
| 4 | Hα1AT | (1 cycle) 95°C 2 min<br>(30 cycles) 95°C 40 sec, 54°C 30 sec, 72°C 5 min<br>(1 cycle) 72°C 7 min |
| 9.3 | Hα1AT | (1 cycle) 95°C 2 min<br>(30 cycles) 95°C 40 sec, 58°C 30 sec, 72°C 18 min<br>(1 cycle) 72°C 10 min |
| 11.9 | Hα1AT | (1 cycle) 95°C 2 min<br>(30 cycles) 95°C 40 sec, 58°C 30 sec, 72°C 24 min<br>(1 cycle) 72°C 10 min |
| 17 | β globin | (one cycle) 92°C 2 min<br>(10 cycles) 92°C 10 sec, 63°C 30 sec, 68°C 30 min<br>(20 cycles) 92°C 10 sec, 63°C 30 sec, 68°C 30 min (plus 10 sec/cycle)<br>(one cycle) 68°C 10 min |

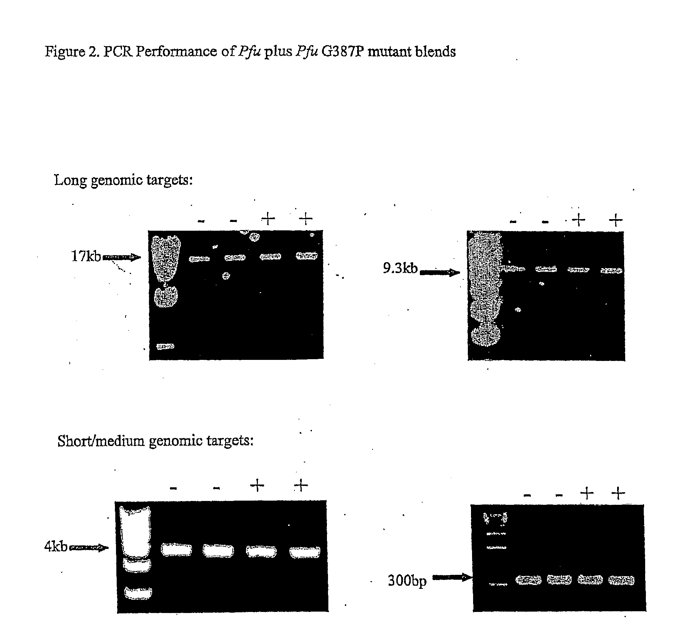

**Results (Pfu blend PCR performance):** As shown in Figure 2, adding 0.5μl of the $His_6$-tagged Pfu G387P mutant to Pfu (in the presence of PEF/dUTPase), has minimal effects on PCR product yield. Additional experiments have shown that up to 1.5μl of the $His_6$-tagged Pfu G387P mutant preparation can be added without significantly reducing PCR product yield.

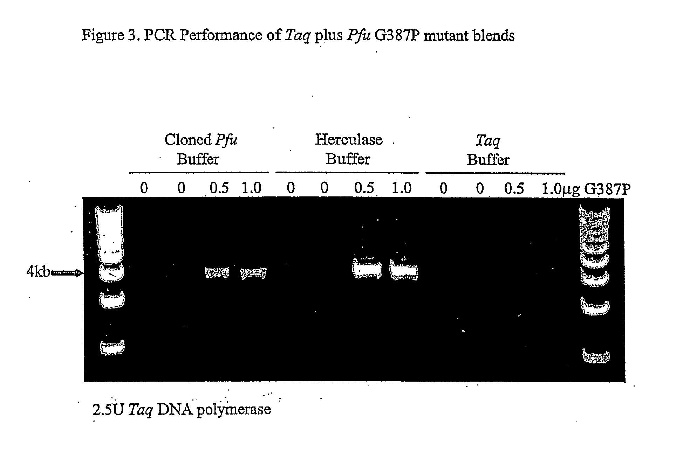

**Results (Taq blend PCR performance):** As shown in Figure 3, adding the $His_6$-tagged Pfu G387P mutant to Taq, in the presence of PEF/dUTPase, significantly increases PCR product yields when amplifications are performed in a reaction buffer that supports the activity of both Taq and Pfu DNA polymerases. One such buffer is the Herculase PCR buffer, which was developed specifically for Herculase Enhanced DNA polymerase (3.33U/μl cloned Pfu, 1.67U/μl cloned Taq, 2U/μl cloned *Pyrococcus furiosus* dUTPase). In the example shown in Figure 3, a 4kb target could not be amplified in high yield using Taq alone in Taq, Pfu, or Herculase PCR buffer. In the presence of the $His_6$-tagged Pfu G387P mutant (and dUTPase), the 4 kb target could be amplified in cloned Pfu buffer (moderate yield) but not Taq buffer, consistent with the buffer preferences of the Pfu G387P mutant. Other experiments have shown that the Pfu G387P mutant inhibits PCR reactions carried out with Taq in Taq PCR buffer, suggesting that the Pfu G387P mutant binds the 3' ends of PCR products without excising mismatches and dissociating (due to inactivity in Taq buffer), and blocks further product extension. As expected, highest product yields are obtained with Taq plus Pfu G387P blends in the presence of Herculase buffer, since both enzymes are highly active in this particular buffer. The Pfu G387P mutant is thought to enhance the yields of Taq PCR reactions (in buffers where Pfu is active) by excising mispairs that would otherwise stall Taq.

**Example 7. Measuring The Fidelity Of DNA Polymerase Blends Containing $His_6$-tagged Pfu DNA Polymerase Mutants**

The error rates of Pfu and Taq blends containing the $His_6$-tagged Pfu G387P and K593T mutants were tested in the *lac*I PCR fidelity assay described in Cline, J., Braman, J.C., and Hogrefe, H.H. (96) NAR 24:3546-3551. Briefly, a 1.9kb fragment encoding the *lacIOlacZα* target gene was amplified from pPRIAZ plasmid DNA using 2.5U Pfu*Turbo* in cloned Pfu PCR buffer or 2.5U Taq in Taq or Herculase PCR buffer. Varying amounts of the Pfu G387P and K593T mutants were added to certain reactions. For comparative purposes, the *lacI* target was also amplified with Pfx (*Thermococcus* sp. KOD DNA polymerase; Invitrogen) and *Tgo* (*Thermococcus gorgonarius* DNA polymerase; Roche) using the manufacturers' recommended PCR buffer. The *lac*I-containing PCR products were then cloned into lambda GT10 arms, and the percentage of *lac*I mutants (MF, mutation frequency) was determined in a color screening assay, as described (Lundberg, K.S., Shoemaker, D.D., Adams, M.W.W., Short, J.M., Sorge, J.A., and Mathur, E.J. (1991) Gene 180:1-8). Error rates are expressed as mutation frequency per bp per duplication (MF/bp/d), where bp is the number of detectable sites in the *lac*I gene sequence (349) and d is the number of effective target doublings. For each enzyme, at least two independent PCR amplifications were performed.

Error rate measurements have shown that Pfu and Pfu*Turbo* DNA polymerases exhibit an average error rate which is ~2-fold lower than that of Vent, Deep Vent, and Pfx (KOD) DNA polymerases, 3 to 6-fold lower than those of DNA polymerase mixtures, and 6- to 12-fold lower than that of Taq DNA polymerase.

**Results (Pfu blend):** As shown Table 5, adding 0.5-3μl of the IE/HA-purified $His_6$-tagged Pfu G387P mutant reduced the error rate of Pfu*Turbo* DNA polymerase by 3.2 to 3.5-fold (assay 1) and by 1.8 to 2.8-fold (assay 2) in two independent fidelity assays. As discussed in Example 5, up to 1.5μl of the IE/HA-purified $His_6$-tagged Pfu G387G mutant can be added to PCR reactions without significantly reducing PCR product yield.

In comparison, adding 0.5μl of the Pfu K593T mutant reduced the error rate of Pfu*Turbo* DNA polymerase slightly (40%), while the addition of 1.5μl and 3.0μl increased error rate by 2.8- and 7.3-fold, respectively. At these amounts, approximately 0.5-1U of additional DNA polymerase activity is added to the PCR reaction (Pfu K593T mutant exhibits 1-2% polymerase activity). The K593T mutation significantly increases the misincorporation or mispair extension rate of Pfu, and when added at high amounts (corresponding to ≥0.5U), the Pfu K593T mutant dramatically increases the error rate of wild type Pfu.

**Results (Taq blend):** As shown Table 6, adding 0.5μl and 3.0μl of the Pfu G387G mutant reduced the error rate of Taq DNA polymerase by 5.1- and 8.3-fold, respectively. Therefore, the error rate of Taq in the presence of the Pfu G387G mutant, can equal the error rate of Pfu alone.

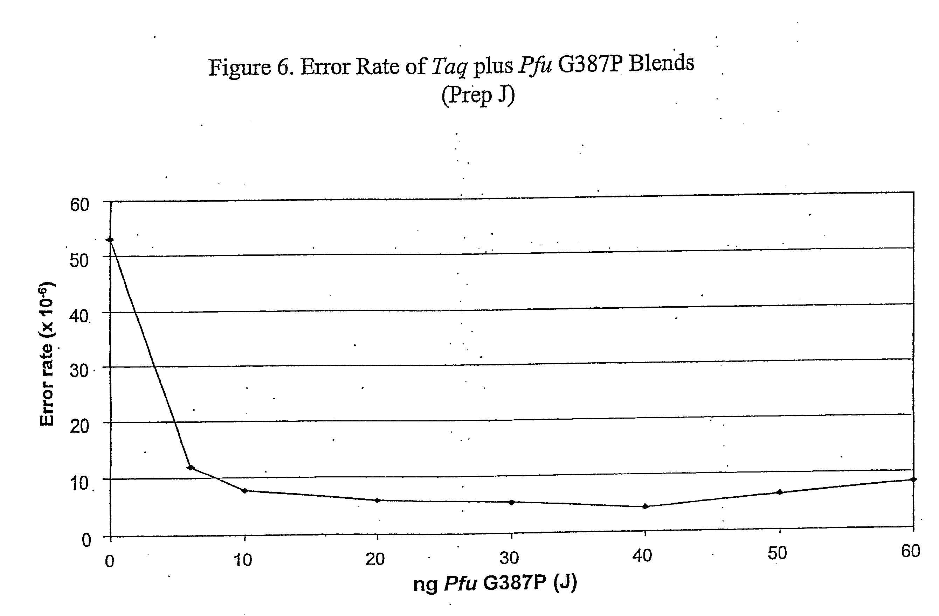

Figure 6. Fidelity of *Pfu* Blends Containing IE/HA Purified $His_6$-*Pfu* Mutants:

| PCR Enzyme | His-*Pfu* Mutant | Mutant Amount (μl) | Error rate* (x $10^{-6}$) | | Mean Relative Accuracy (*Pfu*) |
|---|---|---|---|---|---|
| | | | Assay 1 | Assay 2 | |
| *Pfu* | None | - | 5.55 | 3.60 | 1.0 |
| | G387P | 0.5 | 1.60 | 2.06 | 2.6 |
| | | 1.5 | 1.65 | 1.18 | 3.2 |
| | | 2.0 | Nd | 1.30 | 2.8 |
| | | 3.0 | 1.75 | Nd | 3.2 |
| | K593T | 0.5 | 3.9 | Nd | 1.4 |
| | | 1.5 | 15.7 | Nd | 0.4 |
| | | 3.0 | 40.3 | Nd | 0.1 |
| *Tgo* | None | - | nd | 6.10 | 0.6 |
| *Taq* | None | - | 34.7 | 19.0 | 0.2 |
| *mean of duplicate measurements | | | | | |

Table 6. Fidelity of *Taq* Blends Containing IE/HA Purified *Pfu* Mutants:

| PCR Enzyme | His-*Pfu* Mutant | Mut ant Amo unt (μl) | Error rate* (x $10^{-6}$) | Relative Accuracy (*Pfu*) |
|---|---|---|---|---|
| *Taq* | None | - | 34.7 | 0.16 |
| | G387P | 0.5 | 6.8 | 0.82 |
| | | 3.0 | 4.2 | 1.32 |
| | K593T | 0.5 | 37.0 | 0.15 |
| *Pfu* | None | - | 5.6 | 1.0 |
| | G387P | 0.5 | 1.60 | 3.47 |
| | | 3.0 | 1.75 | 3.17 |

(continued)

| PCR Enzyme | His-*Pfu* Mutant | Mut ant Amo unt (μl) | Error rate* (x $10^{-6}$) | Relative Accuracy (*Pfu*) |
|---|---|---|---|---|
| | K593T | 0.5 | 3.90 | 1.42 |
| *mean of duplicate measurements | | | | |

**Example 8. Determining The TA Cloning Efficiencies Of PCR Products Amplified With Taq In The Presence Of Pfu Mutants**

To determine the effects of polymerase deficient Pfu mutants on the terminal transferase activity of Taq, we amplified a series of amplicons with Taq in the absence of the Pfu G387P mutant (in Taq PCR buffer) or in the presence of the Pfu G387P mutant (in Herculase PCR buffer). Similar amplifications were performed using Pfu*Turbo* and Herculase in their recommended PCR buffers. PCR product yields were quantified by analyzing the products on 1% agarose gels, stained with SYBR gold. The same amount of each PCR product was added to 1μl of the pCR 2.1-TOPO vector (Invitrogen) in a final reaction volume of 6μl, according the manual for the TOPO TA Cloning Kit (#K4500-01). The reactions were incubated for 5 minutes at room temperature, and then transferred to ice. The reactions were transformed into One-Shot cells (Invitrogen), according to the manufacture's recommendations. Aliquots of each transformation were plated on amplicillin/IPTG/X-gal plates, prepared as described in the Invitrogen TOPO TA Cloning manual. The frequency of clones containing the desired insert (% cloning efficiency) was quantified as the number of (white colonies)/(total number of colonies plated).

**Results:** As shown in Table 7, PCR products amplified with Taq in the presence of the Pfu G387P mutant are cloned into the TOPO TA cloning vector as efficiently as PCR products amplified with Taq alone. In contrast, PCR products amplified with Pfu*Turbo* DNA polymerase are cloned into the TOPO TA cloning vector much less efficiently, presumably due to the lack of 3' dAs. As discussed in Example 7, PCR products amplified with Taq blends containing the Pfu G387P mutant, should also exhibit fewer errors (5- to 8-fold less) compared to PCR products amplified with Taq alone. Therefore, Taq blends containing the Pfu G387P mutant should be useful to researchers using TA cloning methods, but desiring high-fidelity amplication of inserts. The high TA cloning efficiencies obtained in the presence of the Pfu G387P mutant indicates that 3'dAs added by Taq during PCR are unexpectedly resistant to exonucleolytic degradation. Presumably, Pfu DNA polymerase is not very efficient at excising 3'dA residues from doublestranded PCR products in the presence of nucleotides.

Table 7. TopoTA Cloning Efficiencies:

| PCR Product (bp) | PCR enzyme/blend | | | Cloning efficiency (%) |
|---|---|---|---|---|
| | DNA polymerase | $His_6$-*Pfu* mutant | | |
| | | mutant | amount (μl) | |
| 900 300 | *Taq* | none | - | 89 |
| | | G387P | 0.5 | 80 |
| | | G387P | 3.0 | 89 |
| | *Pfu* | none | - | 8 |
| | *Taq* | none | - | 69 |
| | | G387P | 0.5 | 73 |
| | | G387P | 3.0 | 78 |
| | *Pfu* | none | - | 33 |
| | Herculase | None | - | 46 |
| 2300 | *Taq* | None | - | 83 |
| | | G387P | 0.5 | 88 |
| | | G387P | 3.0 | 92 |
| | *Pfu* | None | - | 22 |
| | Herculase | None | - | 85 |

**Example 9. Expression And Activity Of Untagged Pfu Mutants**

The $His_6$- tag was deleted from the $His_6$-tagged Pfu G387P clone and the untagged mutant was expressed and purified as described in Example 4. Four Pfu G387P mutant samples were prepared and their protein concentrations determined by amino acid analysis. Exonuclease activity was measured using $^3$H-*E. coli* genomic DNA as substrate and the specific exonuclease activities of the mutant preparations are compared to that of wild type Pfu in Table 8. The specific exonuclease activities of the Pfu G387P mutant preparations ranged from 1300 to 2200 U/mg, and appeared to be somewhat higher than that of wild type Pfu (350-950U/mg).

Table 8. Exonuclease specific activity of Pfu G387P Preparations

| DNA Polymerase | Lot/prep # | Protein concentration (μg/μl) | Exonuclease activity (U/μl) | Exonuclease specific Activity (U/mg) (# assays) | Polymerase activity (U/μl) |
|---|---|---|---|---|---|
| Pfu | 1184447 | ~0.05 | 0.0174 | 348 (1) | 2.5 |
| Pfu | SCS 61 | 2.29 | 2.176 | 950 (1) | 250 |
| Pfu G387P | J | 4.17 | 8.72 | 2090 (5) | 0 |
| Pfu G387P | SCS 1 | 6.8 | 8.86 | 1320 (2) | 0 |
| Pfu G387P | SCS 2 | 3.0 | 5.87 | 1957 (1) | 0 |
| Pfu G387P | SCS 3 | 2.6 | 5.70 | 2192 (1) | 0 |

**Example 10. Measuring The Fidelity Of DNA Polymerase Blends Containing The Untagged *Pfu* G387P Mutant**

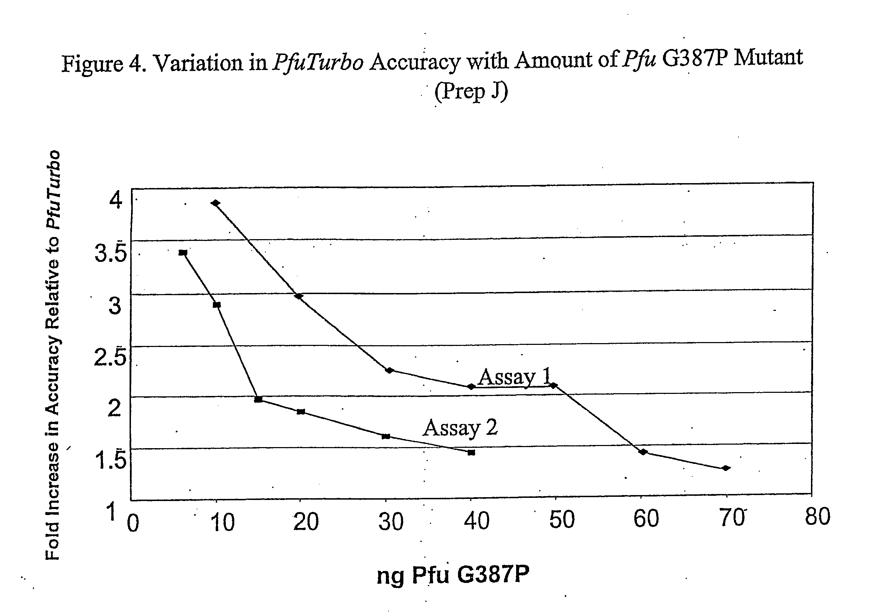

The error rates of *Pfu* and *Taq* blends containing the untagged *Pfu* G387P mutant preparations were tested in the *lac*I PCR fidelity assay as described in Example 7. As shown in Figure 4, the highest reductions in error rate (~3-fold) were observed when 6 to 10ng of *Pfu* G387P prep J was added to 2.5U Pfu (50μl reaction). Unexpectedly, fidelity appeared to decrease with increasing amounts (>10ng) of *Pfu* P387G mutant. The yield of *lac*I amplicon also decreased with increasing amount of *Pfu* P387G mutant, suggesting that lower fidelity may in some way be correlated with reduced yield. Using prep J, optimal fidelity (lowest error rate) was achieved by adding 0.0125U to 0.0208U of exonuclease activity (prep J; 2090U/mg), which is the amount of 3'-5' exonuclease activity exhibited by ~ 1-3U of wild type Pfu. These assumptions are based upon *Pfu* exhibiting a specific activity of 348-950U exonuclease/mg and exo/pol ratios of 0.0174U/2.5U-0.02176U/2.5U, see Table 7.

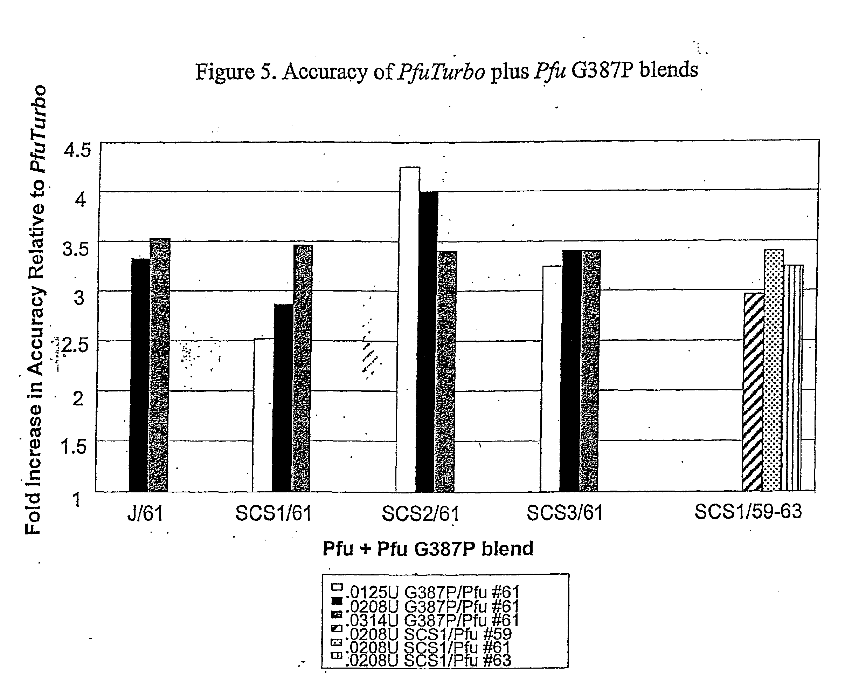

Additional testing with G387P preparations SCS 1-3 showed that 6-24 ng or amounts of protein equivalent to 0.0125U, 0.0209U, or 0.0314U of prep J consistently reduced the error rate of *PfuTurbo* DNA polymerase by ~3-fold (Figure 5). There was minimal variation in error rate with lot of *PfuTurbo* DNA polymerase employed (lots #59, 61, 63).

As shown in Figure 6, adding 6ng to 60ng *Pfu* G387P prep J reduced the error rate of *Taq* DNA polymerase by 4.4- to 12.6-fold. Maximum reduction in error rate was achieved by adding 40ng of prep J, or the equivalent of 0.0836U of exonuclease activity. In this assay, the accuracy of the *Taq* + 40ng *Pfu* G387P blend was 50% higher than that of *PfuTurbo* DNA polymerase.

**Example 11. Range Of Ratios Of Exonuclease And Polymerase Activities To Use In Blends**

| Enzyme blend | | | | | |
|---|---|---|---|---|---|
| **Polymerase proficient enzyme** | | **Polymerase deficient enzyme** | | | |
| Polymerase | Amount Polymerase (3'-5' Exo) | *Pfu* Mutant | Range of Amounts Tested that Produce Highest Fidelity and Yield | | |
| | | | Ng | Polymerase (U) | 3'-5' Exo (U) |
| *Pfu*/ *PfuTurbo* | 2.5 (0.02U exo) | G387P | 5.7-24<br>4<br>preps | <0.01 | 0.008-0.0314 |

(continued)

| Enzyme blend | | | | | | |
|---|---|---|---|---|---|---|
| **Polymerase proficient enzyme** | | **Polymerase deficient enzyme** | | | | |
| Polymerase | Amount Polymerase (3'-5' Exo) | *Pfu* Mutant | Range of Amounts Tested that Produce Highest Fidelity and Yield | | | |
| | | | Ng | Polymerase (U) | 3'-5' Exo (U) | |
| *Taq* | 2.5U (0U exo) | G387P | 20-40 prep J | <0.01 | 0.0418-0.0836 | |

## Claims

1. An enzyme mixture comprising a first enzyme and a second enzyme, wherein the first enzyme is a wild type Taq DNA polymerase, and the second enzyme is a mutant pfu DNA polymerase comprising a 3'-5' exonuclease activity comprising one or more mutations in the polymerization domain at amino acid positions D405E, Y410F, T542P, D543G, K593T, Y595S and within the partitioning domain including amino acids 384-389 (SYTGGF) and wherein the DNA polymerization activity is lower than that of the wild-type enzyme.

2. A method for DNA synthesis, comprising:

(a) providing an enzyme mixture according to claim 1; and
(b) contacting said enzyme mixture with a nucleic acid template, wherein said enzyme mixture permits DNA synthesis.

3. A method for TA cloning of a DNA synthesis product comprising:

(a) providing an enzyme mixture according to claim 1;
(b) contacting said enzyme mixture with a nucleic acid template, wherein said enzyme mixture permits DNA synthesis to generate a synthesised DNA product; and
(c) inserting said synthesised DNA product into a TA cloning vector.

4. A kit comprising the enzyme mixture of claim 1, and packaging material therefore.

5. A kit for DNA synthesis according to the method of claim 2 comprising a first enzyme, a second enzyme, and packaging material therefor, where the first enzyme is a wild-type Taq DNA polymerase, and the second enzyme is a mutant pfu DNA polymerase comprising 3'-5' exonuclease activity comprising one or more mutations in the polymerization domain at amino acid positions D405E, Y410F, T542P, D543G, K593T, Y595S and within the partitioning domain including amino acids 384-389 (SYTGGF) and wherein the DNA polymerization activity is lower than that of the wild-type enzyme.

6. The kit for DNA synthesis of claims 4 or 5, further comprising at least one of polynucleotide precursors, primers, buffers and instructions.

## Patentansprüche

1. Enzymmischung, die ein erstes Enzym und ein zweites Enzym umfasst, wobei das erste Enzym eine Wildtyp-Taq-DNA-Polymerase ist, und das zweite Enzym eine mutierte pfu-DNA-Polymerase ist, die eine 3'-5'-Exonukleaseaktivität aufweist und eine oder mehrere Mutationen in der Polymerisierungsdomäne an den Aminosäure-Positionen D405E, Y410F, T542P, D543G, K593T, Y595S und innerhalb der Partitionierungsdomäne einschließlich Aminosäuren 384-389 (SYTGGF) umfasst, und wobei die DNA-Polymerisierungsaktivität geringer ist als diejenige des Wildtyp-Enzyms.

2. Verfahren zur DNA-Synthese, umfassend:

(a) Bereitstellen einer Enzymmischung nach Anspruch 1; und
(b) in Kontakt bringen der Enzymmischung mit einer Nukleinsäurematrize, wobei die Enzymmischung die DNA-Synthese ermöglicht.

**3.** Verfahren zur TA-Klonierung eines DNA-Syntheseprodukts, umfassend:

(a) Bereitstellen einer Enzymmischung nach Anspruch 1;
(b) in Kontakt bringen der Enzymmischung mit einer Nukleinsäurematrize, wobei die Enzymmischung die DNA-Synthese ermöglicht, um ein synthetisiertes DNA-Produkt zu erzeugen; und
(c) Einfügen des synthetisierten DNA-Produkts in einen TA-Klonierungsvektor.

**4.** Kit, der die Enzymmischung nach Anspruch 1 und Verpackungsmaterial dafür umfasst.

**5.** Kit zur DNA-Synthese gemäß dem Verfahren nach Anspruch 2, der ein erstes Enzym, ein zweites Enzym und Verpackungsmaterial dafür umfasst, wobei das erste Enzym eine Wildtyp-Taq-DNA-Polymerase ist, und das zweite Enzym eine mutierte pfu-DNA-Polymerase ist, die eine 3'-5'-Exonukleaseaktivität aufweist und eine oder mehrere Mutationen in der Polymerisierungsdomäne an den Aminosäure-Positionen D405E, Y410F, T542P, D543G, K593T, Y595S und innerhalb der Partitionierungsdomäne einschließlich Aminosäuren 384-389 (SYTGGF) umfasst, und wobei die DNA-Polymerisierungsaktivität geringer ist als diejenige des Wildtyp-Enzyms.

**6.** Kit zur DNA-Synthese nach Anspruch 4 oder 5, das weiterhin mindestens einen von Polynukleotid-Vorläufern, Primern, Puffern und Instruktionen umfasst.

## Revendications

**1.** Un mélange d'enzyme comprenant un premier enzyme et un second enzyme, dans lequel la première enzyme est un type sauvage d'ADN Taq polymérase, et la seconde enzyme est un mutant de l'ADN pfu polymérase comprenant une activité exonucléase en 3'-5' comprenant une ou plusieurs mutations dans le domaine de polymérisation aux positions des acides aminés D405E, Y410F, T542P, D543G, K593T, Y595S et au sein du domaine de partitionement incluant les acides aminés 384-389 (SYTGGF) et dans laquelle l'activité de polymérisation d'ADN est inférieure à celle de l'enzyme de type sauvage.

**2.** Une méthode pour la synthèse d'ADN comprenant:

(a) fournir un mélange d'enzymes selon la revendication 1; et
(b) mettre en contact ledit mélange d'enzymes avec un modèle d'acide nucléique, dans laquelle ledit mélange d'enzymes permet la synthèse de l'ADN.

**3.** Une méthode pour le clonage TA d'un produit de synthèse d'ADN comprenant:

(a) fournir un mélange d'enzymes selon la revendication 1;
(b) mettre en contact ledit mélange d'enzymes avec un modèle d'acide nucléique, dans laquelle ledit mélange d'enzymes permet à la synthèse de l'ADN de générer un produit de synthèse d'ADN; et
(c) insérer ledit produit de synthèse d'ADN dans le vecteur de clonage TA.

**4.** Un kit comprenant le mélange d'enzymes selon la revendication 1, et le matériau d'emballage de celui-ci.

**5.** Un kit pour la synthèse d'ADN selon la méthode de la revendication 2 comprenant un premier enzyme, un second enzyme, et les matériaux d'emballage de ces derniers, où la première enzyme est un type sauvage d'ADN Taq polymérase, et la seconde enzyme est un mutant de l'ADN pfu polymérase comprenant une activité exonucléase en 3'-5' comprenant une ou plusieurs mutations dans le domaine de polymérisation aux positions des acides aminés D405E, Y410F, T542P, D543G, K593T, Y595S et au sein du domaine de partitionement incluant les acides aminés 384-389 (SYTGGF) et dans laquelle l'activité de polymérisation d'ADN est inférieure à celle de l'enzyme de type sauvage.

**6.** Le kit de synthèse d'ADN selon la revendication 4 ou 5 comprenant en outre au moins un des précurseurs, des amorces, des tampons et des instructions poynucléotidiques.

Figure 1. PCR Proofreading Activity Assay

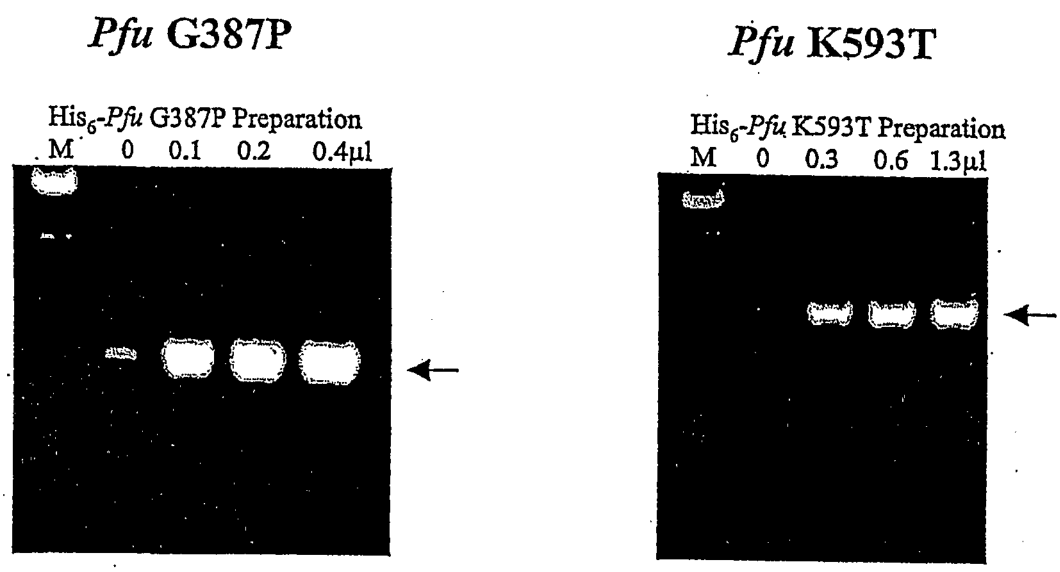

Figure 2. PCR Performance of *Pfu* plus *Pfu* G387P mutant blends

Long genomic targets:

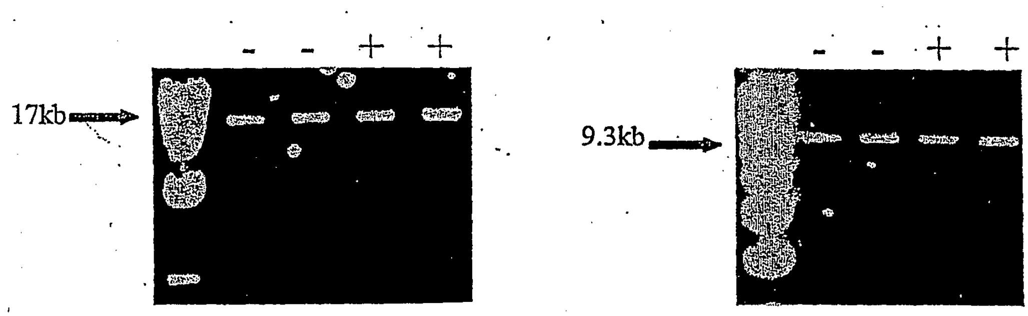


Short/medium genomic targets:

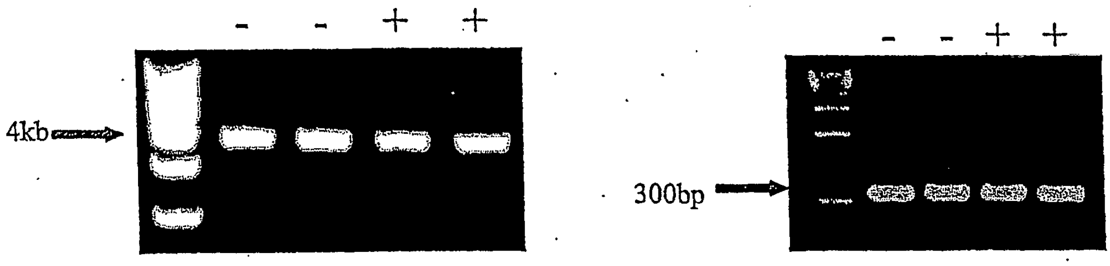

Figure 3. PCR Performance of *Taq* plus *Pfu* G387P mutant blends

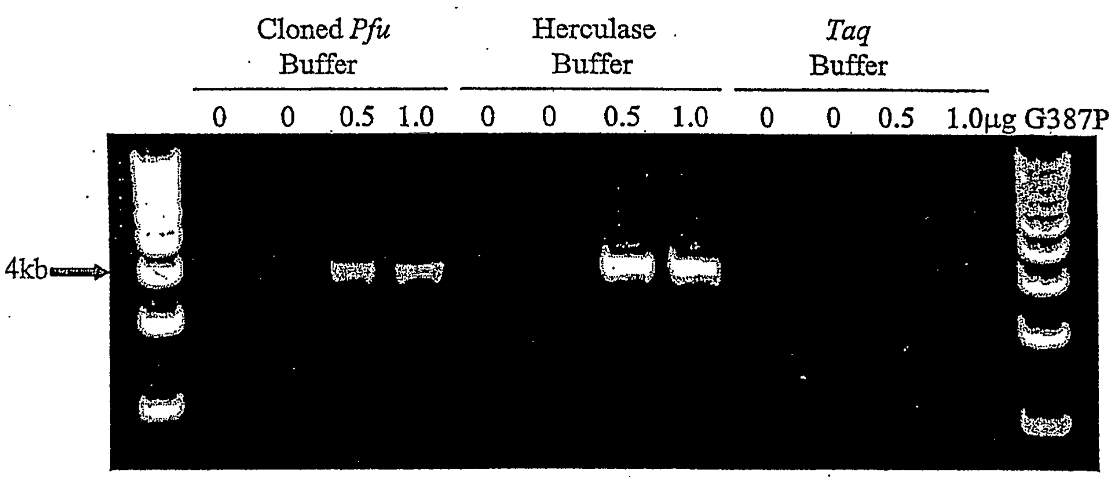


2.5U *Taq* DNA polymerase

Figure 4. Variation in *PfuTurbo* Accuracy with Amount of *Pfu* G387P Mutant (Prep J)

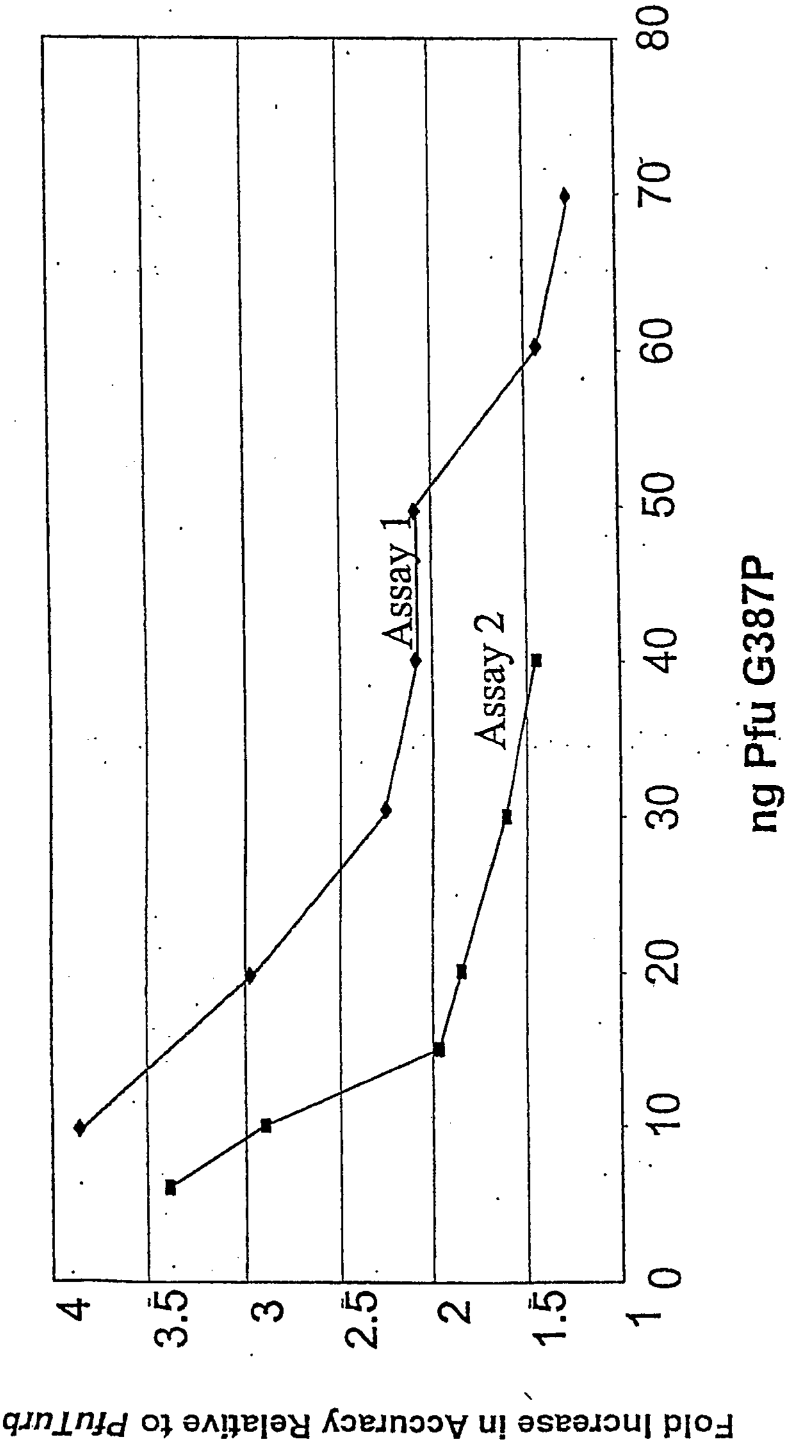

Figure 5. Accuracy of *PfuTurbo* plus *Pfu* G387P blends

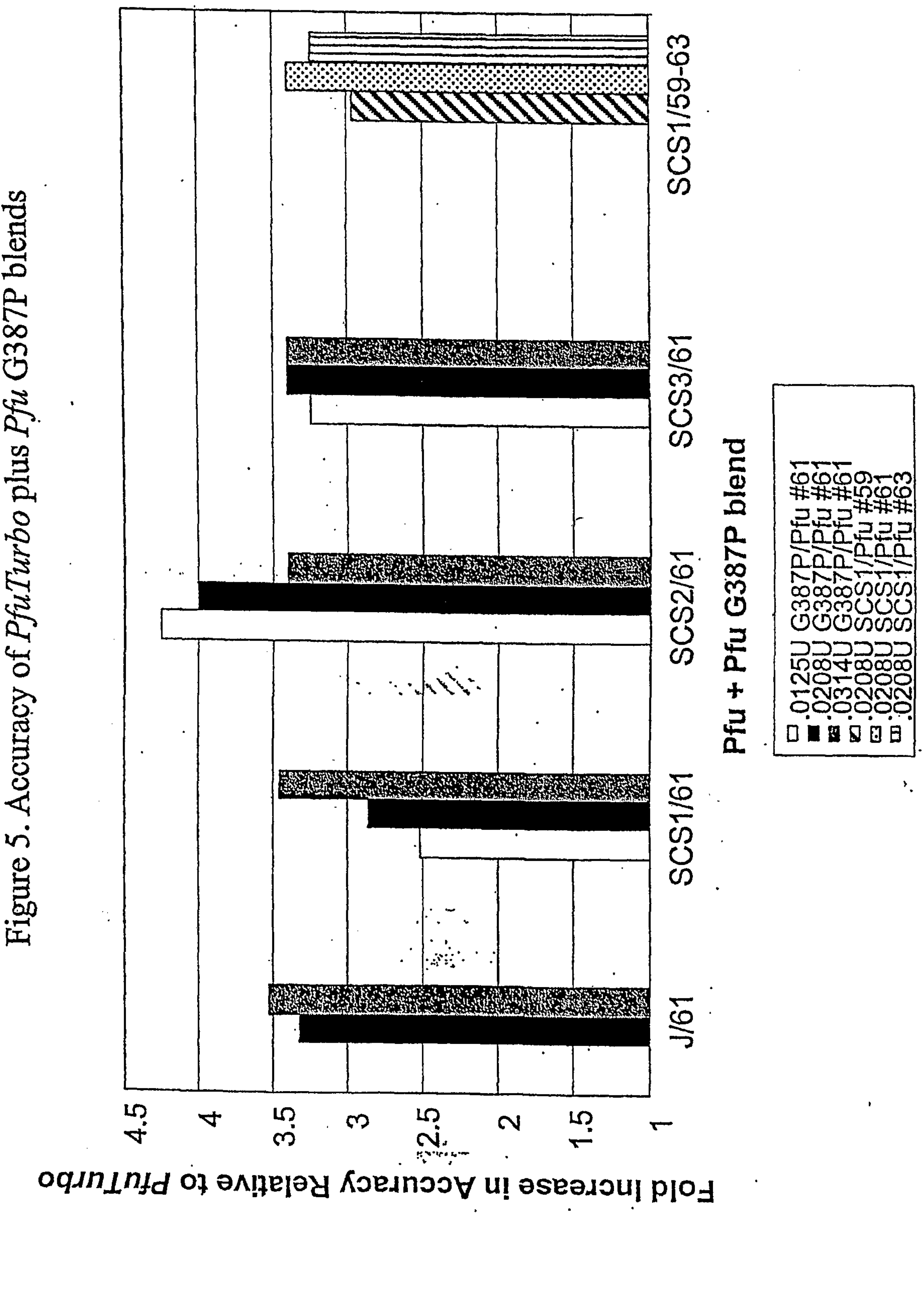

Figure 6. Error Rate of *Taq* plus *Pfu* G387P Blends
(Prep J)

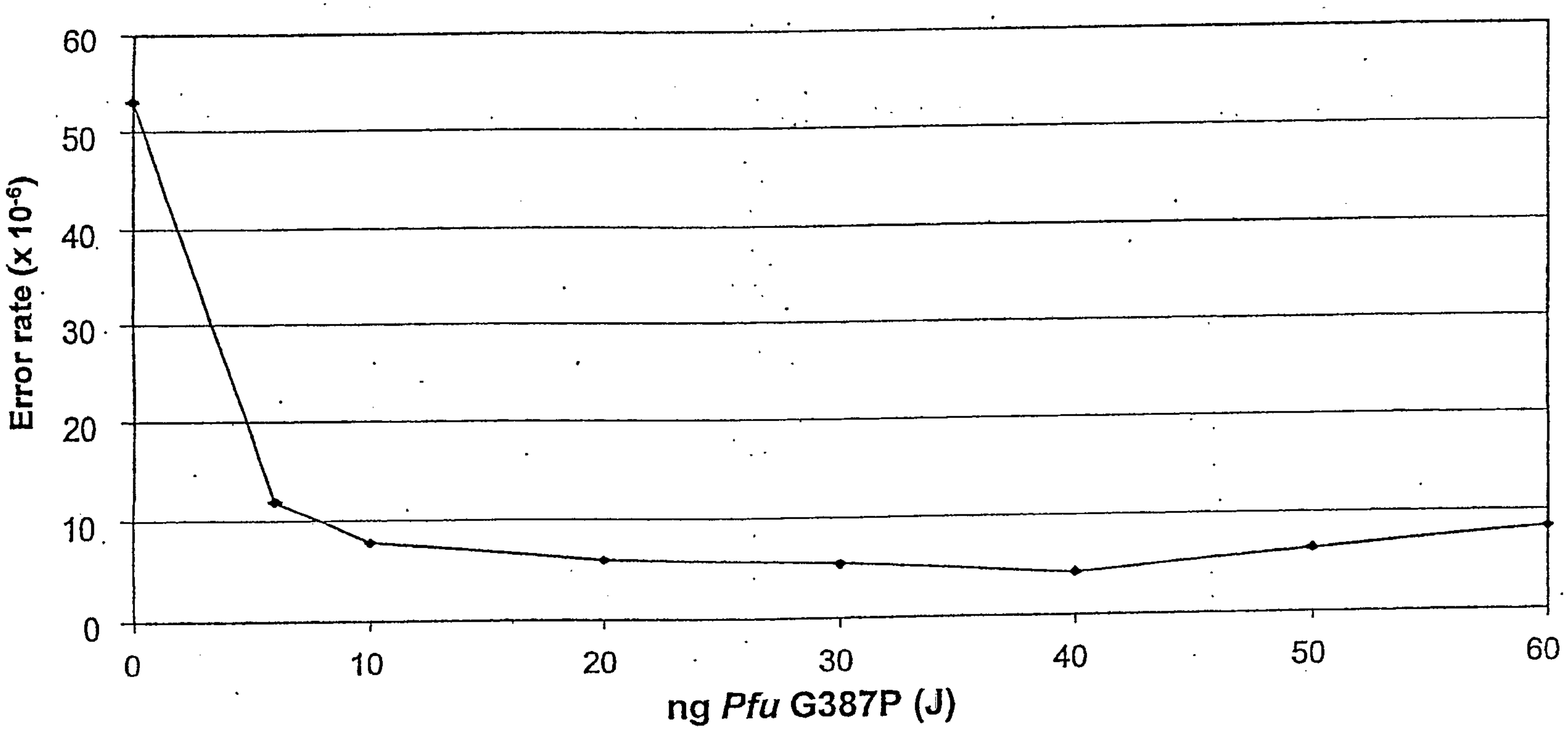

Figure 7 Polynucleotide and polypeptide sequences of various DNA polymerase mutants according to some embodiments of the invention

Partitioning Domain Mutants

>Pfu wild type
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks // [SEQ ID NO. 19]

>Pfu Y385N
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresn
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 20]

>Pfu Y385L
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresl
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 21]

>Pfu Y385H
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresh
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw

seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 22]

>Pfu Y385Q
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresq
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 23]

>Pfu Y385S
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlress
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 24]

>Pfu G387S
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tsgfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 25]

>Pfu G387P
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tpgfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk

ikpgmvigyivlrgdgpisnrailaeeydpkkhkydaeyyienqvlpavlrilegfgyrkedlryqktrqvgltswl
nikks [SEQ ID NO. 26]

>Pfu G388A
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tgafvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 27]

>Pfu G388P
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tgpfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 28]

>Tgo wild type
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresya
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 29]

>Tgo Y384N
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresna
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki

rpgtvisyivlkgsgrigdraipfdefdpakhkydaeyyienqvlpaverilrafgyrkedlryqktrqvglgawlk
pkt [SEQ ID NO. 30]

>Tgo Y384L
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresla
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 31]

>Tgo Y384H
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresha
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 32]

>Tgo Y384Q
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresqa
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 33]

>Tgo Y384S
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarressa
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki

rpgtvisyivlkgsgrigdraipfdefdpakhkydaeyyienqvlpaverilrafgyrkedlryqktrqvglgawlk
pkt [SEQ ID NO. 34]

>Tgo G386S
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
sgyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 35]

>Tgo G386P
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
pgyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 36]

>Tgo G387A
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
gayvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 37]

>Tgo G387P
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
gpyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki

rpgtvisyivlkgsgrigdraipfdefdpakhkydaeyyienqvlpaverilrafgyrkedlryqktrqvglgawlk
pkt [SEQ ID NO. 38]

>KOD wild type
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqsye
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 39]

>KOD Y384N
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqsne
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 40]

>KOD Y384L
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqsle
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 41]

>KOD Y384H
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqshe
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki

rpgtvisyivlkgsgrigdraipfdefdptkhkydaeyyienqvlpaverilrafgyrkedlryqktrqvglsawlk
pkgt [SEQ ID NO. 42]

>KOD Y384Q
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsqe
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 43]

>KOD Y384S
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsse
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 44]

>KOD G386S
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
sgyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 45]

>KOD G386P
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
pgyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki

rpgtvisyivlkgsgrigdraipfdefdptkhkydaeyyienqvlpaverilrafgyrkedlryqktrqvglsawlk
pkgt [SEQ ID NO. 46]

>KOD G387A
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
gayvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklvihеqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 47]

>KOD G387P
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
gpyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklvihеqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 48]

>Vent wild type
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklvihеqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 49]

>Vent Y387N
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tnlggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklvihеqitrdlkdykaigphvaiakrlaarg

ikvkpgtiisyivlkgsgkisdrvillteydprkhkydpdyyienqvlpavlrileafgyrkedlryqsskqtglda
wlkr [SEQ ID NO. 50]

>Vent Y387L
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tllggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 51]

>Vent Y387H
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
thlggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 52]

>Vent Y387Q
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tqlggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 53]

>Vent Y387S
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tslggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg

ikvkpgtiisyivlkgsgkisdrvillteydprkhkydpdyyienqvlpavlrileafgyrkedlryqsskqtglda
wlkr [SEQ ID NO. 54]

>Vent G389S
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylsgyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 55]

>Vent G389P
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylpgyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 56]

>Vent G390A
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylgayvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 57]

>Vent G390P
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylgpyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg

ikvkpgtiisyivlkgsgkisdrvillteydprkhkydpdyyienqvlpavlrileafgyrkedlryqsskqtglda
wlkr [SEQ ID NO. 58]

>Deep Vent wild type
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 59]

>Deep Vent Y385N
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresn
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 60]

>Deep Vent Y385L
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresl
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 61]

>Deep Vent Y385H
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresh
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk

vrpgmvigyivlrgdgpiskrailaeefdlrkhkydaeyyienqvlpavlrileafgyrkedlrwqktkqtgltawl
nikkk [SEQ ID NO. 62]

>Deep Vent Y385Q
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresq
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 63]

>Deep Vent Y385S
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlress
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 64]

>Deep Vent G387S
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
asgyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 65]

>Deep Vent G387P
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
apgyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk

vrpgmvigyivlrgdgpiskrailaeefdlrkhkydaeyyienqvlpavlrileafgyrkedlrwqktkqtgltawl
nikkk [SEQ ID NO. 66]

>Deep Vent G388A
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
agayvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 67]

>Deep Vent G388P
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
agpyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 68]

**Polymerase Domain Mutants**

>Pfu D405E
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tggfvkepekglwenivylefralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 69]

>Pfu T542P
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idpdglyatipggeseeikkkalefvkyinsklpglleleyegfykrgffvtkkryavideegkvitrgleivrrdw

seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 70]

>Pfu D543G
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtgglyatipggeseeikkkalefvkyinsklpglleleyegfykrgffvtkkryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 71]

>Pfu K593T
mildvdyiteegkpvirlfkkengkfkiehdrtfrpyiyallrddskieevkkitgerhgkivrivdvekvekkflg
kpitvwklylehpqdvptirekvrehpavvdifeydipfakrylidkglipmegeeelkilafdietlyhegeefgk
gpiimisyadeneakvitwknidlpyvevvsseremikrflriirekdpdiivtyngdsfdfpylakraeklgiklt
igrdgsepkmqrigdmtavevkgrihfdlyhvitrtinlptytleavyeaifgkpkekvyadeiakawesgenlerv
akysmedakatyelgkeflpmeiqlsrlvgqplwdvsrsstgnlvewfllrkayernevapnkpseeeyqrrlresy
tggfvkepekglwenivyldfralypsiiithnvspdtlnlegcknydiapqvghkfckdipgfipsllghlleerq
kiktkmketqdpiekilldyrqkaikllansfygyygyakarwyckecaesvtawgrkyielvwkeleekfgfkvly
idtdglyatipggeseeikkkalefvkyinsklpglleleyegfykrgffvtktryavideegkvitrgleivrrdw
seiaketqarvletilkhgdveeavrivkeviqklanyeippeklaiyeqitrplheykaigphvavakklaakgvk
ikpgmvigyivlrgdgpisnrailaeeydpkkhkydaeyyienqvlpavlrilegfgyrkedlryqktrqvgltswl
nikks [SEQ ID NO. 72]

>Tgo D404E
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresya
ggyvkeperglwenivylefrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 73]

>Tgo T541P
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarresya
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dpdgffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki

rpgtvisyivlkgsgrigdraipfdefdpakhkydaeyyienqvlpaverilrafgyrkedlryqktrqvglgawlk
pkt [SEQ ID NO. 74]

>Tgo D542G
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtggffatipgadaetvkkkakefldyinaklpglleleyegfykrgffvtkkkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 75]

>Tgo K592T
mildtdyitedgkpvirifkkengefkidydrnfepyiyallkddsaiedvkkitaerhgttvrvvraekvkkkflg
rpievwklyfthpqdvpairdkikehpavvdiyeydipfakrylidkglipmegdeelkmlafdietlyhegeefae
gpilmisyadeegarvitwknidlpyvdvvstekemikrflkvvkekdpdvlityngdnfdfaylkkrseklgvkfi
lgregsepkiqrmgdrfavevkgrihfdlypvirrtinlptytleavyeaifgqpkekvyaeeiaqawetgeglerv
arysmedakvtyelgkeffpmeaqlsrlvgqslwdvsrsstgnlvewfllrkayernelapnkpderelarrresya
ggyvkeperglwenivyldfrslypsiiithnvspdtlnregceeydvapqvghkfckdfpgfipsllgdlleerqk
vkkkmkatidpiekklldyrqraikilansfygyygyakarwyckecaesvtawgrqyiettireieekfgfkvlya
dtdgffatipgadaetvkkkakefldyinaklpglleleyegfykrgffvtktkyavideedkittrgleivrrdws
eiaketqarvleailkhgdveeavrivkevteklskyevppeklviyeqitrdlkdykatgphvavakrlaargiki
rpgtvisyivlkgsgrigdraipfdefdpakhkydaeyyienqvlpaverilrafgyrkedlryqktrqvglgawlk
pkt [SEQ ID NO. 76]

>KOD D404E
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqsye
ggyvkeperglwenivylefrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 77]

>KOD T541P
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrrqsye
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dpdgffatipgadaetvkkkameflnyinaklpgaleleyegfykrgffvtkkkyavideegkittrgleivrrdws
eiaketqarvleallkdgdvekavrivkevteklskyevppeklviheqitrdlkdykatgphvavakrlaargvki

rpgtvisyivlkgsgrigdraipfdefdptkhkydaeyyienqvlpaverilrafgyrkedlryqktrqvglsawlk
pkgt [SEQ ID NO. 78]

>KOD D542G
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtggffatipgadaetvkkkameflnyinaklpgaleleyegfykrgffvtkkkyavideegkittrgleivrrdws
eiaketqarvleallkdgdvekavrivkevteklskyevppeklvihegitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 79]

>KOD K592T
mildtdyitedgkpvirifkkengefkieydrtfepyfyallkddsaieevkkitaerhgtvvtvkrvekvqkkflg
rpvevwklyfthpqdvpairdkirehgavidiyeydipfakrylidkglvpmegdeelkmlafdiqtlyhegeefae
gpilmisyadeegarvitwknvdlpyvdvvsteremikrflrvvkekdpdvlityngdnfdfaylkkrceklginfa
lgrdgsepkiqrmgdrfavevkgrihfdlypvirrtinlptytleavyeavfgqpkekvyaeeitpawetgenlerv
arysmedakvtyelgkeflpmeaqlsrligqslwdvsrsstgnlvewfllrkayernelapnkpdekelarrqsye
ggyvkeperglwenivyldfrslypsiiithnvspdtlnregckeydvapqvghrfckdfpgfipsllgdlleerqk
ikkkmkatidpierklldyrqraikilansyygyygyararwyckecaesvtawgreyitmtikeieekygfkviys
dtdgffatipgadaetvkkkameflnyinaklpgaleleyegfykrgffvtktkyavideegkittrgleivrrdws
eiaketqarvleallkdgdvekavrivkevteklskyevppeklvihegitrdlkdykatgphvavakrlaargvki
rpgtvisyivlkgsgrigdraipfdefdptkhkydaeyyienqvlpaverilrafgyrkedlryqktrqvglsawlk
pkgt [SEQ ID NO. 80]

>Vent D407E
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylggyvkepekglweniiylefrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtkkryavideegrittrglevvrr
dwseiaketqakvleailkegsvekavevvrdvvekiakyrvpleklvihegitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 81]

>Vent T544P
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadpdgfyatipgekpelikkkakeflnyinsklpglleleyegfylrgffvtkkryavideegrittrglevvrr
dwseiaketqakvleailkegsvekavevvrdvvekiakyrvpleklvihegitrdlkdykaigphvaiakrlaarg

ikvkpgtiisyivlkgsgkisdrvillteydprkhkydpdyyienqvlpavlrileafgyrkedlryqsskqtglda
wlkr [SEQ ID NO. 82]

>Vent D545G
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtggfyatipgekpelikkkakeflnyinsklpglleleyegfylrgffvtkkryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 83]

>Vent K595T
mildtdyitkdgkpiirifkkengefkieldphfqpyiyallkddsaieeikaikgerhgktvrvldavkvrkkflg
revevwklifehpqdvpamrgkirehpavvdiyeydipfakrylidkglipmegdeelkllafdietfyhegdefgk
geiimisyadeeearvitwknidlpyvdvvsneremikrfvqvvkekdpdviityngdnfdlpylikraeklgvrlv
lgrdkehpepkiqrmgdsfaveikgrihfdlfpvvrrtinlptytleavyeavlgktksklgaeeiaaiweteesmk
klaqysmedaratyelgkeffpmeaelakligqsvwdvsrsstgnlvewyllrvayarnelapnkpdeeeykrrlrt
tylggyvkepekglweniiyldfrslypsiivthnvspdtlekegcknydvapivgyrfckdfpgfipsilgdliam
rqdikkkmkstidpiekkmldyrqraikllansyygymgypkarwyskecaesvtawgrhyiemtireieekfgfkv
lyadtdgfyatipgekpelikkkakeflnyinsklpglleleyegfylrgffvtktryavideegrittrglevvrr
dwseiaketqakvleailkegsvekavevvrdvvekiakyrvpleklviheqitrdlkdykaigphvaiakrlaarg
ikvkpgtiisyivlkgsgkisdrvillteydprkhkydpdyyienqvlpavlrileafgyrkedlryqsskqtglda
wlkr [SEQ ID NO. 84]

>Deep Vent D405E
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvslefrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 85]

>Deep Vent T542P
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idpdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk

vrpgmvigyivlrgdgpiskrailaeefdlrkhkydaeyyienqvlpavlrileafgyrkedlrwqktkqtgltawl
nikkk [SEQ ID NO. 86]

>Deep Vent D543G
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtgglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 87]

>Deep Vent K593T
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtktkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 88]

**Pfu DNA polymerase nucleotide sequence**

atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacgggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag ggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacagg tggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg ggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328 [SEQ ID NO. 89]

Pfu Y385N NNN=AAT, AAC (All possible N codons)
Pfu Y385L NNN=TTA, TTG, CTT, CTC, CTA, CTG (All possible L codons)
Pfu Y385H NNN= CAT, CAC (All possible H codons)
Pfu Y385Q NNN= CAA, CAG (All possible Q codons)
Pfu Y385S NNN= TCT, TCC, TCA, TCG, AGT, AGC (All possible S codons)

atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacgggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360

```
ctcatcgaca aaggcctaat accaatggag ggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gcNNNacagg tggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg ggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328  [SEQ ID NO. 90]
```

Pfu G387S NNN= TCT, TCC, TCA, TCG, AGT, AGC (All possible S codons)
Pfu G387P NNN= CCT, CCA, CCG, CCC (All possible P codons)

```
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacggggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag ggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
```

```
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacaNN Nggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg ggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328  [SEQ ID NO. 91]
```

Pfu G388A NNN= GCA, GCT, GCC, GCG (All possible A codons)
Pfu G388P NNN= CCT, CCA, CCG, CCC (All possible P codons)

```
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacggggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag ggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacagg tNNNttcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
```

```
gcaaaagcaa gatggtactg taaggagtgt gctgagagcg ttactgcctg gggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328  [SEQ ID NO. 92]
```

Pfu D405E NNN= GAA, GAG (All possible E codons)

```
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacgggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag ggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacagg tggattcgtt aaagagccag aaaaggggtt gtgggaaaac 1200
atagtatacc taNNNtttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg gggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
```

tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328 [SEQ ID NO. 93]

Pfu T542P NNN= CCT, CCA, CCG, CCC (All possible P codons)
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacgggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag gggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacagg tggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg gggaagaaag 1560
tacatcgagt tagtatggaa ggagctcgaa gaaaagtttg gatttaaagt cctctacatt 1620
gacNNNgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328 [SEQ ID NO. 94]

Pfu D543G NNN=GGT, GGC, GGA, GGG (All possible G codons)
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacgggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag gggaagaag agctaaagat tcttgccttc 420

```
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
ctcagggaga gctacacagg tggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg gggaagaaag 1560
tacatcgagt tagtatggaa ggagctcgaa gaaaagtttg gatttaaagt cctctacatt 1620
gacactNNNg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagaaga ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328  [SEQ ID NO. 95]
```

Pfu K593T NNN=ACT, ACC, ACA, ACG (All possible T codons)

```
atgattttag atgtggatta cataactgaa gaaggaaaac ctgttattag gctattcaaa 60
aaagagaacg gaaaatttaa gatagagcat gatagaactt ttagaccata catttacgct 120
cttctcaggg atgattcaaa gattgaagaa gttaagaaaa taacggggga aaggcatgga 180
aagattgtga gaattgttga tgtagagaag gttgagaaaa agtttctcgg caagcctatt 240
accgtgtgga aactttattt ggaacatccc caagatgttc ccactattag agaaaaagtt 300
agagaacatc cagcagttgt ggacatcttc gaatacgata ttccatttgc aaagagatac 360
ctcatcgaca aaggcctaat accaatggag gggggaagaag agctaaagat tcttgccttc 420
gatatagaaa ccctctatca cgaaggagaa gagtttggaa aaggcccaat tataatgatt 480
agttatgcag atgaaaatga agcaaaggtg attacttgga aaaacataga tcttccatac 540
gttgaggttg tatcaagcga gagagagatg ataaagagat ttctcaggat tatcagggag 600
aaggatcctg acattatagt tacttataat ggagactcat tcgcattccc atatttagcg 660
aaaagggcag aaaaacttgg gattaaatta accattggaa gagatggaag cgagcccaag 720
atgcagagaa taggcgatat gacggctgta gaagtcaagg gaagaataca tttcgacttg 780
tatcatgtaa taacaaggac aataaatctc ccaacataca cactagaggc tgtatatgaa 840
gcaatttttg gaaagccaaa ggagaaggta tacgccgacg agatagcaaa agcctgggaa 900
agtggagaga accttgagag agttgccaaa tactcgatgg aagatgcaaa ggcaacttat 960
gaactcggga aagaattcct tccaatggaa attcagcttt caagattagt tggacaacct 1020
ttatgggatg tttcaaggtc aagcacaggg aaccttgtag agtggttctt acttaggaaa 1080
gcctacgaaa gaaacgaagt agctccaaac aagccaagtg aagaggagta tcaaagaagg 1140
```

```
ctcagggaga gctacacagg tggattcgtt aaagagccag aaaaggggtt gtgggaaaac 1200
atagtatacc tagattttag agccctatat ccctcgatta taattaccca caatgtttct 1260
cccgatactc taaatcttga gggatgcaag aactatgata tcgctcctca agtaggccac 1320
aagttctgca aggacatccc tggttttata ccaagtctct tgggacattt gttagaggaa 1380
agacaaaaga ttaagacaaa aatgaaggaa actcaagatc ctatagaaaa aatactcctt 1440
gactatagac aaaaagcgat aaaactctta gcaaattctt tctacggata ttatggctat 1500
gcaaaagcaa gatggtactg taaggagtgt gctgagagcg ttactgcctg gggaagaaag 1560
tacatcgagt tagtatggaa ggagctcgaa gaaaagtttg gatttaaagt cctctacatt 1620
gacactgatg gtctctatgc aactatccca ggaggagaaa gtgaggaaat aaagaaaaag 1680
gctctagaat ttgtaaaata cataaattca aagctccctg gactgctaga gcttgaatat 1740
gaagggtttt ataagagggg attcttcgtt acgaagNNNa ggtatgcagt aatagatgaa 1800
gaaggaaaag tcattactcg tggtttagag atagttagga gagattggag tgaaattgca 1860
aaagaaactc aagctagagt tttggagaca atactaaaac acggagatgt tgaagaagct 1920
gtgagaatag taaaagaagt aatacaaaag cttgccaatt atgaaattcc accagagaag 1980
ctcgcaatat atgagcagat aacaagacca ttacatgagt ataaggcgat aggtcctcac 2040
gtagctgttg caaagaaact agctgctaaa ggagttaaaa taaagccagg aatggtaatt 2100
ggatacatag tacttagagg cgatggtcca attagcaata gggcaattct agctgaggaa 2160
tacgatccca aaaagcacaa gtatgacgca gaatattaca tggagaacca ggttcttcca 2220
gcggtactta ggatattgga gggatttgga tacagaaagg aagacctcag ataccaaaag 2280
acaagacaag tcggcctaac ttcctggctt aacattaaaa aatcctag 2328. [SEQ ID NO. 96]
```

KOD DNA polymerase wild type

```
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca tacccttcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggagg ctatgtaaaa gagcccgaga gagggttgtg ggagaacata 1200
gtgtacctag attttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 97]
```

KOD Y384N NNN=AAT, AAC (All possible N codons)
KOD Y384L NNN=TTA, TTG, CTT, CTC, CTA, CTG (All possible L codons)
KOD Y384H NNN= CAT, CAC (All possible H codons)
KOD Y384Q NNN= CAA, CAG (All possible Q codons)
KOD Y384S NNN= TCT, TCC, TCA, TCG, AGT, AGC (All possible S codons)

```
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca tacccttcgc caagcgctac 360
```

ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagcN NNgaaggagg ctatgtaaaa gagcccgaga gagggttgtg ggagaacata 1200
gtgtacctag attttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 98]

KOD G386S NNN= TCT, TCC, TCA, TCG, AGT, AGC (All possible S codons)
KOD G386P NNN= CCT, CCA, CCG, CCC (All possible P codons)
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca tacccttcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020

ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaNNNgg ctatgtaaaa gagcccgaga gagggttgtg ggagaacata 1200
gtgtacctag attttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 99]

KOD G387A NNN= GCA, GCT, GCC, GCG (All possible A codons)
KOD G387P NNN= CCT, CCA, CCG, CCC (All possible P codons)
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca taccettcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggaNN Ntatgtaaaa gagcccgaga gagggttgtg ggagaacata 1200
gtgtacctag attttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680

atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 100]

KOD D404E NNN= GAA, GAG (All possible E codons)
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca taccctttcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggagg ctatgtaaaa gagcccgaga gagggttgtg ggagaacata 1200
gtgtacctaN NNtttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 101]

KOD T541P NNN= CCT, CCA, CCG, CCC (All possible P codons)
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca taccottcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggagg ctatgtaaaa gagcccgaga gagggttgtg ggagaacata 1200
gtgtacctag attttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
NNNgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 102]

KOD D542G NNN=GGT, GGC, GGA, GGG (All possible G codons)
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca taccottcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660

```
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggagg ctatgtaaaa gagcccgaga gagggttgtg ggagaacata 1200
gtgtacctag atttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accNNNggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagaagaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga 2325 [SEQ ID NO. 103]
```

**KOD K592T NNN=ACT, ACC, ACA, ACG (All possible T codons)**

```
atgatcctcg acactgacta cataaccgag gatggaaagc ctgtcataag aattttcaag 60
aaggaaaacg gcgagtttaa gattgagtac gaccggactt ttgaacccta cttctacgcc 120
ctcctgaagg acgattctgc cattgaggaa gtcaagaaga taaccgccga gaggcacggg 180
acggttgtaa cggttaagcg ggttgaaaag gttcagaaga agttcctcgg gagaccagtt 240
gaggtctgga aactctactt tactcatccg caggacgtcc cagcgataag ggacaagata 300
cgagagcatc cagcagttat tgacatctac gagtacgaca tacccttcgc caagcgctac 360
ctcatagaca agggattagt gccaatggaa ggcgacgagg agctgaaaat gctcgccttc 420
gacattgaaa ctctctacca tgagggcgag gagttcgccg aggggccaat ccttatgata 480
agctacgccg acgaggaagg ggccagggtg ataacttgga agaacgtgga tctcccctac 540
gttgacgtcg tctcgacgga gagggagatg ataaagcgct tcctccgtgt tgtgaaggag 600
aaagacccgg acgttctcat aacctacaac ggcgacaact tcgacttcgc ctatctgaaa 660
aagcgctgtg aaaagctcgg aataaacttc gccctcggaa gggatggaag cgagccgaag 720
attcagagga tgggcgacag gtttgccgtc gaagtgaagg gacggataca cttcgatctc 780
tatcctgtga taagacggac gataaacctg cccacataca cgcttgaggc cgtttatgaa 840
gccgtcttcg gtcagccgaa ggagaaggtt tacgctgagg aaataaccac agcctgggaa 900
accggcgaga accttgagag agtcgcccgc tactcgatgg aagatgcgaa ggtcacatac 960
gagcttggga aggagttcct tccgatggag gcccagcttt ctcgcttaat cggccagtcc 1020
ctctgggacg tctcccgctc cagcactggc aacctcgttg agtggttcct cctcaggaag 1080
gcctatgaga ggaatgagct ggccccgaac aagcccgatg aaaaggagct ggccagaaga 1140
cggcagagct atgaaggagg ctatgtaaaa gagcccgaga gagggttgtg ggagaacata 1200
gtgtacctag atttagatc cctgtacccc tcaatcatca tcacccacaa cgtctcgccg 1260
gatacgctca acagagaagg atgcaaggaa tatgacgttg ccccacaggt cggccaccgc 1320
ttctgcaagg acttcccagg atttatcccg agcctgcttg gagacctcct agaggagagg 1380
```

```
cagaagataa agaagaagat gaaggccacg attgacccga tcgagaggaa gctcctcgat 1440
tacaggcaga gggccatcaa gatcctggca aacagctact acggttacta cggctatgca 1500
agggcgcgct ggtactgcaa ggagtgtgca gagagcgtaa cggcctgggg aagggagtac 1560
ataacgatga ccatcaagga gatagaggaa aagtacggct ttaaggtaat ctacagcgac 1620
accgacggat tttttgccac aatacctgga gccgatgctg aaaccgtcaa aaagaaggct 1680
atggagttcc tcaagtatat caacgccaaa cttccgggcg cgcttgagct cgagtacgag 1740
ggcttctaca aacgcggctt cttcgtcacg aagNNNaagt atgcggtgat agacgaggaa 1800
ggcaagataa caacgcgcgg acttgagatt gtgaggcgtg actggagcga gatagcgaaa 1860
gagacgcagg cgagggttct tgaagctttg ctaaaggacg gtgacgtcga gaaggccgtg 1920
aggatagtca aagaagttac cgaaaagctg agcaagtacg aggttccgcc ggagaagctg 1980
gtgatccacg agcagataac gagggattta aaggactaca aggcaaccgg tccccacgtt 2040
gccgttgcca agaggttggc cgcgagagga gtcaaaatac gccctggaac ggtgataagc 2100
tacatcgtgc tcaagggctc tgggaggata ggcgacaggg cgataccgtt cgacgagttc 2160
gacccgacga agcacaagta cgacgccgag tactacattg agaaccaggt tctcccagcc 2220
gttgagagaa ttctgagagc cttcggttac cgcaaggaag acctgcgcta ccagaagacg 2280
agacaggttg gtttgagtgc ttggctgaag ccgaagggaa cttga  2325  [SEQ ID NO. 104]
```

**Vent DNA polymerase wild type**

```
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggagga tatgtaaaag agccagaaaa aggtttgtgg 1200
gaaaatatca tttatttgga tttccgcagt ctgtaccctt caataatagt tactcacaac 1260
gtatccccag atacccttga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
ggtatcccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 105]
```

**Vent Y387N NNN=AAT, AAC (All possible N codons)**
**Vent Y387L NNN=TTA, TTG, CTT, CTC, CTA, CTG (All possible L codons)**
**Vent Y387H NNN= CAT, CAC (All possible H codons)**
**Vent Y387Q NNN= CAA, CAG (All possible Q codons)**
**Vent Y387S NNN= TCT, TCC, TCA, TCG, AGT, AGC (All possible S codons)**

```
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaata 300
agggaacatc cagctgtggt tgacatttac gaatatgaca taccctttgc caagcgttat 360
```

ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactNN Nctgggagga tatgtaaaag agccagaaaa aggtttgtgg 1200
gaaaatatca tttatttgga tttccgcagt ctgtaccctt caataatagt tactcacaac 1260
gtatccccag ataccttga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
gggtatccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc aggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 106]

**Vent G389S NNN= TCT, TCC, TCA, TCG, AGT, AGC (All possible S codons)**
**Vent G389P NNN= CCT, CCA, CCG, CCC (All possible P codons)**
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg gggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020

caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgNNNgga tatgtaaaag agccagaaaa aggtttgtgg 1200
gaaaatatca tttatttgga tttccgcagt ctgtaccctt caataatagt tactcacaac 1260
gtatccccag ataccettga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
gggtatccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc aggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 107]

**Vent G390A NNN= GCA, GCT, GCC, GCG (All possible A codons)**
**Vent G390P NNN= CCT, CCA, CCG, CCC (All possible P codons)**

atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggaNNN tatgtaaaag agccagaaaa aggtttgtgg 1200
gaaaatatca tttatttgga tttccgcagt ctgtaccctt caataatagt tactcacaac 1260
gtatccccag atacccttga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
gggtatccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680

aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 108]

**Vent D407E NNN= GAA, GAG (All possible E codons)**
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggagga tatgtaaaag agccagaaaa aggtttgtgg 1200
gaaaatatca tttatttgNNN Nttccgcagt ctgtaccctt caataatagt tactcacaac 1260
gtatccccag ataccettga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
ggtatcccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 109]

Vent T544P NNN= CCT, CCA, CCG, CCC (All possible P codons)
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggagga tatgtaaaag agccagaaaa aggtttgtgg 1200
gaaaatatca tttatttgga tttccgcagt ctgtaccctt caataatagt tactcacaac 1260
gtatccccag ataccttgga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
ggtatcccta aggcaagatg gtactcgaag gaatgtgctg aaagcgttac cgcatggggg 1560
agacactaca tagagatgac gataagagaa atagaggaaa agttcggctt taaggttctt 1620
tatgcggacN NNgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 110]

Vent D545G NNN=GGT, GGC, GGA, GGG (All possible G codons)
atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg ggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660

aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggagga tatgtaaaag agccagaaaa aggtttgtgg 1200
gaaaatatca tttatttgga tttccgcagt ctgtaccctt caataatagt tactcacaac 1260
gtatccccag ataccettga aaaagagggc tgtaagaatt acgatgttgc tccgatagta 1320
ggatataggt tctgcaagga ctttccgggc tttattccct ccatactcgg ggacttaatt 1380
gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
gggtatccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctNNNggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaaagcgcta tgcagtcata 1800
gatgaagagg gcaggataac aacaagggge ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aattttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 111]

**Vent K595T NNN=ACT, ACC, ACA, ACG (All possible T codons)**

atgatactgg acactgatta cataacaaaa gatggcaagc ctataatccg aatttttaag 60
aaagagaacg gggagtttaa aatagaactt gaccctcatt ttcagcccta tatatatgct 120
cttctcaaag atgactccgc tattgaggag ataaaggcaa taaagggcga gagacatgga 180
aaaactgtga gagtgctcga tgcagtgaaa gtcaggaaaa aatttttggg aagggaagtt 240
gaagtctgga agctcatttt cgagcatccc caagacgttc cagctatgcg gggcaaaata 300
agggaacatc cagctgtggt tgacatttac gaatatgaca taccctttgc caagcgttat 360
ctcatagaca agggcttgat tcccatggag ggagacgagg agcttaagct ccttgccttt 420
gatattgaaa cgttttatca tgagggagat gaatttggaa agggcgagat aataatgatt 480
agttatgccg atgaagaaga ggccagagta atcacatgga aaaatatcga tttgccgtat 540
gtcgatgttg tgtccaatga aagagaaatg ataaagcgtt ttgttcaagt tgttaaagaa 600
aaagaccccg atgtgataat aacttacaat ggggacaatt ttgatttgcc gtatctcata 660
aaacgggcag aaaagctggg agttcggctt gtcttaggaa gggacaaaga acatcccgaa 720
cccaagattc agaggatggg tgatagtttt gctgtggaaa tcaagggtag aatccacttt 780
gatcttttcc cagttgtgcg aaggacgata aacctcccaa cgtatacgct tgaggcagtt 840
tatgaagcag ttttaggaaa aaccaaaagc aaattaggag cagaggaaat tgccgctata 900
tgggaaacag aagaaagcat gaaaaaacta gcccagtact caatggaaga tgctagggca 960
acgtatgagc tcgggaagga attcttcccc atggaagctg agctggcaaa gctgataggt 1020
caaagtgtat gggacgtctc gagatcaagc accggcaacc tcgtggagtg gtatctttta 1080
agggtggcat acgcgaggaa tgaacttgca ccgaacaaac ctgatgagga agagtataaa 1140
cggcgcttaa gaacaactta cctgggagga tatgtaaaag agccagaaaa aggtttgtgg 1200
gaaaatatca tttatttgga tttccgcagt ctgtaccctt caataatagt tactcacaac 1260
gtatccccag ataccettga aaaagagggc tgtaagaatt acgatgttgc tccgatagta 1320
ggatataggt tctgcaagga ctttccgggc tttattccct ccatactcgg ggacttaatt 1380

gcaatgaggc aagatataaa gaagaaaatg aaatccacaa ttgacccgat cgaaaagaaa 1440
atgctcgatt ataggcaaag ggctattaaa ttgcttgcaa acagctatta cggctatatg 1500
gggtatccta aggcaagatg gtactcgaag gaatgtgctg aaagcgttac cgcatggggg 1560
agacactaca tagagatgac gataagagaa atagaggaaa agttcggctt taaggttctt 1620
tatgcggaca ctgacggctt ttatgccaca atacccgggg aaaagcctga actcattaaa 1680
aagaaagcca aggaattcct aaactacata aactccaaac ttccaggtct gcttgagctt 1740
gagtatgagg gcttttactt gagaggattc tttgttacaa aaNNNcgcta tgcagtcata 1800
gatgaagagg gcaggataac aacaaggggc ttggaagtag taaggagaga ttggagtgag 1860
atagctaagg agactcaggc aaaggtttta gaggctatac ttaaagaggg aagtgttgaa 1920
aaagctgtag aagttgttag agatgttgta gagaaaatag caaaatacag ggttccactt 1980
gaaaagcttg ttatccatga gcagattacc agggatttaa aggactacaa agccattggc 2040
cctcatgtcg cgatagcaaa aagacttgcc gcaagaggga taaaagtgaa accgggcaca 2100
ataataagct atatcgttct caaagggagc ggaaagataa gcgatagggt aatttactt 2160
acagaatacg atcctagaaa acacaagtac gatccggact actacataga aaaccaagtt 2220
ttgccggcag tacttaggat actcgaagcg tttggataca gaaaggagga tttaaggtat 2280
caaagctcaa aacaaaccgg cttagatgca tggctcaaga ggtag 2325 [SEQ ID NO. 112]

Deep Vent
mildadyitedgkpiirifkkengefkveydrnfrpyiyallkddsqidevrkitaerhgkivriidaekvrkkflg
rpievwrlyfehpqdvpairdkirehsavidifeydipfakrylidkglipmegdeelkllafdietlyhegeefak
gpiimisyadeeeakvitwkkidlpyvevvsseremikrflkvirekdpdviityngdsfdlpylvkraeklgiklp
lgrdgsepkmqrlgdmtaveikgrihfdlyhvirrtinlptytleavyeaifgkpkekvyaheiaeawetgkglerv
akysmedakvtyelgreffpmeaqlsrlvgqplwdvsrsstgnlvewyllrkayernelapnkpdereyerrlresy
aggyvkepekglweglvsldfrslypsiiithnvspdtlnregcreydvapevghkfckdfpgfipsllkrllderq
eikrkmkaskdpiekkmldyrqraikilansyygyygyakarwyckecaesvtawgreyiefvrkeleekfgfkvly
idtdglyatipgakpeeikkkalefvdyinaklpglleleyegfyvrgffvtkkkyalideegkiitrgleivrrdw
seiaketqakvleailkhgnveeavkivkevteklskyeippeklviyeqitrplheykaigphvavakrlaargvk
vrpgmvigyivlrgdgpiskrailaeefdlrkhkydaeyyienqvlpavlrileafgyrkedlrwqktkqtgltawl
nikkk [SEQ ID NO. 113]

**Deep Vent Y385N NNN= AAT, AAC (All possible N codons)**
**Deep Vent Y385L NNN= TTA, TTG, CTT, CTC, CTA, CTG (All possible L codons)**
**Deep Vent Y385H NNN= CAT, CAC (All possible H codons)**
**Deep Vent Y385Q NNN= CAA, CAG (All possible Q codons)**
**Deep Vent Y385S NNN= TCT, TCC, TCA, TCG, AGT, AGC (All possible S codons)**

```
ATGATACTTG ACGCTGACTA CATCACCGAG GATGGGAAGC CGATTATAAG GATTTTCAAG     60
AAAGAAAACG GCGAGTTTAA GGTTGAGTAC GACAGAAACT TTAGACCTTA CATTTACGCT    120
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCNNNGCTGG GGGATACGTT AAGGAGCCGG AGAAAGGGCT CTGGGAGGGG   1200
TTAGTTTCCC TAGATTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACACAGATG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGAAGA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
```

GGGTACATAG TGCTGAGGGG AGACGGGCCA ATAAGCAAGA GGGCTATCCT TGCAGAGGAG 2160
TTCGATCTCA GGAAGCATAA GTATGACGCT GAGTATTACA TAGAAAATCA GGTTTTACCT 2220
GCCGTTCTTA GAATATTAGA GGCCTTTGGG TACAGGAAAG AAGACCTCAG GTGGCAGAAG 2280
ACTAAACAGA CAGGTCTTAC GGCATGGCTT AACATCAAGA AGAAGTAA 2328
[SEQ ID NO. 114]

**Deep Vent G387S NNN= TCT, TCC, TCA, TCG, AGT, AGC (All possible S codons)**
**Deep Vent G387P NNN= CCT, CCA, CCG, CCC (All possible P codons)**
ATGATACTTG ACGCTGACTA CATCACCGAG GATGGGAAGC CGATTATAAG GATTTTCAAG 60
AAAGAAAACG GCGAGTTTAA GGTTGAGTAC GACAGAAACT TTAGACCTTA CATTTACGCT 120
CTCCTCAAAG ATGACTCGCA GATTGATGAG GTTAGGAAGA TAACCGCCGA GAGGCATGGG 180
AAGATAGTGA GAATTATAGA TGCCGAAAAG GTAAGGAAGA AGTTCCTGGG GAGGCCGATT 240
GAGGTATGGA GGCTGTACTT TGAACACCCT CAGGACGTTC CCGCAATAAG GGATAAGATA 300
AGAGAGCATT CCGCAGTTAT TGACATCTTT GAGTACGACA TTCCGTTCGC GAAGAGGTAC 360
CTAATAGACA AAGGCCTAAT TCCAATGGAA GGCGATGAAG AGCTCAAGTT GCTCGCATTT 420
GACATAGAAA CCCTCTATCA CGAAGGGGAG GAGTTCGCGA AGGGGCCCAT TATAATGATA 480
AGCTATGCTG ATGAGGAAGA AGCCAAAGTC ATAACGTGGA AAAAGATCGA TCTCCCGTAC 540
GTCGAGGTAG TTTCCAGCGA GAGGGAGATG ATAAAGCGGT TCCTCAAGGT GATAAGGGAG 600
AAAGATCCCG ATGTTATAAT TACCTACAAC GGCGATTCTT TCGACCTTCC CTATCTAGTT 660
AAGAGGGCCG AAAAGCTCGG GATAAAGCTA CCCCTGGGAA GGGACGGTAG TGAGCCAAAG 720
ATGCAGAGGC TTGGGGATAT GACAGCGGTG GAGATAAAGG GAAGGATACA CTTTGACCTC 780
TACCACGTGA TTAGGAGAAC GATAAACCTC CCAACATACA CCCTCGAGGC AGTTTATGAG 840
GCAATCTTCG GAAAGCCAAA GGAGAAAGTT TACGCTCACG AGATAGCTGA GGCCTGGGAG 900
ACTGGAAAGG GACTGGAGAG AGTTGCAAAG TATTCAATGG AGGATGCAAA GGTAACGTAC 960
GAGCTCGGTA GGGAGTTCTT CCCAATGGAG GCCCAGCTTT CAAGGTTAGT CGGCCAGCCC 1020
CTGTGGGATG TTTCTAGGTC TTCAACTGGC AACTTGGTGG AGTGGTACCT CCTCAGGAAG 1080
GCCTACGAGA GGAATGAATT GGCTCCAAAC AAGCCGGATG AGAGGGAGTA CGAGAGAAGG 1140
CTAAGGGAGA GCTACGCTNN NGGATACGTT AAGGAGCCGG AGAAAGGGCT CTGGGAGGGG 1200
TTAGTTTCCC TAGATTTCAG GAGCCTGTAC CCCTCGATAA TAATCACCCA TAACGTCTCA 1260
CCGGATACGC TGAACAGGGA AGGGTGTAGG GAATACGATG TCGCCCCAGA GGTTGGGCAC 1320
AAGTTCTGCA AGGACTTCCC GGGGTTTATC CCCAGCCTGC TCAAGAGGTT ATTGGATGAA 1380
AGGCAAGAAA TAAAAAGGAA GATGAAAGCT TCTAAAGACC CAATCGAGAA GAAGATGCTT 1440
GATTACAGGC AACGGGCAAT CAAAATCCTG GCAAACAGCT ATTATGGGTA TTATGGGTAC 1500
GCAAAAGCCC GTTGGTACTG TAAGGAGTGC GCAGAGAGCG TTACGGCCTG GGGGAGGGAA 1560
TATATAGAGT TCGTAAGGAA GGAACTGGAG GAAAAGTTCG GGTTCAAAGT CTTATACATA 1620
GACACAGATG GACTCTACGC CACAATTCCT GGGGCAAAAC CCGAGGAGAT AAAGAAGAAA 1680
GCCCTAGAGT TCGTAGATTA TATAAACGCC AAGCTCCCAG GGCTGTTGGA GCTTGAGTAC 1740
GAGGGCTTCT ACGTGAGAGG GTTCTTCGTG ACGAAGAAGA AGTATGCGTT GATAGATGAG 1800
GAAGGGAAGA TAATCACTAG GGGGCTTGAA ATAGTCAGGA GGGACTGGAG CGAAATAGCC 1860
AAAGAAACCC AAGCAAAAGT CCTAGAGGCT ATCCTAAAGC ATGGCAACGT TGAGGAGGCA 1920
GTAAAGATAG TTAAGGAGGT AACTGAAAAG CTGAGCAAGT ACGAAATACC TCCAGAAAAG 1980
CTAGTTATTT ACGAGCAGAT CACGAGGCCC CTTCACGAGT ACAAGGCTAT AGGTCCGCAC 2040
GTTGCCGTGG CAAAAAGGTT AGCCGCTAGA GGAGTAAAGG TGAGGCCTGG CATGGTGATA 2100
GGGTACATAG TGCTGAGGGG AGACGGGCCA ATAAGCAAGA GGGCTATCCT TGCAGAGGAG 2160
TTCGATCTCA GGAAGCATAA GTATGACGCT GAGTATTACA TAGAAAATCA GGTTTTACCT 2220
GCCGTTCTTA GAATATTAGA GGCCTTTGGG TACAGGAAAG AAGACCTCAG GTGGCAGAAG 2280
ACTAAACAGA CAGGTCTTAC GGCATGGCTT AACATCAAGA AGAAGTAA 2328
[SEQ ID NO. 115]

**Deep Vent G388A NNN= GCA, GCT, GCC, GCG (All possible A codons)**
**Deep Vent G388P NNN= CCT, CCA, CCG, CCC (All possible P codons)**
ATGATACTTG ACGCTGACTA CATCACCGAG GATGGGAAGC CGATTATAAG GATTTTCAAG 60
AAAGAAAACG GCGAGTTTAA GGTTGAGTAC GACAGAAACT TTAGACCTTA CATTTACGCT 120

```
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCTACGCTGG GNNNTACGTT AAGGAGCCGG AGAAAGGGCT CTGGGAGGGG   1200
TTAGTTTCCC TAGATTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACACAGATG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGAAGA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
GGGTACATAG TGCTGAGGGG AGACGGGCCA ATAAGCAAGA GGGCTATCCT TGCAGAGGAG   2160
TTCGATCTCA GGAAGCATAA GTATGACGCT GAGTATTACA TAGAAAATCA GGTTTTACCT   2220
GCCGTTCTTA GAATATTAGA GGCCTTTGGG TACAGGAAAG AAGACCTCAG GTGGCAGAAG   2280
ACTAAACAGA CAGGTCTTAC GGCATGGCTT AACATCAAGA AGAAGTAA                2328
[SEQ ID NO. 116]
```

**Deep Vent D405E NNN= GAA, GAG (All possible E codons)**

```
ATGATACTTG ACGCTGACTA CATCACCGAG GATGGGAAGC CGATTATAAG GATTTTCAAG     60
AAAGAAAACG GCGAGTTTAA GGTTGAGTAC GACAGAAACT TTAGACCTTA CATTTACGCT    120
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
```

```
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCTACGCTGG GGATACGTT AAGGAGCCGG AGAAAGGGCT CTGGGAGGGG   1200
TTAGTTTCCC TANNNTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACACAGATG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGAAGA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
GGGTACATAG TGCTGAGGGG AGACGGGCCA ATAAGCAAGA GGGCTATCCT TGCAGAGGAG   2160
TTCGATCTCA GGAAGCATAA GTATGACGCT GAGTATTACA TAGAAAATCA GGTTTTACCT   2220
GCCGTTCTTA GAATATTAGA GGCCTTTGGG TACAGGAAAG AAGACCTCAG GTGGCAGAAG   2280
ACTAAACAGA CAGGTCTTAC GGCATGGCTT AACATCAAGA AGAAGTAA                2328
[SEQ ID NO. 117]
```

**Deep Vent T542P NNN= CCT, CCA, CCG, CCC (All possible P codons)**

```
ATGATACTTG ACGCTGACTA CATCACCGAG GATGGGAAGC CGATTATAAG GATTTTCAAG     60
AAAGAAAACG GCGAGTTTAA GGTTGAGTAC GACAGAAACT TTAGACCTTA CATTTACGCT    120
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCTACGCTGG GGATACGTT AAGGAGCCGG AGAAAGGGCT CTGGGAGGGG   1200
TTAGTTTCCC TAGATTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
```

```
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACNNNGATG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGAAGA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
GGGTACATAG TGCTGAGGGG AGACGGGCCA ATAAGCAAGA GGGCTATCCT TGCAGAGGAG   2160
TTCGATCTCA GGAAGCATAA GTATGACGCT GAGTATTACA TAGAAAATCA GGTTTTACCT   2220
GCCGTTCTTA GAATATTAGA GGCCTTTGGG TACAGGAAAG AAGACCTCAG GTGGCAGAAG   2280
ACTAAACAGA CAGGTCTTAC GGCATGGCTT AACATCAAGA AGAAGTAA                2328
[SEQ ID NO. 118]
```

**Deep Vent D543G NNN=GGT, GGC, GGA, GGG (All possible G codons)**

```
ATGATACTTG ACGCTGACTA CATCACCGAG GATGGGAAGC CGATTATAAG GATTTTCAAG     60
AAAGAAAACG GCGAGTTTAA GGTTGAGTAC GACAGAAACT TTAGACCTTA CATTTACGCT    120
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCTACGCTGG GGGATACGTT AAGGAGCCGG AGAAAGGGCT CTGGGAGGGG   1200
TTAGTTTCCC TAGATTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACACANNNG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGAAGA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
```

```
GGGTACATAG TGCTGAGGGG AGACGGGCCA ATAAGCAAGA GGGCTATCCT TGCAGAGGAG   2160
TTCGATCTCA GGAAGCATAA GTATGACGCT GAGTATTACA TAGAAAATCA GGTTTTACCT   2220
GCCGTTCTTA GAATATTAGA GGCCTTTGGG TACAGGAAAG AAGACCTCAG GTGGCAGAAG   2280
ACTAAACAGA CAGGTCTTAC GGCATGGCTT AACATCAAGA AGAAGTAA                2328
[SEQ ID NO. 119]
```

**Deep Vent K593T NNN=ACT, ACC, ACA, ACG (All possible T codons)**

```
ATGATACTTG ACGCTGACTA CATCACCGAG GATGGGAAGC CGATTATAAG GATTTTCAAG     60
AAAGAAAACG GCGAGTTTAA GGTTGAGTAC GACAGAAACT TTAGACCTTA CATTTACGCT    120
CTCCTCAAAG ATGACTCGCA GATTGATGAG GTTAGGAAGA TAACCGCCGA GAGGCATGGG    180
AAGATAGTGA GAATTATAGA TGCCGAAAAG GTAAGGAAGA AGTTCCTGGG GAGGCCGATT    240
GAGGTATGGA GGCTGTACTT TGAACACCCT CAGGACGTTC CCGCAATAAG GGATAAGATA    300
AGAGAGCATT CCGCAGTTAT TGACATCTTT GAGTACGACA TTCCGTTCGC GAAGAGGTAC    360
CTAATAGACA AAGGCCTAAT TCCAATGGAA GGCGATGAAG AGCTCAAGTT GCTCGCATTT    420
GACATAGAAA CCCTCTATCA CGAAGGGGAG GAGTTCGCGA AGGGGCCCAT TATAATGATA    480
AGCTATGCTG ATGAGGAAGA AGCCAAAGTC ATAACGTGGA AAAAGATCGA TCTCCCGTAC    540
GTCGAGGTAG TTTCCAGCGA GAGGGAGATG ATAAAGCGGT TCCTCAAGGT GATAAGGGAG    600
AAAGATCCCG ATGTTATAAT TACCTACAAC GGCGATTCTT TCGACCTTCC CTATCTAGTT    660
AAGAGGGCCG AAAAGCTCGG GATAAAGCTA CCCCTGGGAA GGGACGGTAG TGAGCCAAAG    720
ATGCAGAGGC TTGGGGATAT GACAGCGGTG GAGATAAAGG GAAGGATACA CTTTGACCTC    780
TACCACGTGA TTAGGAGAAC GATAAACCTC CCAACATACA CCCTCGAGGC AGTTTATGAG    840
GCAATCTTCG GAAAGCCAAA GGAGAAAGTT TACGCTCACG AGATAGCTGA GGCCTGGGAG    900
ACTGGAAAGG GACTGGAGAG AGTTGCAAAG TATTCAATGG AGGATGCAAA GGTAACGTAC    960
GAGCTCGGTA GGGAGTTCTT CCCAATGGAG GCCCAGCTTT CAAGGTTAGT CGGCCAGCCC   1020
CTGTGGGATG TTTCTAGGTC TTCAACTGGC AACTTGGTGG AGTGGTACCT CCTCAGGAAG   1080
GCCTACGAGA GGAATGAATT GGCTCCAAAC AAGCCGGATG AGAGGGAGTA CGAGAGAAGG   1140
CTAAGGGAGA GCTACGCTGG GGGATACGTT AAGGAGCCGG AGAAAGGGCT CTGGGAGGGG   1200
TTAGTTTCCC TAGATTTCAG GAGCCTGTAC CCCTCGATAA TAATCACCCA TAACGTCTCA   1260
CCGGATACGC TGAACAGGGA AGGGTGTAGG GAATACGATG TCGCCCCAGA GGTTGGGCAC   1320
AAGTTCTGCA AGGACTTCCC GGGGTTTATC CCCAGCCTGC TCAAGAGGTT ATTGGATGAA   1380
AGGCAAGAAA TAAAAAGGAA GATGAAAGCT TCTAAAGACC CAATCGAGAA GAAGATGCTT   1440
GATTACAGGC AACGGGCAAT CAAAATCCTG GCAAACAGCT ATTATGGGTA TTATGGGTAC   1500
GCAAAAGCCC GTTGGTACTG TAAGGAGTGC GCAGAGAGCG TTACGGCCTG GGGGAGGGAA   1560
TATATAGAGT TCGTAAGGAA GGAACTGGAG GAAAAGTTCG GGTTCAAAGT CTTATACATA   1620
GACACAGATG GACTCTACGC CACAATTCCT GGGGCAAAAC CCGAGGAGAT AAAGAAGAAA   1680
GCCCTAGAGT TCGTAGATTA TATAAACGCC AAGCTCCCAG GGCTGTTGGA GCTTGAGTAC   1740
GAGGGCTTCT ACGTGAGAGG GTTCTTCGTG ACGAAGNNNA AGTATGCGTT GATAGATGAG   1800
GAAGGGAAGA TAATCACTAG GGGGCTTGAA ATAGTCAGGA GGGACTGGAG CGAAATAGCC   1860
AAAGAAACCC AAGCAAAAGT CCTAGAGGCT ATCCTAAAGC ATGGCAACGT TGAGGAGGCA   1920
GTAAAGATAG TTAAGGAGGT AACTGAAAAG CTGAGCAAGT ACGAAATACC TCCAGAAAAG   1980
CTAGTTATTT ACGAGCAGAT CACGAGGCCC CTTCACGAGT ACAAGGCTAT AGGTCCGCAC   2040
GTTGCCGTGG CAAAAAGGTT AGCCGCTAGA GGAGTAAAGG TGAGGCCTGG CATGGTGATA   2100
GGGTACATAG TGCTGAGGGG AGACGGGCCA ATAAGCAAGA GGGCTATCCT TGCAGAGGAG   2160
TTCGATCTCA GGAAGCATAA GTATGACGCT GAGTATTACA TAGAAAATCA GGTTTTACCT   2220
GCCGTTCTTA GAATATTAGA GGCCTTTGGG TACAGGAAAG AAGACCTCAG GTGGCAGAAG   2280
ACTAAACAGA CAGGTCTTAC GGCATGGCTT AACATCAAGA AGAAGTAA                2328
[SEQ ID NO. 120]
```

Tgo Y384N NNN=AAT, AAC
Tgo Y384L NNN=TTA, TTG, CTT, CTC, CTA, CTG
Tgo Y384H NNN=CAT, CAC
Tgo Y384Q NNN=CAA, CAG

Tgo Y384S NNN=TCT, TCC, TCA, TCG, AGT, AGC

```
atgatcctcg atacagacta cataactgag gatggaaagc ccgtcatcag gatcttcaag     60
aaggagaacg gcgagttcac catagactac gacagaaact ttgagccata catctacgcg    120
ctcttgaagg acgactctcc gattgaggac gtcaagaaga taactgccga gaggcacggc    180
actaccgtta gggttgtcag ggccgagaaa gtgaagaaga agttcctagg caggccgata    240
gaggtctgga agctctactt cactcacccc caggacgttc ccgcaatcag ggacaagata    300
aaggagcatc ctgccgttgt ggacatctac gagtacgaca tccccttcgc gaagcgctac    360
ctcatagaca aaggcttaat cccgatggag ggcgacgagg aacttaagat gctcgccttc    420
gacatcgaga cgctctatca cgagggcgag gagttcgccg aagggcctat cctgatgata    480
agctacgccg acgaggaagg ggcgcgcgtt attacctgga agaatatcga ccttccctat    540
gtcgacgtcg tttccaccga gaaggagatg ataaagcgct tcctcaaggt cgtcaaggaa    600
aaggatcccg acgtcctcat aatctacaac ggcgacaact tcgacttcgc ctacctcaag    660
aagcgctccg agaagctcgg agtcaagttc atcctcggaa gggaagggag cgaaccgaaa    720
atccagcgca tgggcgatcg ctttgcggtg gaggtcaagg gaaggattca cttcgacctc    780
tacccgtca ttaggagaac gattaacctc cccacttaca cccttgaggc agtatatgaa    840
gccatctttg gacagccgaa ggagaaggtc tacgctgagg agatagcgca ggcctgggaa    900
acgggcgagg gattagaaag ggtggcccgc tactcgatgg aggacgcgaa ggtaacctat    960
gaactcggaa aagagttctt ccctatggaa gcccagctct cgcgcctcgt aggccagagc   1020
ctctgggatg tatctcgctc gagtaccgga aacctcgtcg agtggttttt gctgaggaag   1080
gcctacgaga ggaatgaact tgcaccaaac aagccggacg agagggagct ggcaagaaga   1140
agggagagcn nngcgggtgg atacgtcaag gagcccgaaa ggggactgtg ggagaacatc   1200
gtgtatctgg acttccgctc cctgtatcct tcgataataa tcacccataa cgtctcccct   1260
gatacactca acagggaggg ttgtgaggag tacgacgtgg ctcctcaggt aggccataag   1320
ttctgcaagg acttccccgg cttcatccca agcctcctcg gagacctctt ggaggagaga   1380
cagaaggtaa agaagaagat gaaggccact atagacccaa tcgagaagaa actcctcgat   1440
tacaggcaac gagcaatcaa aatccttgct aatagcttct acggttacta cggctataca   1500
aaggcccgct ggtactacaa ggagtgcgcc gagagcgtta ccggttgggg cagggagtac   1560
atcgagacca cgataaggga aatagaggag aaatttggct ttaaagtcct ctacgcggac   1620
acagatggat ttttcgcaac aatacctgga gcggacgccg aaaccgtcaa aaagaaggca   1680
aaggagttcc tggactacat caacgccaaa ctgcccggcc tgctcgaact cgaatacgag   1740
ggcttctaca agcgcggctt cttcgtgacg aagaagaagt acgcggttat agacgaggag   1800
gacaagataa cgacgcgcgg gcttgaaata gttaggcgtg actggagcga gatagcgaag   1860
gagacgcagg cgagggttct tgaggcgata ctaaagcacg gtgacgttga agaagcggta   1920
aggattgtca aagaggttac ggagaagctg agcaagtacg aggttccacc ggagaagctg   1980
gtcatctacg agcagataac ccgcgacctg aaggactaca aggccaccgg gccgcatgtg   2040
gctgttgcaa aacgcctcgc cgcaaggggg ataaaaatcc ggcccggaac ggtcataagc   2100
tacatcgtgc tcaaaggctc gggaaggatt ggggacaggg ctataccctt tgacgaattt   2160
gacccggcaa agcacaagta cgatgcagaa tactacatcg agaaccaggt tcttccagct   2220
gtggagagga ttctgagggc ctttggttac cgtaaagaag atttaaggta tcagaaaacg   2280
cggcaggttg gcttgggggc gtggctaaaa cctaagacat ga                        2322
```

[SEQ ID NO. 121]

Tgo G386S NNN=TCT, TCC, TCA, TCG, AGT, AGC
Tgo G386P NNN=CCT, CCA, CCG, CCC

```
atgatcctcg atacagacta cataactgag gatggaaagc ccgtcatcag gatcttcaag     60
aaggagaacg gcgagttcac catagactac gacagaaact ttgagccata catctacgcg    120
ctcttgaagg acgactctcc gattgaggac gtcaagaaga taactgccga gaggcacggc    180
actaccgtta gggttgtcag ggccgagaaa gtgaagaaga agttcctagg caggccgata    240
gaggtctgga agctctactt cactcacccc caggacgttc ccgcaatcag ggacaagata    300
aaggagcatc ctgccgttgt ggacatctac gagtacgaca tccccttcgc gaagcgctac    360
```

```
ctcatagaca aaggcttaat cccgatggag ggcgacgagg aacttaagat gctcgccttc     420
gacatcgaga cgctctatca cgagggcgag gagttcgccg aagggcctat cctgatgata     480
agctacgccg acgaggaagg ggcgcgcgtt attacctgga agaatatcga ccttccctat     540
gtcgacgtcg tttccaccga gaaggagatg ataaagcgct tcctcaaggt cgtcaaggaa     600
aaggatcccg acgtcctcat aatctacaac ggcgacaact tcgacttcgc ctacctcaag     660
aagcgctccg agaagctcgg agtcaagttc atcctcggaa gggaagggag cgaaccgaaa     720
atccagcgca tgggcgatcg ctttgcggtg gaggtcaagg gaaggattca cttcgacctc     780
taccccgtca ttaggagaac gattaacctc cccacttaca cccttgaggc agtatatgaa     840
gccatctttg gacagccgaa ggagaaggtc tacgctgagg agatagcgca ggcctgggaa     900
acgggcgagg gattagaaag ggtggcccgc tactcgatgg aggacgcgaa ggtaacctat     960
gaactcggaa aagagttctt ccctatggaa gcccagctct cgcgcctcgt aggccagagc    1020
ctctgggatg tatctcgctc gagtaccgga aacctcgtcg agtggttttt gctgaggaag    1080
gcctacgaga ggaatgaact tgcaccaaac aagccggacg agagggagct ggcaagaaga    1140
agggagagct acgcgnnngg atacgtcaag gagcccgaaa ggggactgtg ggagaacatc    1200
gtgtatctgg acttccgctc cctgtatcct tcgataataa tcacccataa cgtctcccct    1260
gatacactca acagggaggg ttgtgaggag tacgacgtgg ctcctcaggt aggccataag    1320
ttctgcaagg acttccccgg cttcatccca agcctcctcg gagacctctt ggaggagaga    1380
cagaaggtaa agaagaagat gaaggccact atagacccaa tcgagaagaa actcctcgat    1440
tacaggcaac gagcaatcaa aatccttgct aatagcttct acggttacta cggctataca    1500
aaggcccgct ggtactacaa ggagtgcgcc gagagcgtta ccggttgggg cagggagtac    1560
atcgagacca cgataaggga aatagaggag aaatttggct ttaaagtcct ctacgcggac    1620
acagatggat ttttcgcaac aatacctgga gcggacgccg aaaccgtcaa aaagaaggca    1680
aaggagttcc tggactacat caacgccaaa ctgcccggcc tgctcgaact cgaatacgag    1740
ggcttctaca agcgcggctt cttcgtgacg aagaagaagt acgcggttat agacgaggag    1800
gacaagataa cgacgcgcgg gcttgaaata gttaggcgtg actggagcga gatagcgaag    1860
gagacgcagg cgagggttct tgaggcgata ctaaagcacg gtgacgttga agaagcggta    1920
aggattgtca aagaggttac ggagaagctg agcaagtacg aggttccacc ggagaagctg    1980
gtcatctacg agcagataac ccgcgacctg aaggactaca aggccaccgg gccgcatgtg    2040
gctgttgcaa aacgcctcgc cgcaaggggg ataaaaatcc ggcccggaac ggtcataagc    2100
tacatcgtgc tcaaaggctc gggaaggatt ggggacaggg ctataccctt tgacgaattt    2160
gacccggcaa agcacaagta cgatgcagaa tactacatcg agaaccaggt tcttccagct    2220
gtggagagga ttctgagggc ctttggttac cgtaaagaag atttaaggta tcagaaaacg    2280
cggcaggttg gcttgggggc gtggctaaaa cctaagacat ga                       2322
[SEQ ID NO. 122]
```

Tgo G387A NNN=GCA, GCT, GCC, GCG
Tgo G386P NNN=CCT, CCA, CCG, CCC

```
atgatcctcg atacagacta cataactgag gatggaaagc ccgtcatcag gatcttcaag      60
aaggagaacg gcgagttcac catagactac gacagaaact ttgagccata catctacgcg     120
ctcttgaagg acgactctcc gattgaggac gtcaagaaga taactgccga gaggcacggc     180
actaccgtta gggttgtcag ggccgagaaa gtgaagaaga agttcctagg caggccgata     240
gaggtctgga agctctactt cactcacccc caggacgttc ccgcaatcag ggacaagata     300
aaggagcatc ctgccgttgt ggacatctac gagtacgaca tccccttcgc gaagcgctac     360
ctcatagaca aaggcttaat cccgatggag ggcgacgagg aacttaagat gctcgccttc     420
gacatcgaga cgctctatca cgagggcgag gagttcgccg aagggcctat cctgatgata     480
agctacgccg acgaggaagg ggcgcgcgtt attacctgga agaatatcga ccttccctat     540
gtcgacgtcg tttccaccga gaaggagatg ataaagcgct tcctcaaggt cgtcaaggaa     600
aaggatcccg acgtcctcat aatctacaac ggcgacaact tcgacttcgc ctacctcaag     660
aagcgctccg agaagctcgg agtcaagttc atcctcggaa gggaagggag cgaaccgaaa     720
atccagcgca tgggcgatcg ctttgcggtg gaggtcaagg gaaggattca cttcgacctc     780
taccccgtca ttaggagaac gattaacctc cccacttaca cccttgaggc agtatatgaa     840
```

```
gccatctttg gacagccgaa ggagaaggtc tacgctgagg agatagcgca ggcctgggaa     900
acgggcgagg gattagaaag ggtggcccgc tactcgatgg aggacgcgaa ggtaacctat     960
gaactcggaa aagagttctt ccctatggaa gcccagctct cgcgcctcgt aggccagagc    1020
ctctgggatg tatctcgctc gagtaccgga aacctcgtcg agtggttttt gctgaggaag    1080
gcctacgaga ggaatgaact tgcaccaaac aagccggacg agagggagct ggcaagaaga    1140
agggagagct acgcgggtnn ntacgtcaag gagcccgaaa ggggactgtg ggagaacatc    1200
gtgtatctgg acttccgctc cctgtatcct tcgataataa tcacccataa cgtctcccct    1260
gatacactca acagggaggg ttgtgaggag tacgacgtgg ctcctcaggt aggccataag    1320
ttctgcaagg acttccccgg cttcatccca agcctcctcg gagacctctt ggaggagaga    1380
cagaaggtaa agaagaagat gaaggccact atagacccaa tcgagaagaa actcctcgat    1440
tacaggcaac gagcaatcaa aatccttgct aatagcttct acggttacta cggctataca    1500
aaggcccgct ggtactacaa ggagtgcgcc gagagcgtta ccggttgggg cagggagtac    1560
atcgagacca cgataaggga aatagaggag aaatttggct ttaaagtcct ctacgcggac    1620
acagatggat tttcgcaac aatacctgga gcggacgccg aaaccgtcaa aaagaaggca     1680
aaggagttcc tggactacat caacgccaaa ctgcccggcc tgctcgaact cgaatacgag    1740
ggcttctaca agcgcggctt cttcgtgacg aagaagaagt acgcggttat agacgaggag    1800
gacaagataa cgacgcgcgg gcttgaaata gttaggcgtg actggagcga gatagcgaag    1860
gagacgcagg cgagggttct tgaggcgata ctaaagcacg gtgacgttga agaagcggta    1920
aggattgtca aagaggttac ggagaagctg agcaagtacg aggttccacc ggagaagctg    1980
gtcatctacg agcagataac ccgcgacctg aaggactaca aggccaccgg gccgcatgtg    2040
gctgttgcaa aacgcctcgc cgcaaggggg ataaaaatcc ggcccggaac ggtcataagc    2100
tacatcgtgc tcaaaggctc gggaaggatt ggggacaggg ctatacccttt tgacgaattt   2160
gacccggcaa agcacaagta cgatgcagaa tactacatcg agaaccaggt tcttccagct    2220
gtggagagga ttctgagggc ctttggttac cgtaaagaag atttaaggta tcagaaaacg    2280
cggcaggttg gcttgggggc gtggctaaaa cctaagacat ga                       2322
[SEQ ID NO. 123]
```

Tgo D404E NNN=GAA, GAG

```
atgatcctcg atacagacta cataactgag gatggaaagc ccgtcatcag gatcttcaag      60
aaggagaacg gcgagttcac catagactac gacagaaact ttgagccata catctacgcg     120
ctcttgaagg acgactctcc gattgaggac gtcaagaaga taactgccga gaggcacggc     180
actaccgtta gggttgtcag ggccgagaaa gtgaagaaga agttcctagg caggccgata     240
gaggtctgga agctctactt cactcacccc caggacgttc ccgcaatcag ggacaagata     300
aaggagcatc ctgccgttgt ggacatctac gagtacgaca tccccttcgc gaagcgctac     360
ctcatagaca aaggcttaat cccgatggag ggcgacgagg aacttaagat gctcgccttc     420
gacatcgaga cgctctatca cgagggcgag gagttcgccg aagggcctat cctgatgata     480
agctacgccg acgaggaagg ggcgcgcgtt attacctgga agaatatcga ccttccctat     540
gtcgacgtcg tttccaccga gaaggagatg ataaagcgct tcctcaaggt cgtcaaggaa     600
aaggatcccg acgtcctcat aatctacaac ggcgacaact tcgacttcgc ctacctcaag     660
aagcgctccg agaagctcgg agtcaagttc atcctcggaa gggaagggag cgaaccgaaa     720
atccagcgca tgggcgatcg ctttgcggtg gaggtcaagg gaaggattca cttcgacctc     780
taccccgtca ttaggagaac gattaacctc cccacttaca cccttgaggc agtatatgaa     840
gccatctttg gacagccgaa ggagaaggtc tacgctgagg agatagcgca ggcctgggaa     900
acgggcgagg gattagaaag ggtggcccgc tactcgatgg aggacgcgaa ggtaacctat     960
gaactcggaa aagagttctt ccctatggaa gcccagctct cgcgcctcgt aggccagagc    1020
ctctgggatg tatctcgctc gagtaccgga aacctcgtcg agtggttttt gctgaggaag    1080
gcctacgaga ggaatgaact tgcaccaaac aagccggacg agagggagct ggcaagaaga    1140
agggagagct acgcgggtgg atacgtcaag gagcccgaaa ggggactgtg ggagaacatc    1200
gtgtatctgn nnttccgctc cctgtatcct tcgataataa tcacccataa cgtctcccct    1260
gatacactca acagggaggg ttgtgaggag tacgacgtgg ctcctcaggt aggccataag    1320
ttctgcaagg acttccccgg cttcatccca agcctcctcg gagacctctt ggaggagaga    1380
```

```
cagaaggtaa agaagaagat gaaggccact atagacccaa tcgagaagaa actcctcgat    1440
tacaggcaac gagcaatcaa aatccttgct aatagcttct acggttacta cggctataca    1500
aaggcccgct ggtactacaa ggagtgcgcc gagagcgtta ccggttgggg cagggagtac    1560
atcgagacca cgataaggga aatagaggag aaatttggct ttaaagtcct ctacgcggac    1620
acagatggat ttttcgcaac aatacctgga gcggacgccg aaaccgtcaa aaagaaggca    1680
aaggagttcc tggactacat caacgccaaa ctgcccggcc tgctcgaact cgaatacgag    1740
ggcttctaca agcgcggctt cttcgtgacg aagaagaagt acgcggttat agacgaggag    1800
gacaagataa cgacgcgcgg gcttgaaata gttaggcgtg actggagcga gatagcgaag    1860
gagacgcagg cgagggttct tgaggcgata ctaaagcacg gtgacgttga agaagcggta    1920
aggattgtca aagaggttac ggagaagctg agcaagtacg aggttccacc ggagaagctg    1980
gtcatctacg agcagataac ccgcgacctg aaggactaca aggccaccgg gccgcatgtg    2040
gctgttgcaa aacgcctcgc cgcaaggggg ataaaaatcc ggcccggaac ggtcataagc    2100
tacatcgtgc tcaaaggctc gggaaggatt ggggacaggg ctataccctt tgacgaattt    2160
gacccggcaa agcacaagta cgatgcagaa tactacatcg agaaccaggt tcttccagct    2220
gtggagagga ttctgagggc ctttggttac cgtaaagaag atttaaggta tcagaaaacg    2280
cggcaggttg gcttggggge gtggctaaaa cctaagacat ga                       2322
[SEQ ID NO. 124]
```

Tgo T541P NNN=CCT, CCA, CCG, CCC

```
atgatcctcg atacagacta cataactgag gatggaaagc ccgtcatcag gatcttcaag      60
aaggagaacg gcgagttcac catagactac gacagaaact ttgagccata catctacgcg     120
ctcttgaagg acgactctcc gattgaggac gtcaagaaga taactgccga gaggcacggc     180
actaccgtta gggttgtcag ggccgagaaa gtgaagaaga agttcctagg caggccgata     240
gaggtctgga agctctactt cactcacccc caggacgttc ccgcaatcag ggacaagata     300
aaggagcatc ctgccgttgt ggacatctac gagtacgaca tccccttcgc gaagcgctac     360
ctcatagaca aaggcttaat cccgatggag ggcgacgagg aacttaagat gctcgccttc     420
gacatcgaga cgctctatca cgagggcgag gagttcgccg aagggcctat cctgatgata     480
agctacgccg acgaggaagg ggcgcgcgtt attacctgga agaatatcga ccttccctat     540
gtcgacgtcg tttccaccga gaaggagatg ataaagcgct tcctcaaggt cgtcaaggaa     600
aaggatcccg acgtcctcat aatctacaac ggcgacaact tcgacttcgc ctacctcaag     660
aagcgctccg agaagctcgg agtcaagttc atcctcggaa gggaagggag cgaaccgaaa     720
atccagcgca tgggcgatcg ctttgcggtg gaggtcaagg gaaggattca cttcgacctc     780
taccccgtca ttaggagaac gattaacctc cccacttaca cccttgaggc agtatatgaa     840
gccatctttg gacagccgaa ggagaaggtc tacgctgagg agatagcgca ggcctgggaa     900
acgggcgagg gattagaaag ggtggcccgc tactcgatgg aggacgcgaa ggtaacctat     960
gaactcggaa aagagttctt ccctatggaa gcccagctct cgcgcctcgt aggccagagc    1020
ctctgggatg tatctcgctc gagtaccgga aacctcgtcg agtggttttt gctgaggaag    1080
gcctacgaga ggaatgaact tgcaccaaac aagccggacg agagggagct ggcaagaaga    1140
agggagagct acgcgggtgg atacgtcaag gagcccgaaa ggggactgtg ggagaacatc    1200
gtgtatctgg acttccgctc cctgtatcct tcgataataa tcacccataa cgtctcccct    1260
gatacactca acagggaggg ttgtgaggag tacgacgtgg ctcctcaggt aggccataag    1320
ttctgcaagg acttccccgg cttcatccca agcctcctcg gagacctctt ggaggagaga    1380
cagaaggtaa agaagaagat gaaggccact atagacccaa tcgagaagaa actcctcgat    1440
tacaggcaac gagcaatcaa aatccttgct aatagcttct acggttacta cggctataca    1500
aaggcccgct ggtactacaa ggagtgcgcc gagagcgtta ccggttgggg cagggagtac    1560
atcgagacca cgataaggga aatagaggag aaatttggct ttaaagtcct ctacgcggac    1620
nnngatggat ttttcgcaac aatacctgga gcggacgccg aaaccgtcaa aaagaaggca    1680
aaggagttcc tggactacat caacgccaaa ctgcccggcc tgctcgaact cgaatacgag    1740
ggcttctaca agcgcggctt cttcgtgacg aagaagaagt acgcggttat agacgaggag    1800
gacaagataa cgacgcgcgg gcttgaaata gttaggcgtg actggagcga gatagcgaag    1860
gagacgcagg cgagggttct tgaggcgata ctaaagcacg gtgacgttga agaagcggta    1920
```

aggattgtca aagaggttac ggagaagctg agcaagtacg aggttccacc ggagaagctg 1980
gtcatctacg agcagataac ccgcgacctg aaggactaca aggccaccgg gccgcatgtg 2040
gctgttgcaa aacgcctcgc cgcaaggggg ataaaaatcc ggcccggaac ggtcataagc 2100
tacatcgtgc tcaaaggctc gggaaggatt ggggacaggg ctataccctt tgacgaattt 2160
gacccggcaa agcacaagta cgatgcagaa tactacatcg agaaccaggt tcttccagct 2220
gtggagagga ttctgagggc ctttggttac cgtaaagaag atttaaggta tcagaaaacg 2280
cggcaggttg gcttgggggc gtggctaaaa cctaagacat ga 2322
[SEQ ID NO. 125]

Tgo D542G NNN=GGT, GGA, GGG, GGC

atgatcctcg atacagacta cataactgag gatggaaagc ccgtcatcag gatcttcaag 60
aaggagaacg gcgagttcac catagactac gacagaaact ttgagccata catctacgcg 120
ctcttgaagg acgactctcc gattgaggac gtcaagaaga taactgccga gaggcacggc 180
actaccgtta gggttgtcag ggccgagaaa gtgaagaaga agttcctagg caggccgata 240
gaggtctgga agctctactt cactcacccc caggacgttc ccgcaatcag ggacaagata 300
aaggagcatc ctgccgttgt ggacatctac gagtacgaca tccccttcgc gaagcgctac 360
ctcatagaca aaggcttaat cccgatggag ggcgacgagg aacttaagat gctcgccttc 420
gacatcgaga cgctctatca cgagggcgag gagttcgccg aagggcctat cctgatgata 480
agctacgccg acgaggaagg ggcgcgcgtt attacctgga agaatatcga ccttccctat 540
gtcgacgtcg tttccaccga gaaggagatg ataaagcgct tcctcaaggt cgtcaaggaa 600
aaggatcccg acgtcctcat aatctacaac ggcgacaact tcgacttcgc ctacctcaag 660
aagcgctccg agaagctcgg agtcaagttc atcctcggaa gggaagggag cgaaccgaaa 720
atccagcgca tgggcgatcg ctttgcggtg gaggtcaagg gaaggattca cttcgacctc 780
taccccgtca ttaggagaac gattaacctc cccacttaca cccttgaggc agtatatgaa 840
gccatctttg gacagccgaa ggagaaggtc tacgctgagg agatagcgca ggcctgggaa 900
acgggcgagg gattagaaag ggtggcccgc tactcgatgg aggacgcgaa ggtaacctat 960
gaactcggaa aagagttctt ccctatggaa gcccagctct cgcgcctcgt aggccagagc 1020
ctctgggatg tatctcgctc gagtaccgga aacctcgtcg agtggttttt gctgaggaag 1080
gcctacgaga ggaatgaact tgcaccaaac aagccggacg agagggagct ggcaagaaga 1140
agggagagct acgcgggtgg atacgtcaag gagcccgaaa ggggactgtg ggagaacatc 1200
gtgtatctgg acttccgctc cctgtatcct tcgataataa tcacccataa cgtctcccct 1260
gatacactca acagggaggg ttgtgaggag tacgacgtgg ctcctcaggt aggccataag 1320
ttctgcaagg acttccccgg cttcatccca agcctcctcg gagacctctt ggaggagaga 1380
cagaaggtaa agaagaagat gaaggccact atagacccaa tcgagaagaa actcctcgat 1440
tacaggcaac gagcaatcaa aatccttgct aatagcttct acggttacta cggctataca 1500
aaggcccgct ggtactacaa ggagtgcgcc gagagcgtta ccggttgggg cagggagtac 1560
atcgagacca cgataaggga aatagaggag aaatttggct ttaaagtcct ctacgcggac 1620
acannnggat ttttcgcaac aatacctgga gcggacgccg aaaccgtcaa aaagaaggca 1680
aaggagttcc tggactacat caacgccaaa ctgcccggcc tgctcgaact cgaatacgag 1740
ggcttctaca agcgcggctt cttcgtgacg aagaagaagt acgcggttat agacgaggag 1800
gacaagataa cgacgcgcgg gcttgaaata gttaggcgtg actggagcga gatagcgaag 1860
gagacgcagg cgagggttct tgaggcgata ctaaagcacg gtgacgttga agaagcggta 1920
aggattgtca aagaggttac ggagaagctg agcaagtacg aggttccacc ggagaagctg 1980
gtcatctacg agcagataac ccgcgacctg aaggactaca aggccaccgg gccgcatgtg 2040
gctgttgcaa aacgcctcgc cgcaaggggg ataaaaatcc ggcccggaac ggtcataagc 2100
tacatcgtgc tcaaaggctc gggaaggatt ggggacaggg ctataccctt tgacgaattt 2160
gacccggcaa agcacaagta cgatgcagaa tactacatcg agaaccaggt tcttccagct 2220
gtggagagga ttctgagggc ctttggttac cgtaaagaag atttaaggta tcagaaaacg 2280
cggcaggttg gcttgggggc gtggctaaaa cctaagacat ga 2322
[SEQ ID NO. 126]

Tgo K592T NNN=ACT, ACC, ACA, ACG

```
atgatcctcg atacagacta cataactgag gatggaaagc ccgtcatcag gatcttcaag     60
aaggagaacg gcgagttcac catagactac gacagaaact ttgagccata catctacgcg    120
ctcttgaagg acgactctcc gattgaggac gtcaagaaga taactgccga gaggcacggc    180
actaccgtta gggttgtcag ggccgagaaa gtgaagaaga agttcctagg caggccgata    240
gaggtctgga agctctactt cactcacccc caggacgttc ccgcaatcag ggacaagata    300
aaggagcatc ctgccgttgt ggacatctac gagtacgaca tccccttcgc gaagcgctac    360
ctcatagaca aaggcttaat cccgatggag ggcgacgagg aacttaagat gctcgccttc    420
gacatcgaga cgctctatca cgagggcgag gagttcgccg aagggcctat cctgatgata    480
agctacgccg acgaggaagg ggcgcgcgtt attacctgga agaatatcga ccttccctat    540
gtcgacgtcg tttccaccga gaaggagatg ataaagcgct tcctcaaggt cgtcaaggaa    600
aaggatcccg acgtcctcat aatctacaac ggcgacaact tcgacttcgc ctacctcaag    660
aagcgctccg agaagctcgg agtcaagttc atcctcggaa gggaagggag cgaaccgaaa    720
atccagcgca tgggcgatcg ctttgcggtg gaggtcaagg gaaggattca cttcgacctc    780
taccccgtca ttaggagaac gattaacctc cccacttaca cccttgaggc agtatatgaa    840
gccatctttg gacagccgaa ggagaaggtc tacgctgagg agatagcgca ggcctgggaa    900
acgggcgagg gattagaaag ggtggcccgc tactcgatgg aggacgcgaa ggtaacctat    960
gaactcggaa aagagttctt ccctatggaa gcccagctct cgcgcctcgt aggccagagc   1020
ctctgggatg tatctcgctc gagtaccgga acctcgtcg agtggttttt gctgaggaag    1080
gcctacgaga ggaatgaact tgcaccaaac aagccggacg agagggagct ggcaagaaga   1140
agggagagct acgcgggtgg atacgtcaag gagcccgaaa ggggactgtg ggagaacatc   1200
gtgtatctgg acttccgctc cctgtatcct tcgataataa tcacccataa cgtctcccct   1260
gatacactca acagggaggg ttgtgaggag tacgacgtgg ctcctcaggt aggccataag   1320
ttctgcaagg acttccccgg cttcatccca agcctcctcg gagacctctt ggaggagaga   1380
cagaaggtaa agaagaagat gaaggccact atagacccaa tcgagaagaa actcctcgat   1440
tacaggcaac gagcaatcaa aatccttgct aatagcttct acggttacta cggctataca   1500
aaggcccgct ggtactacaa ggagtgcgcc gagagcgtta ccggttgggg cagggagtac   1560
atcgagacca cgataaggga aatagaggag aaatttggct ttaaagtcct ctacgcggac   1620
acagatggat tttcgcaac aatacctgga gcggacgccg aaaccgtcaa aaagaaggca    1680
aaggagttcc tggactacat caacgccaaa ctgcccggcc tgctcgaact cgaatacgag   1740
ggcttctaca agcgcggctt cttcgtgacg aagnnnaagt acgcggttat agacgaggag   1800
gacaagataa cgacgcgcgg gcttgaaata gttaggcgtg actggagcga gatagcgaag   1860
gagacgcagg cgagggttct tgaggcgata ctaaagcacg gtgacgttga agaagcggta   1920
aggattgtca aagaggttac ggagaagctg agcaagtacg aggttccacc ggagaagctg   1980
gtcatctacg agcagataac ccgcgacctg aaggactaca aggccaccgg gccgcatgtg   2040
gctgttgcaa aacgcctcgc cgcaaggggg ataaaaatcc ggcccggaac ggtcataagc   2100
tacatcgtgc tcaaaggctc gggaaggatt ggggacaggg ctataccctt tgacgaattt   2160
gacccggcaa agcacaagta cgatgcagaa tactacatcg agaaccaggt tcttccagct   2220
gtggagagga ttctgagggc ctttggttac cgtaaagaag atttaaggta tcagaaaacg   2280
cggcaggttg gcttgggggc gtggctaaaa cctaagacat ga                       2322
```

[SEQ ID NO. 127]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4492130 A
- US 4946786 A
- US 5210036 A
- US 5420029 A
- US 5489523 A
- US 5506137 A
- US 5545552 A
- US 5618711 A
- US 5624833 A
- US 6238905 B
- US 6100078 A
- US 6077664 A
- US 5968799 A
- US 5948663 A
- US 5885713 A
- US 5834285 A
- US 5756334 A
- US 5747298 A
- US 5744312 A
- US 5602011 A
- US 5556772 A
- US 5436149 A
- EP 1132474 A
- WO 0125483 A
- WO 0123583 A2
- WO 0132887 A
- US 5691142 A
- US 5614402 A
- US 5541311 A
- US 5866395 A
- US 4683195 A
- US 4683202 A
- US 4800159 A
- US 4965188 A
- US 4889818 A
- US 5075216 A
- US 5079352 A
- US 5104792 A
- US 5023171 A
- US 5091310 A
- US 5066584 A
- US 6183997 B
- US 5994079 A
- US 5827657 A
- US 5487993 A


### Non-patent literature cited in the description


- **KORNBERG ; BAKER.** DNA Replication. W. H. Freeman & Company, 1991
- **BARNES.** *Proceedings of the National Academy of Sciences,* 1994, vol. 91, 2216-20
- **MATTILA et al.** *Nucleic acid Research,* 1991, vol. 19, 4967-73
- **MATILLA P. et al.** *nucleic acids research,* January 1991, vol. 19 (18), 4967-4973
- **WATSON, J. D. et al.** Molecular Biology of the Gene. W. A. Benjamin, Inc, 1987
- **TRUNIGER et al.** *EMBO J.,* 1996, vol. 15, 3430-3441
- **LEVIN.** *Cell,* 1997, vol. 88, 5-8
- **BROSIUS et al.** *Virus Genes,* 1995, vol. 11, 163-79
- **BOSWORTH et al.** *Nature,* 1989, vol. 341, 167-168
- **LUNDBURG et al.** *Gene,* 1991, vol. 108, 1-6
- **KORNBERG ; BAKER.** DNA Replication. W. H. Freeman, 1991
- **LUNDBERG et al.** *Gene,* 1991, vol. 108, 1
- **HINNISDAELS et al.** *Biotechniques,* 1996, vol. 20, 186-8
- **MYERS ; GELFAND.** *Biochemistry,* 1991, vol. 30, 7661
- **STENESH ; MCGOWAN.** *Biochim Biophys Acta,* 1977, vol. 475, 32
- **CARIELLO et al.** *Polynucleotides Res,* 1991, vol. 19, 4193
- **DIAZ ; SABINO.** *Braz J. Med. Res,* 1998, vol. 31, 1239
- **CHIEN et al.** *J. Bacteoriol,* 1976, vol. 127, 1550
- **TAKAGI et al.** *Appl. Environ. Microbiol.,* 1997, vol. 63, 4504
- **JUNCOSA-GINESTA et al.** *Biotechniques,* 1994, vol. 16, 820
- **LECOMTE ; DOUBLEDAY.** *Polynucleotide Res.,* 1983, vol. 11, 7505
- **NORDSTROM et al.** *J. Biol. Chem.,* 1981, vol. 256, 3112
- **CANN et al.** *Proc Natl Acad Sci U S A,* 1998, vol. 95, 14250-5

- **KORNBERG ; BAKER.** DNA Replication
- **DIXON ; WEBB.** Enzymes. Academic Press, 1979
- **VERMA.** *Biochim Biophys Acta,* 1977, vol. 473, 1-38
- **WU et al.** *CRC Crit Rev Biochem,* 1975, vol. 3, 289-347
- **KUO.** *Ann N Y Acad Sci.,* 1994, vol. 726, 223-34
- **CANN et al.** *PNAS,* 1998, vol. 95, 14250-5
- **RODRIGUEZ et al.** *J. Mol. Biol.,* 2000, vol. 299, 447-62
- **ZHAO et al.** *Structure Fold Des.,* 1999, vol. 7, 1189-99
- **BAKER et al.** *Proc Natl Acad Sci U S A.,* 1998, vol. 95, 3507-12
- **KIEFER et al.** *Structure,* 1997, vol. 5, 95-108
- **KIM et al.** *Nature,* 1995, vol. 376, 612-6
- **KONG et al.** *J Biol Chem.,* 1993, vol. 268, 1965-75
- **BLANCO et al.** *Gene,* 1991, vol. 100, 27-38
- **BERNAD et al.** *Cell,* 1989, vol. 59, 219-28
- **JOHNSON.** *Annu Rev Biochem.,* 1993, vol. 62, 685-713
- *Protein Eng.,* 1990, vol. 3, 461-7
- **BLANCO et al.** *Methods of Enzymology,* 1995, vol. 262, 283-294
- **ABDUS SATTAR et al.** *Biochemistry,* 1996, vol. 35, 16621-9
- **TUSKE et al.** *J. Biological Chemistry,* 2000, vol. 275, 23759-68
- **BOHLKE et al.** *Nucleic Acid Research,* 2000, vol. 28, 3910-3917
- **PISANI et al.** *Biochemistry,* 1998, vol. 37, 15005-15012
- **KOMORI et al.** *Protein Eng,* 2000, vol. 13, 41-7
- **SHEN et al.** *J. Biological Chemistry,* 2001, vol. 276, 27376-83
- **BLANCO, L. ; SALAS, M.** *Methods of Enzymology,* 1995, vol. 262, 283-294
- **BOHLKE, K. et al.** *NAR,* 2000, vol. 28, 3910-3917
- **TRUNIGER, V. et al.** *EMBO J.,* 1996, vol. 15, 3430-3441
- **PISANI, F.M. ; DEFELICE, M. ; ROSSI, M.** *Biochemistry,* 1998, vol. 37, 15005-15012
- **AUSUBEL.** Short Protocols in Molecular Biology. John Wiley & Sons, Inc, 1995
- **NIELSON et al.** *Strategies,* 1994, vol. 7, 27
- **MATTEUCCI et al.** *J. Am. Chem. Soc.,* 1981, vol. 103, 3185-3191
- **DIXON ; WEBB.** *Enzymes*
- **SAGNER, G. ; RUGER, R. ; KESSLER, C.** *Gene,* 1991, vol. 97, 119-123
- **HOGREFE et al.** *Methods in Enzymology,* 2001, vol. 343, 91-116
- **KONG et al.** *J. Biol. Chem.,* 1993, vol. 268, 1965
- **LAWYER et al.** *PCR Meth. & App.,* 1993, vol. 2, 275
- **WRIGHT.** *Acta Biochim Pol.,* 1996, vol. 43, 115-24
- **ELION.** *Am J Med.,* 1982, vol. 73, 7-13
- **SAMBROOK et al.** Molecular Cloning. Cold Spring Harbor Laboratory Press, 1989
- **M. J. MCPHERSON et al.** PCR: A Practical Approach. IRL Press, 1991
- **INNIS et al.** PCR Protocols: A Guide to Methods and Applications. Academic Press, 1990
- **H. A. ERLICH.** DNA Amplification. Stockton Press, 1989
- **HIGUCHI ; KWOK.** *Nature,* 1989, vol. 339, 237-238
- **KWOK ; ORREGO et al.** PCR Protocols: A Guide to Methods and Applications. Academic Press, Inc, 1990
- **HOGREFE et al.** *Strategies,* 1997, vol. 10, 93-96
- **ERLICH.** *Rev Immunogenet.,* 1989, vol. 1, 127-34
- **PREDIGER.** *Methods Mol. Biol.,* 2001, vol. 160, 49-63
- **JURECIC et al.** *Curr. Opin. Microbiol.,* 2000, vol. 3, 316-21
- **TRIGLIA.** *Methods Mol. Biol.,* 2000, vol. 130, 79-83
- **MACLELLAND et al.** *PCR Methods Appl.,* 1994, vol. 4, 66-81
- **ABRAMSON ; MYERS.** *Current Opinion in Biotechnology,* 1993, vol. 4, 41-47
- **VERMA.** *Biochem.Biophys.Acta,* 1977, vol. 473, 1
- **PERBAL.** A Practical Guide to Molecular Cloning. Wiley & Sons, 1984
- **SAUNDERS ; SAUNDERS.** Microbial Genetics Applied to Biotechnology. Croom Helm, 1987
- **BERGER et al.** *Biochemistry,* 1983, vol. 22, 2365-2372
- **HAMES ; HIGGINS.** Polynucleotide Hybridisation: A Practical Approach. IRL Press Limited, 1985
- **LESNICK ; FREIER.** *Biochemistry,* 1995, vol. 34, 10807-10815
- **MCGRAW et al.** *Biotechniques,* 1990, vol. 8, 674-678
- **RYCHLIK et al.** *Polynucleotides Res.,* 1990, vol. 18, 6409-6412
- **DAVIS et al.** Basic Methods in Molecular Biology. Elsevier, 1986
- **AUSUBEL et al.** Current Protocols in Molecular Biology. John Wiley and Sons, 1987
- **KAWASAKI ; WANG.** PCR Technology. Stockton Press, 1989
- **KAWASAKI et al.** PCR Protocols: A Guide to Methods and Applications. Academic Press, 1990

- **AUSUBEL et al.** Current Protocols in Molecular Biology. John Wiley & Sons, 1987
- **CLINE, J. ; BRAMAN, J.C. ; HOGREFE, H.H.** *NAR,* 1996, vol. 24, 3546-3551
- **LUNDBERG, K.S. ; SHOEMAKER, D.D. ; ADAMS, M.W.W. ; SHORT, J.M. ; SORGE, J.A. ; MATHUR, E.J.** *Gene,* 1991, vol. 180, 1-8